(19) Europäisches Patentamt / European Patent Office / Office européen des brevets

(11) **EP 4 225 146 B1**

(12) **EUROPEAN PATENT SPECIFICATION**

(45) Date of publication and mention
of the grant of the patent:
**28.05.2025 Bulletin 2025/22**

(21) Application number: **21794930.4**

(22) Date of filing: **08.10.2021**

(51) International Patent Classification (IPC):
*A61B 6/03* (2006.01)   *G16H 50/50* (2018.01)
*G16H 50/30* (2018.01)   *G16H 50/20* (2018.01)
*A61B 6/00* (2024.01)   *A61B 6/50* (2024.01)

(52) Cooperative Patent Classification (CPC):
**A61B 6/5217; A61B 6/032; A61B 6/504;
G16H 50/20; G16H 50/30; G16H 50/50;**
A61B 6/507; G16H 20/40

(86) International application number:
**PCT/GB2021/052610**

(87) International publication number:
**WO 2022/074398 (14.04.2022 Gazette 2022/15)**

(54) **AORTIC ANEURYSM GROWTH RATE PREDICTION FROM GEOMETRIC ANALYSIS**

VORHERSAGE DER WACHSTUMSRATE VON AORTENANEURYSMEN AUS GEOMETRISCHER ANALYSE

PRÉDICTION DE TAUX DE CROISSANCE D'UN ANÉVRISME AORTIQUE À PARTIR D'UNE ANALYSE GÉOMÉTRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.10.2020 GB 202015970**

(43) Date of publication of application:
**16.08.2023 Bulletin 2023/33**

(73) Proprietor: **Oxford University Innovation Limited
Botley, Oxford OX2 0JB (GB)**

(72) Inventors:
• **REGENT, Lee**
**Botley Oxford OX2 0JB (GB)**
• **CHANDRASHEKAR, Anirudh**
**Botley Oxford OX2 0JB (GB)**
• **GRAU, Vicente**
**Botley Oxford OX2 0JB (GB)**
• **HANDA, Ashok**
**Botley Oxford OX2 0JB (GB)**

(74) Representative: **HGF**
**HGF Limited
1 City Walk
Leeds LS11 9DX (GB)**

(56) References cited:
**US-A1- 2016 203 288**

• **GARCIA-GARCIA FERNANDO ET AL: "Prognosis of Abdominal Aortic Aneurysms: A Machine Learning-Enabled Approach Merging Clinical, Morphometric, Biomechanical and Texture Information", 2017 IEEE 30TH INTERNATIONAL SYMPOSIUM ON COMPUTER-BASED MEDICAL SYSTEMS (CBMS), IEEE, 22 June 2017 (2017-06-22), pages 463 - 468, XP033253559, DOI: 10.1109/CBMS.2017.158**
• **JUDY SHUM ET AL: "Quantitative Assessment of Abdominal Aortic Aneurysm Geometry", ANNALS OF BIOMEDICAL ENGINEERING, KLUWER ACADEMIC PUBLISHERS-PLENUM PUBLISHERS, NE, vol. 39, no. 1, 2 October 2010 (2010-10-02), pages 277 - 286, XP019855119, ISSN: 1573-9686, DOI: 10.1007/S10439-010-0175-3**

(Cont. next page)

- AKKOYUN EMRAH ET AL: "Predicting abdominal aortic aneurysm growth using patient-oriented growth models with two-step Bayesian inference", COMPUTERS IN BIOLOGY AND MEDICINE, NEW YORK, NY, US, vol. 117, 13 January 2020 (2020-01-13), XP086049484, ISSN: 0010-4825, [retrieved on 20200113], DOI: 10.1016/J.COMPBIOMED.2020.103620
- WU WEI ET AL: "Wall Stress and Geometry Measures in Electively Repaired Abdominal Aortic Aneurysms", ANNALS OF BIOMEDICAL ENGINEERING, SPRINGER US, NEW YORK, vol. 47, no. 7, 8 April 2019 (2019-04-08), pages 1611 - 1625, XP036794790, ISSN: 0090-6964, [retrieved on 20190408], DOI: 10.1007/S10439-019-02261-W
- KIMURA MASARU ET AL: "Geometric analysis of ruptured and nonruptured abdominal aortic aneurysms", JOURNAL OF VASCULAR SURGERY, ELSEVIER, AMSTERDAM, NL, vol. 69, no. 1, 15 June 2018 (2018-06-15), pages 86 - 91, XP085562436, ISSN: 0741-5214, DOI: 10.1016/J.JVS.2018.04.035
- GOERTZ LUKAS ET AL: "Angiographic Characteristics of Lobulated Intracranial Aneurysms", WORLD NEUROSURGERY, ELSEVIER, AMSTERDAM, NL, vol. 131, 27 July 2019 (2019-07-27), XP085878048, ISSN: 1878-8750, [retrieved on 20190727], DOI: 10.1016/J.WNEU.2019.07.163

## Description

## Technical Field

The present disclosure relates to methods, apparatuses and computer readable media for estimating a growth rate of an aortic aneurysm.

## Background

Aneurysms, for example abdominal aortic aneurysms (AAAs), are associated with biological changes in the vasculature, features of systemic inflammation and endothelial dysfunction. Rupture of an AAA results in high mortality even with emergency surgery. AAAs may present in a subject in a variety of sizes. An AAA is typically defined as a region of an abdominal aortic artery having an outer aortic diameter greater than 30 millimetres. If the outer diameter of the aorta in the region of the aneurysm exceeds 55 millimetres, the abdominal aortic artery is considered to be large. Surgery to treat AAAs is usually considered where an AAA is identified having a diameter greater than 55 millimetres because the risk of rupture of the AAA is greater than the traditional risks associated with surgery. In current practice, surgery is typically not recommended where the identified AAA has a diameter of less than 55 millimetres as the risks of surgery are generally considered to outweigh the risk of aneurysm rupture. Currently, where an AAA is identified in a patient, and the AAA has a size between approximately 30 millimetres and 55 millimetres, the size of the AAA is monitored over time. Aneurysms may grow at different rates, if at all. If the aneurysm grows to exceed 55 millimetres, then surgery may need to be performed to treat the aneurysm.

Accordingly, the threshold at which a surgical intervention will be made is usually defined by the size of the AAA and not the biological behaviour of the AAA within the affected individual. However, it has been found that such an approach has intrinsic shortcomings because aneurysm size may not be an absolute predictor of the risk of rupture. Furthermore, the rate of AAA progression may vary significantly between individuals. For example, there may be a risk of rupture in those individuals with relatively smaller AAAs (e.g. 35-55mm or 40-55mm), and many patients with an initially small/moderate size AAA may progress and require surgery within 5 years. This may be an important consideration in ageing populations, as the risks associated with undergoing interventions increase with age. The growth rate of an AAA is a good indicator as to whether surgical intervention is required.

There is currently no well-established, non-invasive means for the prediction of aneurysm growth, which can be used to predict if and when surgical intervention will be required.

The present disclosure provides at least an alternative to the methods of analysing aneurysms of the prior art by providing means and methods for predicting the growth rate of an aneurysm.

US2016203288A1 discloses analysing aneurysms and finding morphological factors such as undulation index, and is concerned with prediction of rupture.

## Summary

According to an aspect of the present disclosure, a computer-implementable method is provided for predicting a growth rate of an aortic aneurysm. The method comprises analysing one or more geometric measures of a volumetric model of at least a portion of an aorta having an aortic aneurysm. The method further comprises determining, from the analysis, a growth rate prediction for the aortic aneurysm.

The growth rate prediction may comprise a categorical growth rate prediction.

The growth rate prediction may comprise a continuous growth rate prediction.

A geometric measure of the one or more geometric measures may comprise a measure of the curvature of a path through the volumetric model. For example, a geometric measure of the one or more geometric measures may comprise a measure of the centreline curvature of the volumetric model. The geometric measure of the one or more geometric measures may comprise a radius of curvature of the path through the volumetric model, for example the radius of curvature of the centreline of the volumetric model. The geometric measure of the one or more geometric measures may comprise a measure of a branchpoint angle at a branchpoint of the volumetric model.

A geometric measure of the one or more geometric measures of the volumetric model may comprise a shape-based measure of the aneurysm. The geometric measure of the one or more geometric measures may comprise a measure of the ellipticity of the portion of the aorta having the modelled aneurysm. A geometric measure of the one or more geometric measures of the volumetric model may comprise a measure of the surface irregularity of the volumetric model. The geometric measure of the one or more geometric measures may comprise a measure of a comparison of the volumetric model with a convex hull. The geometric measure may comprise an undulation index.

A geometric measure of the one or more geometric measures of the volumetric model may comprise a function of a measure of surface irregularity of the modelled aneurysm and a measure of the centreline curvature of the volumetric model.

A geometric measure of the one or more geometric measures of the volumetric model may comprise measures derived using principal component analysis, PCA. For example, the PCA derived measures may comprise shape-based modes extracted from the volumetric model, where extracting the shape-based modes may comprise extracting a centreline from the volumetric model, generating planes orthogonal to the centreline, determining a maximum diameter of each orthogonal plane, and performing PCA on a set of coordinates corresponding to the extracted centreline and the maximum diameter of each orthogonal plane. As another example, the PCA derived measures may comprise surface-based modes extracted from the volumetric model, where extracting the surface-based modes may extracting a surface line from the volumetric model by generating a curve along the aortic surface, and performing PCA on a set of coordinates corresponding to the extracted surface line. As another example, the PCA derived measures may comprise branch-point, BP, modes, extracted from the volumetric model, where extraction of the BP modes may comprise extracting a set of branch-point coordinates corresponding to regions along the volumetric model where an aortic side branch originates; and performing PCA on the extracted set of branch-point coordinates.

Determining a growth rate prediction for the aortic aneurysm may comprise comparing the analysed one or more geometric measures with one or more known values, wherein the one or more known values correspond to a geometric measure of a volumetric model of a portion of an aorta having a reference aneurysm for which the growth rate of reference aneurysm is substantially known.

Determining a growth rate prediction for the aortic aneurysm may comprise using a regression model and the analysed one or more geometric measures to determine the growth rate prediction.

The volumetric model may comprise a 3D model.

The method may further comprise receiving the volumetric model of the portion of the aorta having the aneurysm.

The method may further comprise generating the volumetric model. The volumetric model may be generated based on computed tomography, CT, image data.

Generating the volumetric model may comprise receiving a plurality of CT image slices, the CT image slices showing the portion of the aorta having an aneurysm. Generating the volumetric model may further comprise segmenting the aorta including the aneurysm in each CT image slice. Generating the volumetric model may further comprise constructing the volumetric model from the segmented images.

Generating the volumetric model may comprise receiving computed tomography, CT, image data, the CT image data showing the portion of the aorta having an aortic aneurysm. Generating the volumetric model may further comprise inputting the received CT image data into a trained machine learning model for identifying features of the aorta and aneurysm within CT image data. Generating the volumetric model may further comprise producing, from the output of the trained machine learning model, a volumetric model of the portion of the aorta having the aneurysm.

The aneurysm may comprise an abdominal aortic aneurysm.

According to an aspect of the present disclosure, an apparatus is provided herein, the apparatus for estimating a growth rate of an aortic aneurysm. The apparatus comprises a memory having instructions stored thereon. The apparatus further comprises a processor configured to execute the instructions stored in the memory to perform a method as described herein.

According to an aspect of the present disclosure, a computer-readable medium is provided. The computer-readable medium has instructions formed thereon which, when executed by a processor, cause the processor to perform a method as described herein. The computer-readable medium may comprise a non-transitory computer-readable medium.

The computer program and/or the code for performing such methods as described herein may be provided to an apparatus, such as a computer, on the computer readable medium or computer program product. The computer readable medium could be, for example, an electronic, magnetic, optical, electromagnetic, infrared, or semiconductor system, or a propagation medium for data transmission, for example for downloading the code over the Internet. Alternatively, the computer readable medium could take the form of a physical computer readable medium such as semiconductor or solid state memory, magnetic tape, a removable computer diskette, a random access memory (RAM), a read-only memory (ROM), a rigid magnetic disc, and an optical disk, such as a CD-ROM, CD-R/W or DVD.

Many modifications and other embodiments of the inventions set out herein will come to mind to a person skilled in the art to which these inventions pertain in light of the teachings presented herein. Therefore, it will be understood that the disclosure herein is not to be limited to the specific embodiments disclosed herein. Moreover, although the description provided herein provides example embodiments in the context of certain combinations of elements, steps and/or functions may be provided by alternative embodiments without departing from the scope of the invention.

**Brief Description Of The Drawings**

Embodiments of the invention are further described hereinafter with reference to the accompanying drawings, in which:

Figure 1 illustrates a first geometric measure, the radius of curvature, usable with a volumetric model of at least a portion of an artery having an aneurysm;
Figure 2 shows three illustrations of volumetric models of aneurysmal regions, each volumetric model shown with its respective centerline and minimum radius of curvature;
Figure 3 shows three illustrations of volumetric models of aneurysmal regions, each volumetric model shown with its respective convex hull and corresponding undulation index;

Figure 4 shows violin plots of the annual growth rates of a plurality of aneurysms as determined from serial CT scans;

Figure 5 shows violin plots of the annual growth rates of the plurality of aneurysms of Figure 4 assigned to a training cohort and a testing cohort;

Figure 6 shows box plots of the initial maximum anteroposterior (AP) diameter of the aneurysms in the training cohort and the testing cohort;

Figure 7 shows a scatter plot of the annual growth rate against the initial maximum AP diameter for the training cohort;

Figure 8 shows box plots of the follow-up time between scans for each aneurysm in the training cohort and in the testing cohort;

Figure 9 shows a scatter plot of the annual growth rate against the follow-up time for the training cohort;

Figure 10 shows a scatter plot of the annual growth rate against the undulation index for the aneurysms of the training cohort;

Figure 11 shows box plots of the undulation indices of the aneurysms in the training cohort and the testing cohort;

Figure 12 shows four illustrations of volumetric models of aneurysmal regions of the training cohort, each volumetric model shown with its respective convex hull and corresponding undulation index and growth rate;

Figure 13 shows a scatter plot of the annual growth rate against the minimum radius of curvature for the AAAs of the training cohort;

Figure 14 shows box plots of the minimum radius of curvature of the aneurysms in the training cohort and the testing cohort;

Figure 15 shows four illustrations of volumetric models of aneurysmal regions of the training cohort, each volumetric model shown with its centerline and corresponding minimum radius of curvature and growth rate;

Figure 16 shows a violin plot of the annual growth rate of a plurality of aneurysms in a training cohort with dashed lines demarcating growth rate categories;

Figure 17 shows box plots of the annual growth rate of the aneurysms of the training cohort analysed in Figure 16 for each growth rate category/subgroup;

Figure 18 shows box plots of the initial maximum AP diameter of the aneurysms of each subgroup of the training cohort for each growth rate category/subgroup;

Figure 19 shows box plots of the follow-up times for the aneurysms of each subgroup of the training cohort for each growth rate category/subgroup;

Figure 20 shows a table indicating the mean and standard deviation initial maximum AP diameter and mean and standard deviation follow-up time for the aneurysms of each subgroup of the training cohort;

Figure 21 shows box plots of the undulation index for the aneurysms of each subgroup of the training cohort;

Figure 22 shows box plots of the minimum radius of curvature for the aneurysms of each subgroup of the training cohort;

Figure 23 shows a table of indicating the relative merits of initial AP diameter, aneurysm volume, undulation index, and minimum radius of curvature in predicting aneurysmal growth rates;

Figure 24 shows ROC curves indicating the true positive rates and false positive rates for several combinations of geometric measures as applied to the "greater than 5.0 mm" subgroup of the testing cohort;

Figure 25 shows ROC curves indicating the true positive rates and false positive rates for several geometric measures as applied to the "less than 5.0 mm" subgroup of the testing cohort;

Figure 26 shows a scatter plot of the predicted growth rate (in mm per year) of the 100 aneurysms of the training cohort as predicted by a first linear regression model against the actual growth rate of the aneurysms;

Figure 27 shows a scatter plot of the predicted growth rate of the 92 aneurysms of the testing cohort as predicted by the first linear regression model against the actual growth rate of the aneurysms;

Figure 28 shows a scatter plot of the predicted growth rate (in mm per year) of the 100 aneurysms of the training cohort as predicted by a second linear regression model against the actual growth rate of the aneurysms;

Figure 29 shows a scatter plot of the predicted growth rate of the 92 aneurysms of the testing cohort as predicted by the second linear regression model against the actual growth rate of the aneurysms;

Figure 30 shows a scatter plot of the predicted growth rate (in mm per year) of the 100 aneurysms of the training cohort as predicted by a third linear regression model against the actual growth rate of the aneurysms;

Figure 31 shows a scatter plot of the predicted growth rate of the 92 aneurysms of the testing cohort as predicted by the third linear regression model against the actual growth rate of the aneurysms;

Figure 32 shows a scatter plot of the predicted growth rate (in mm per year) of the 100 aneurysms of the training cohort as predicted by a fourth linear regression model against the actual growth rate of the aneurysms;

Figure 33 shows a scatter plot of the predicted growth rate of the 92 aneurysms of the testing cohort as predicted by the fourth linear regression model against the actual growth rate of the aneurysms;

Figure 34 shows a scatter plot of the predicted growth rate (in mm per year) of the 100 aneurysms of the training cohort as predicted by a fifth linear regression model against the actual growth rate of the aneurysms;

Figure 35 shows a scatter plot of the predicted growth rate of the 92 aneurysms of the testing cohort as predicted by the fifth linear regression model against the actual growth rate of the aneurysms;

Figure 36 shows a scatter plot of the predicted growth rate (in mm per year) of the 100 aneurysms of the training cohort as predicted by a sixth linear regression model against the actual growth rate of the aneurysms;

Figure 37 shows a scatter plot of the predicted growth rate of the 92 aneurysms of the testing cohort as predicted by the sixth linear regression model against the actual growth rate of the aneurysms;

Figure 38 shows a scatter plot of the predicted growth rate (in mm per year) of the 100 aneurysms of the training cohort as predicted by a seventh linear regression model against the actual growth rate of the aneurysms;

Figure 39 shows a scatter plot of the predicted growth rate of the 92 aneurysms of the testing cohort as predicted by the seventh linear regression model against the actual growth rate of the aneurysms;

Figure 40 shows a table summarising the properties of the seven regression models used in conjunction with Figures 26-39;

Figure 41 shows a flow chart of a method for predicting a growth rate of an aneurysm using PCA derived geometric features;

Figure 42 shows the AAA centrelines before and after re-orientation;

Figure 43 shows an example of centreline extraction and orthogonal plane extraction for four different AAA volumetric models;

Figures 44A-B show examples of graphs relating to the x, y and z coordinates against points (pts) and the maximum orthogonal diameter measurements (mm) against points (pts);

Figure 45 shows examples of graphs relating to the x, y and z coordinates against points (pts) and the maximum orthogonal diameter measurements (mm) against points (pts) for 192 patients;

Figure 46 shows a flowchart for performing PCA;

Figure 47 shows the cumulative variance captured by the derived PCA modes and reconstruction of AAA;

Figure 48 shows a bar graph relating to the AUROC scores obtained using models IV-VI and tables which summarise the AUROC scores and the features used to train models IV-VI for outcome 1 (slow vs not-slow growth) and outcome 2 (fast vs not-fast growth);

Figures 49A-E illustrate the performance of the different variables relating to PCA derived shape-based modes 3 and 7 and geometric measures (UI, RC and maximum diameter) used to train the models IV-VI for predicting outcome 1 (slow vs not-slow growth);

Figures 50A-E illustrate the performance of the different variables relating to PCA derived shape-based modes 1 and 4 and geometric measures (UI, RC and maximum diameter) used to train models IV-VI for predicting outcome 2 (fast vs not-fast growth);

Figure 51 shows a flow chart of a method for predicting a growth rate of an aneurysm using PCA derived geometric features;

Figure 52 shows spline curve segmentation and interpolation of points within the spline curve for 3 axial slices and also a bar chart relating to the spacing between the interpolated points;

Figure 53 shows the generation of surface lines for three adjacent axial slices;

Figure 54 shows four volumetric models of an aorta with centrelines and surface lines shown for each of the four volumetric models;

Figure 55 shows the x, y and z coordinates for 5 extracted surface lines for all patients;

Figure 56 shows a flowchart for performing PCA;

Figure 57 shows a bar graph relating to the AUROC scores obtained using models VII-IX and tables which summarise the AUROC scores and the features used to train models VII-IX for outcome 1 (slow vs not-slow growth) and outcome 2 (fast vs not-fast growth);

Figures 58A-E illustrate the performance of the different variables relating to PCA derived surface-based mode 6 and geometric measures (UI, RC and maximum diameter) used to train the models VII-IX for predicting outcome 1 (slow vs not-slow growth);

Figures 59A-E illustrate the performance of the different variables relating to PCA derived surface-based mode 3 and geometric measures (UI, RC and maximum diameter) used to train the models VII-IX for predicting outcome 2 (fast vs not-fast growth);

Figure 60 shows a flowchart of a method for extracting the locations of the aortic side branches;

Figure 61 shows the classification of the aortic side branches;

Figure 62 shows four expanded segmentations of a volumetric model of AAA with classified branch centrelines and the branch origins;

Figure 63 shows a flowchart of a third method for predicting a growth rate of an aortic aneurysm using PCA derived geometric features;

Figure 64 shows a PCA input matrix and examples of the x, y and z coordinates for the celiac artery and the SMA;

Figure 65 shows a flowchart for performing PCA;

Figure 66 shows a bar graph relating to the AUROC scores obtained using models X-XII and tables which summarise the AUROC scores and the features used to train models X-XII for outcome 1 (slow vs not-slow growth) and outcome 2 (fast vs not-fast growth);

Figures 67A-E illustrate the performance of the different variables relating to PCA derived BP mode 1 and geometric measures (UI, RC and maximum diameter) used to train models X-XII for predicting outcome 1 (slow vs not-slow growth).

Figures 68A-E illustrate the performance of the different variables relating to PCA derived BP mode 1 and geometric measures (UI, RC and maximum diameter) used to train models X-XII for predicting outcome 2 (fast vs not-fast growth);

Figure 69 shows a bar graph relating to the AUROC scores obtained using models XIII-XV and tables which summarise the AUROC scores and the features used to train models XIII-XV for outcome 1 (slow vs not-slow growth) and outcome 2 (fast vs not-fast growth);

Figures 70A-E illustrate the performance of the different variables relating to PCA derived shape-based modes 3 and 7, surface-based mode 6, BP mode 10 and geometric measures (UI, RC and maximum diameter) used to train models X-XIV for predicting outcome 1 (slow vs not-slow growth);

Figures 71A-E illustrate the performance of the different variables relating to PCA derived shape-based mode 4, surface-based mode 3, BP mode 3 and geometric measures (UI, RC and maximum diameter) used to train models X-XIV for predicting outcome 2 (fast vs not-fast growth);

Figure 72 shows a table relating to model XV which highlights the model performance using AUROC. The coefficients and p-values for each of the variables within the optimized logistic regression models as well as their performance are also highlighted in the table;

Figures 73A-G illustrate the performance of the variables used to train model XV for predicting outcome 1 (slow vs not-slow growth);

Figures 74A-G illustrate the performance of the used to train model XV for predicting outcome 2 (fast vs not-fast growth);

Figure 75 shows the performance of four linear regression models (A-D) at predicting a continuous AAA growth rate;

Figure 76 shows tables I-IV with the feature coefficients corresponding to models A-D trained to predict AAA continuous growth;

Figures 77A-B show the relevance of mode 3 (shape-based PCA) on the average AAA shape and its impact on aneurysmal growth (slow vs not-slow growth);

Figure 78A-B show the relevance of mode 7 (shape-based PCA) on the average AAA shape and its impact on aneurysmal growth (slow vs not-slow growth);

Figures 79A-B show the relevance of mode 4 (shape-based PCA) on the average AAA shape and its impact on aneurysmal growth (fast vs not-fast growth;

Figures 80A-B show the influence of BP mode 10 on the average branch locations and its impact on aneurysmal growth (Slow vs Not-Slow Growth);

Figure 81A-B show the influence of BP mode 3 on the average branch locations and its impact on aneurysmal growth (Fast vs Not-Fast Growth);

Figure 82 shows a flow chart of a method for predicting a growth rate of an aneurysm;

Figure 83 shows a block diagram of a computing apparatus; and

Figure 84 shows a diagram of a computer readable medium.

Throughout the description and drawings, like reference numerals refer to like parts.

## Detailed Description

The present invention provides computer-implementable methods and systems for improved prediction of growth rates of aortic aneurysms. Whilst various embodiments of the invention are described below, the invention is not limited to these embodiments, and variations of these embodiments may be made without departing from the scope of the invention.

A computerised tomography (CT) scan, sometimes referred to as a CAT scan, is a diagnostic imaging procedure which uses x-rays impinging on a subject, such as the human body, to produce cross-sectional images, sometimes called slices, of a targeted region of the subject. The CT images are usually captured at a range of angles about the subject. The cross-sectional slices are then collated to produce a detailed three-dimensional image of the targeted region of the subject, which can be used to diagnose conditions including damage to bones, injuries to internal organs, problems with blood flow, stroke, and cancer.

An abdominal aortic aneurysm (AAA) is an example of a condition that may be diagnosed using CT images obtained from a CT scan. AAA is a bulging, dilation, or ballooning in the wall of the abdominal aorta, caused due to weakness or degeneration that develops in a portion of the aorta. Due to the constant pressure on the walls of the abdominal aorta, the aneurysm enlarges, stretching the walls of the artery thinner, thereby compromising the artery wall's ability to stretch any further. At this point, the aneurysm is at risk of rupturing and causing potentially fatal bleeding, just as a balloon will pop when blown up too much. Images obtained from a CT scan can enable medical/surgical professionals to monitor the growth of the aneurysm in the patient and/or make plans for surgical repair of the aneurysm. Of course, CT scans are also beneficial in diagnosing and treating other conditions. In particular, CT angiograms are widely utilised in all fields of cardiovascular surgery/medicine.

In order to make blood vessels such as the aorta visible on a CT image, a radiocontrast agent (hereafter referred to as a contrast agent) can be introduced into the patient. As the radiodensity of blood and the surrounding tissue is similar it can be difficult for the human eye to distinguish the interface between blood vessels and the surrounding tissue on CT images obtained without a contrast agent. The introduction of a contrast agent helps distinguish or "contrast" selected areas of the body from the surrounding tissue.

In the case of an aortic aneurysm, the aorta often contains an intra-luminal thrombus within the aneurysm sac and full visualisation of the thrombus morphology, and its relation to the artery wall is important for monitoring the growth of the aneurysm and/or making plans for surgical repair of the aneurysm.

Current clinical guidelines for the management of AAAs are based on criteria readily derived from the maximum diameter. The rationale behind the use of this singular parameter arises from the Young-Laplace equation, which states that wall tension in regular, symmetric and thin-walled spheres is directly proportional to their radii. Therefore, aneurysms larger than 55 mm and those with an expansion rate $\geq$ 10 mm/year are recommended for surgical intervention. Aneurysmal screening by measuring this parameter is a cost-effective modality to reduce the incidence of AAA rupture and is being increasingly adopted in many countries.

However, aneurysmal growth is a complex process that is poorly understood and requires significant exploration. AAAs are often asymmetric, tortuous, and the intra-luminal thrombus can have a varied thickness and density. Furthermore, AAA diameter is a crude unidimensional measurement of growth and in many instances remains constant despite significant changes in volume and morphology. This complicates the model assumed by the implementation of the Young-Laplace equation and illustrates the difficulty of individualizing surveillance protocols.

Against this backdrop, the inventors have hypothesised that certain patient-specific geometric and volumetric measurements of an AAA may more readily influence AAA growth rates. As small AAAs enlarge, a variety of geometrical changes have been observed to either promote rupture risk or growth deceleration. Isolating and deciphering these changes may allow a medical/surgical professional to predict AAA growth and progression in each patient.

As has been explained above, a CT scan may be used to generate a 3D model of a targeted region of a subject. Traditionally, reconstruction of the aorta with the aneurysmal outer wall is a prerequisite for the extraction of many relevant parameters such as the diameter of an AAA or a blood vessel. Manual segmentation, although possible, is tedious, time-consuming and dependent on the training of the user. In addition, semi-automatic segmentation methods using open-source or existing commercially available software are limited to that of the inner aortic wall. Nevertheless, such methods may be used to construct a volumetric model of at least a portion of an artery having an aneurysm. Other methods of identifying structural features of a portion of an artery have been described elsewhere, for example in international patent application number PCT/GB2020/052013, entitled "Computerised Tomography Image Processing", filed on 21 August 2020, and in international patent application number PCT/GB2020/052014, entitled "Computerised Tomography Image Processing", filed on 21 August 2020.

CT image slices may be segmented using a dedicated algorithm to generate a volumetric model of at least a portion of an artery having an aneurysm.

As described herein, one or more geometric measures of a volumetric model of at least a portion of an aorta having an aneurysm may be analysed in order to determine a growth rate prediction for the aneurysm. As small AAAs enlarge, a variety of geometrical changes are observed including altered aortic tortuosity, branch-point angulation, aneurysmal asymmetry and intra-luminal thrombus growth, and the inventors have hypothesised that many of these changes promote aortic instability and increase the risk of rupture.

In addition to above, the inventors have investigated if principal component analysis (PCA) can be used to characterize the aneurysmal shape or associated features and provide additional growth predictive capacity. PCA is a commonly-used method for dimensionality reduction. It is performed by projecting each data point onto a range of principal components to obtain lower-dimensional data while preserving as much of the data's variation as possible. In this instance, the inventors hypothesized that most of the diversity or variance of the aneurysmal region can be captured using PCA and that the reduced principal components, which may describe a certain aspect of the AAA geometry, may be helpful in predicting AAA growth. One of the advantages of this approach is its ability to highlight features/relationships without any implicit user bias. Additionally, this framework allows for the ability to interpret the relevant features, which is extremely valuable in the clinical setting. The approach relating to deriving PCA based geometric features is described in relation to Figures X-X below.

A volumetric model of at least a portion of an artery having an aneurysm is understood to mean data representative of the volume of at least a part of the portion of the artery. For example, the volumetric model may comprise at least a partial 3D model of the aneurysmal region. The volumetric model may comprise a plurality of 2D images of the aneurysmal region from which the volume information can be derived.

Aneurysmal growth is often described with reference to the maximum diameter of the aneurysm. A growth rate prediction may be understood to mean a predicted rate of growth of the maximum AP diameter of the aneurysm, and this is the growth rate described herein. However, the skilled person would appreciate that the growth rate may refer to other metrics of the aneurysm such as the aneurysm volume.

According to examples described herein, a geometric measure of the one or more geometric measures may comprise a measure of the centreline curvature of the volumetric model. The centreline may be considered as the central curve through the volumetric model and may represent the centralised flow of blood through the modelled portion of the artery. The inventors have hypothesised that centreline curvature is correlated with aneurysmal growth and progression. In particular, it is thought that regions of increased curvature of the centreline in the aneurysmal region correlate with altered hemodynamic forces, which in turn lead to growth of the aneurysm and disease progression.

While the central path through the modelled artery (centreline) is discussed herein, the skilled person would appreciate that a geometric measure may comprise a measure of the curvature of any path through the volumetric model that may represent a path along which blood may flow through the modelled portion of the artery.

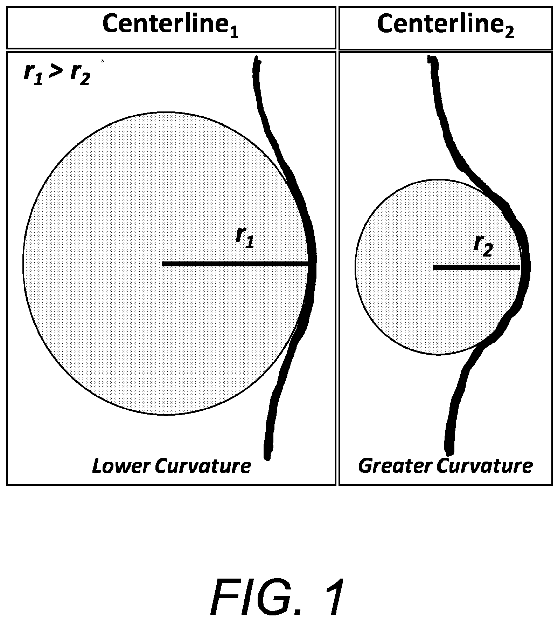

One example of a geometric measure of the centreline curvature (or of the curvature of any other path through the volumetric model) is the radius of curvature of the path (and the link between this measure and aneurysmal growth rate is demonstrated further in this description). The radius of curvature at a point on a curve is equal to the radius of the circular arc which best approximates the curve at that point. Figure 1 illustrates what is meant by the radius of curvature. In particular, in Figure 1, two centerlines are illustrated (Centerline; and Centerline$_2$). Centerline$_1$ has a lower curvature than Centerline$_2$ and accordingly the radius of the first circle $r_1$ is greater than the radius of the second circle $r_2$. The radius of curvature may be calculated at multiple points along a path through a volumetric model and the minimal value of the radius of curvature (minimum radius of curvature) may be taken as a measure of the curvature of the path through the volumetric model.

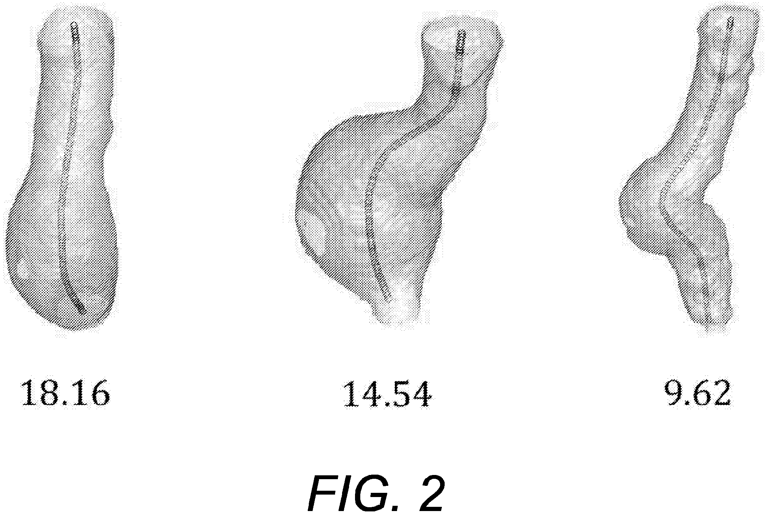

Figure 2 shows three representations of volumetric models of portions of aortas having aneurysms. In each model the centreline is also indicated, and the value of the minimum radius of curvature is indicated below the respective model. The leftmost volumetric model has a minimum radius of curvature of 18.16. The central volumetric model has a minimum radius of curvature of 14.54. The rightmost volumetric model has a minimum radius of curvature of 9.62. As can be seen from the figure, the centreline curvature of the rightmost volumetric model is more curved than the central volumetric model, which is in turn more curved than the leftmost volumetric model.

According to examples described herein, a geometric measure of the one or more geometric measures may comprise a shape-based measure of the aneurysm. For example, the geometric measure may comprise a measure of the surface irregularity of the aneurysmal wall. The inventors have hypothesised that surface irregularity is correlated with aneurysmal growth and progression. In particular, it is thought that regions of surface irregularity in the aneurysmal region correlate with altered hemodynamic forces, which in turn lead to growth of the aneurysm and disease progression.

One example of a measure of the surface irregularity is the undulation index (UI) which is a measure of the degree of surface concavity and is given by:

$$\text{UI} = 1 - \frac{Volume_{AN}}{Volume_{CH}}$$

In this equation, the quantity $Volume_{AN}$ refers to the volume of the modelled aneurysm in the volumetric model. The quantity $Volume_{CH}$ refers to the volume of the convex hull that can encompass the modelled aneurysm in the volumetric model. The convex hull or convex envelope or convex closure of a shape is the smallest convex set that contains that shape. The undulation index for a volumetric model of an aneurysm is one minus the ratio of the volume of the modelled aneurysm to the volume of the convex hull for that aneurysm.

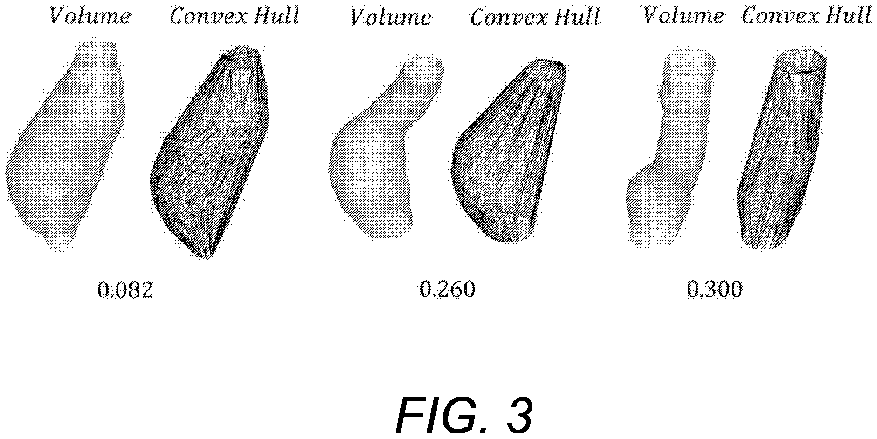

Figure 3 illustrates three representations of volumetric models (labelled as "volume" in the Figure) and their corresponding convex hulls. The value of the undulation index is indicated below the respective volumetric model. The leftmost volumetric model has an undulation index of 0.082. The central volumetric model has an undulation index of 0.260. The rightmost volumetric model has an undulation index of 0.300.

In what follows, it will now be demonstrated that such geometric measures can be used to estimate the growth rate of an aneurysm.

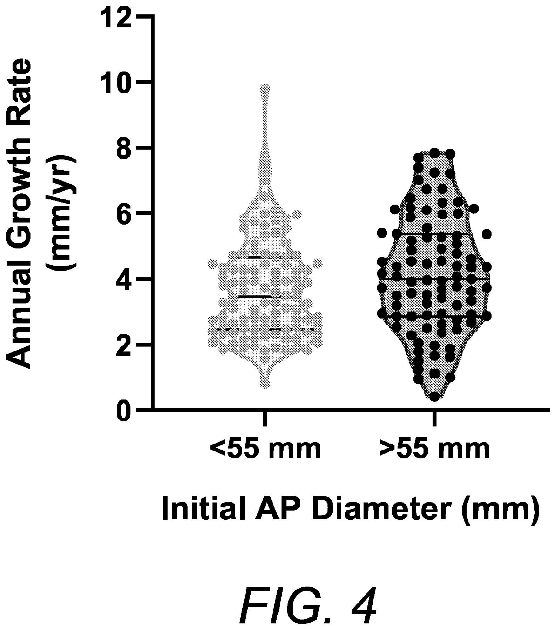

A sample of 202 AAA patients with serial CT scans was analysed. Of those 202 patients, 192 patients had had serial CT scans of the AAA 8 months apart or greater, and the data from these 192 patient scans was used. Of these 192 patients, 100 patients had an initial anteroposterior (AP) aneurysm diameter of less than 55 mm and 92 patients had an initial AP diameter of greater than 55 mm. The "actual" growth rate of the aneurysm (in mm/year) is calculated for each patient based on the difference between the AP diameter of the aneurysm determined from the second CT scan and the AP diameter of the aneurysm in the initial CT scan. Figure 4 shows the annual growth rate of the aneurysm plotted for each of the 192 patients, with those patients for which the initial AP diameter was less than 55 mm separated from those patients for which the initial diameter was greater than 55 mm.

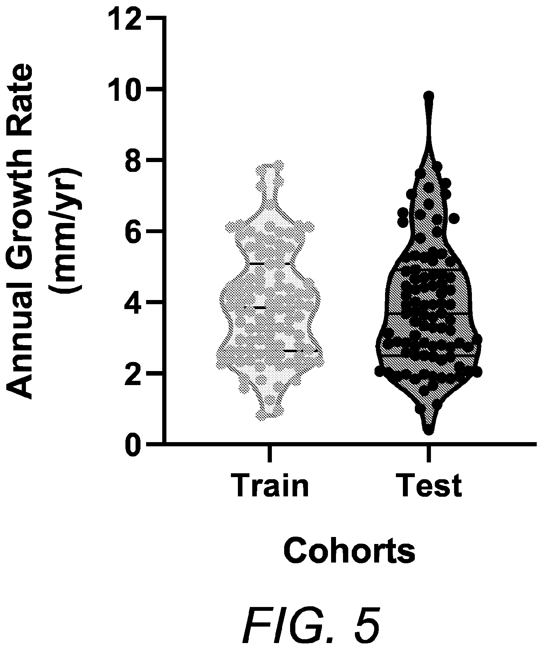

Of the 192 patients, 100 were randomly assigned to a training cohort and the remaining 92 were assigned to a testing cohort. Both cohorts consisted of data from a mix of patients. That is, both cohorts included data from patients having an aneurysm with an initial AP diameter of less than 55 mm and data from patients having an aneurysm with an initial AP diameter of greater than 55 mm. Figure 5 shows the annual growth rate for each patient in the training cohort and each patient in the testing cohort.

The mean initial maximum AP diameter in the training set was 53.6 mm, with a standard deviation of 8.2 mm. The mean annual growth rate for the aneurysms in the training cohort was 3.9 mm per year with a standard deviation of 1.6mm per year. The mean initial maximum AP diameter of the test cohort was 54.7 mm with a standard deviation of 9.7 mm. The mean annual growth rate for the aneurysms in the training cohort was 3.9 mm per year with a standard deviation of 1.8 mm per year.

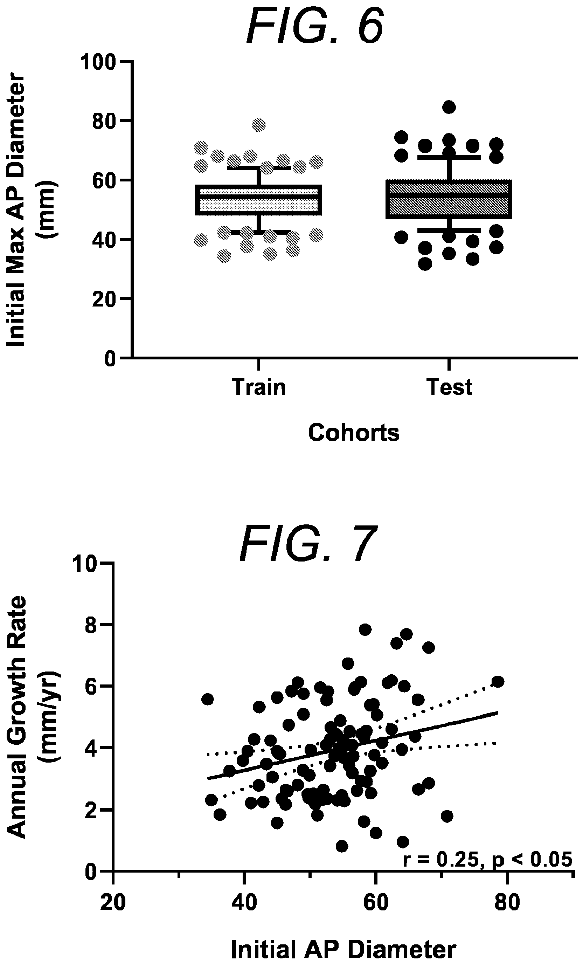

Figure 6 shows box plots of the initial maximum AP diameters of the aneurysms in the training set and the aneurysms in the testing set. The boxes show the 10th percentile of aneurysm diameters in the cohort and the whiskers show the 90th percentile of aneurysm diameters.

Figure 7 is a scatter plot of the annual growth rate against the initial maximum AP diameter for the training cohort. There is a positive correlation between the initial AP diameter and the growth rate in the training cohort, and the Spearman correlation coefficient for these samples is given by r = 0.25 with a p-value of *p* < 0.05. The solid line in Figure 7 indicates this correlation (and has a gradient of 0.25). The dashed lines in Figure 7 indicate the 95% confidence interval.

Accordingly, it is clear that there is some correlation between the initial AP diameter of an aneurysm of the training set and the annual growth rate of the aneurysm.

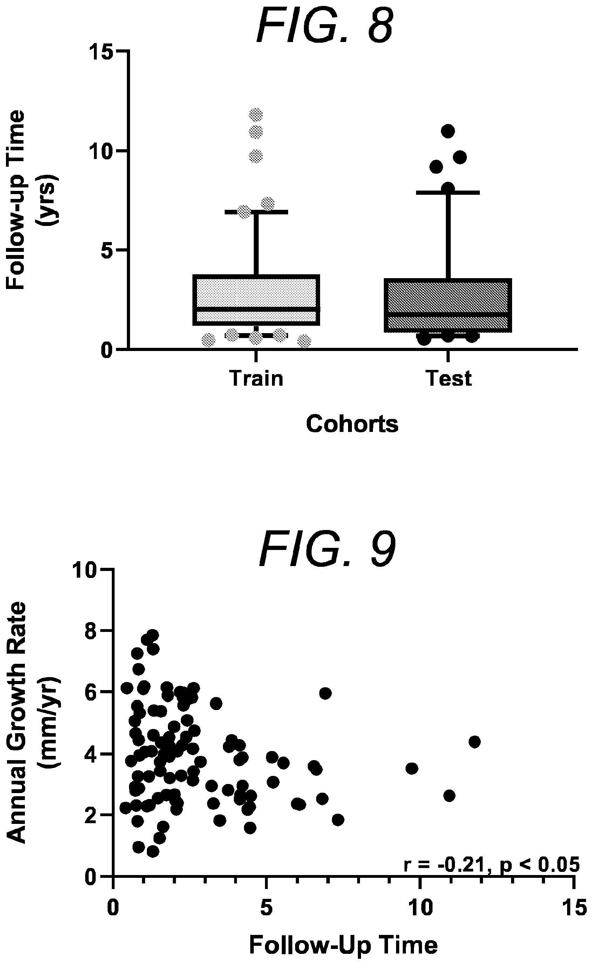

The follow-up time (that is, the time between the scans for each aneurysm) was next considered. For the training cohort, the mean follow-up time for the aneurysm was 2.6 years with a standard deviation of 2.3 years and the mean follow-up time for the test cohort was 2.6 years with a standard deviation of 2.3 years. Figure 8 shows box plots of the follow-up time for the training cohort and the test cohort, with the boxes showing the 10th percentile and the whiskers showing the 90th percentile.

Figure 9 shows a scatter plot of the annual growth rate against the follow-up time for the training cohort. As can be seen from the figure there is a weak negative correlation between the follow-up time and the annual growth rate of the aneurysm. For the training cohort, the Spearman correlation coefficient is given by r = -0.21 with a p-value of *p* < 0.05.

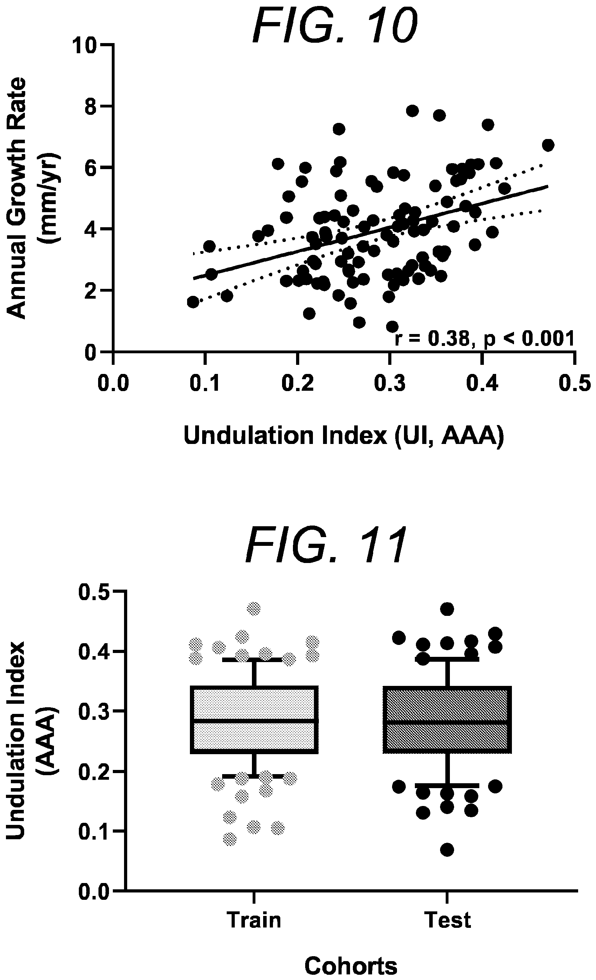

Figure 10 is a scatter plot of the annual growth rate against the undulation index for the training cohort. The undulation index here represents the undulation index for the earlier of the serial scans for each patient. As can be seen, the Spearman correlation coefficient is r = 0.38 with a p-value of *p* < 0.01. There is a stronger positive correlation between the annual growth rate and the undulation index than there is between the annual growth rate and the initial maximum AP diameter for the training cohort. For the training cohort the mean undulation index was 0.28 with a standard deviation of 0.08. For the testing cohort the mean undulation index was also 0.28 with a standard deviation of 0.08. Box plots of the undulation indices for the training and testing cohorts are shown in Figure 11. The boxes show the 10th percentile, and the whiskers show the 90th percentile.

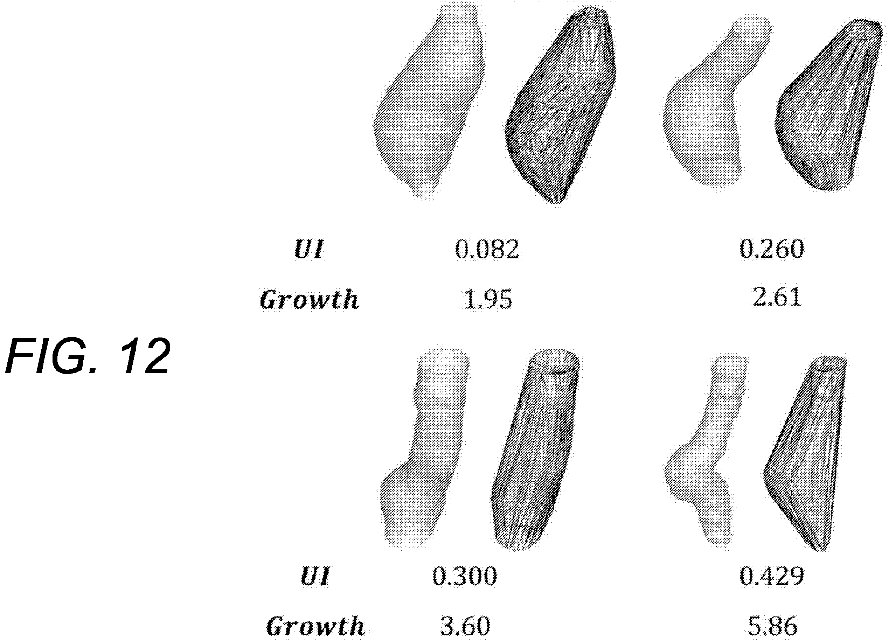

Figure 12 shows illustrations of four volumetric models from the training cohort with their corresponding convex hulls. The volumetric models are based on the earlier of the serial scans. Also shown in Figure 12 is the undulation index for each volumetric model and the respective growth rate in mm per year.

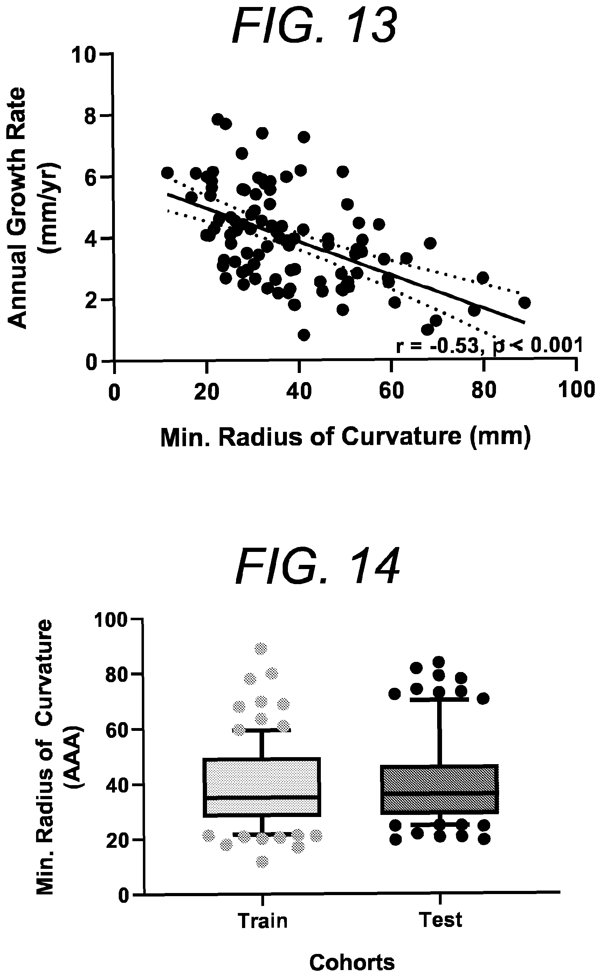

Figure 13 is a scatter plot of the annual growth rate against the minimum radius of curvature of the centreline for the modelled aneurysms of the training set. As can be seen, there is a comparatively strong negative correlation between the minimum radius of curvature of a modelled aneurysm's centreline and the aneurysm growth rate, as indicated by a Spearman correlation coefficient of r = -0.53, with a p-value of p < 0.01. The solid line shows this negative correlation. The dashed lines indicate the 95% confidence interval.

For the training cohort, the mean minimum radius of curvature was 38.9 mm with a standard deviation of 14.3 mm. For the testing cohort, the mean minimum radius of curvature was 41.6 mm with a standard deviation of 17.9 mm. Box plots of the minimum radius of curvature for the training and testing cohorts are shown in Figure 14. The boxes show the 10th percentile, and the whiskers show the 90th percentile.

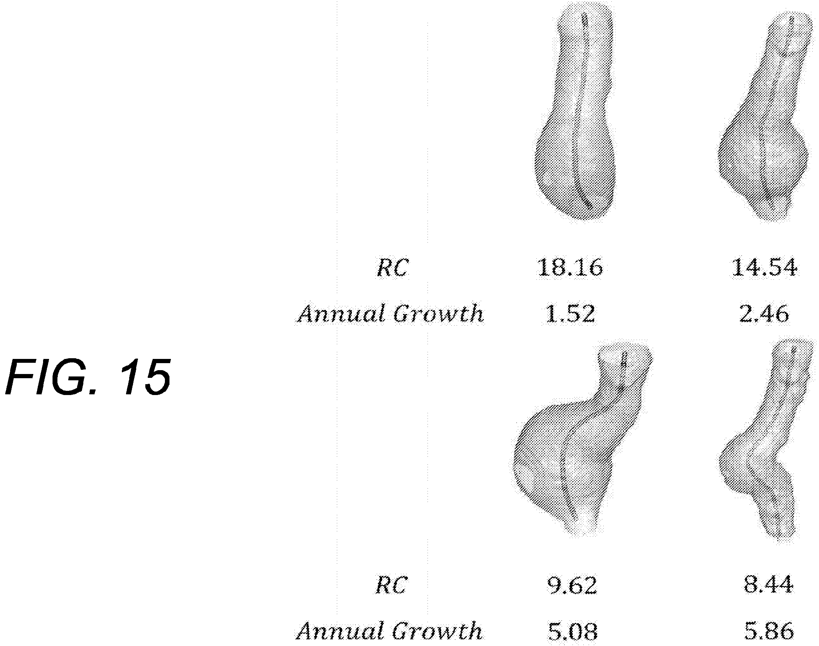

Figure 15 shows illustrations of four volumetric models from the training cohort. The centreline of each volumetric model is visible also. The minimum radius of curvature (denoted "RC") is shown for each of the four volumetric models, along with the respective annual growth rate for that model.

As has been shown from the discussion above in relation to Figures 5-15, there appears to be a clear positive correlation between the annual growth rate of an AAA and the undulation index, and there appears to be a clear negative correlation between the annual growth rate of an AAA and the minimum radius of curvature. These correlations were stronger for the training cohort than the correlations between the annual growth rate and either the initial AP diameter or the time between scans.

The inventors have investigated whether geometric features from aortic aneurysms can be used to predict categorical growth i.e. whether one can predict that the growth rate of an aneurysm will fall into a growth rate category.

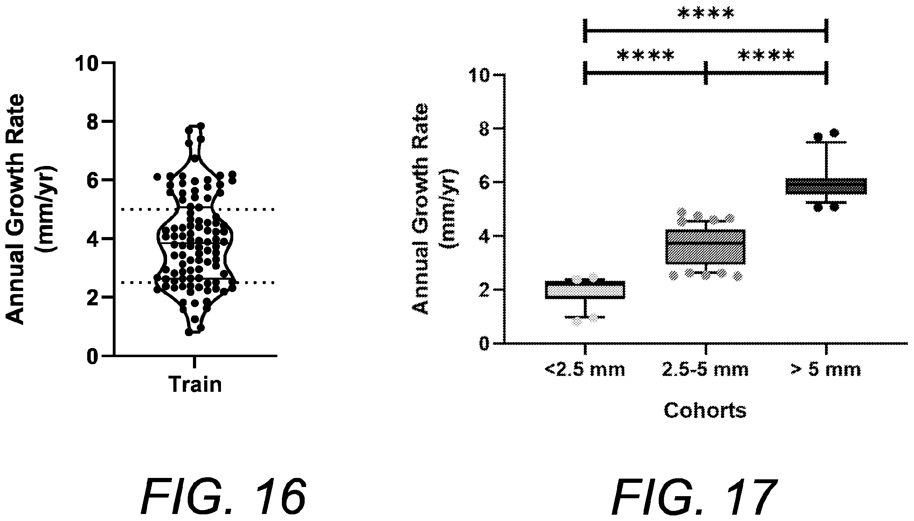

The categories considered were "less than 2.5 mm per year", "2.5-5 mm per year" and "greater than 5 mm per year". Of the training cohort, the scans of 20 patients fell into the "less than 2.5 mm per year" category, with a mean annual growth rate of 2.0 mm per year and a standard deviation of 0.5 mm per year. Of the training cohort, the scans of 54 patients fell into the "2.5-5 mm per year" category, with a mean annual growth of 3.6 mm per year and a standard deviation of 0.7 mm per year. Of the training cohort, the scans of 26 patients fell into the "greater than 5mm per year" category, with a mean annual growth rate of 6.1 mm per year and a standard deviation of 0.8 mm per year. Figure 16 shows a violin plot of the annual growth rate of the 100 samples of the training cohort. The lower region of the plot (below the lower dashed line) is the "less than 2.5 mm per year" category, the middle region (between the dashed lines) is the "2.5-5 mm per year" category, and the upper region (above the upper dashed line) is the "greater than 5 mm per year" category. Figure 17 shows box plots of the annual growth rate of aneurysms in each category/-subgroup in the training cohort. A one way ANOVA was performed with multiple comparisons between each of the categories/subgroups. The horizontal bars (with asterisks) indicate each of the sub-group comparisons that are of statistical significance. In particular, four asterisks indicate a p value of p < 0.001. That is, the one way ANOVA indicates that the groupings of the samples is statistically significant.

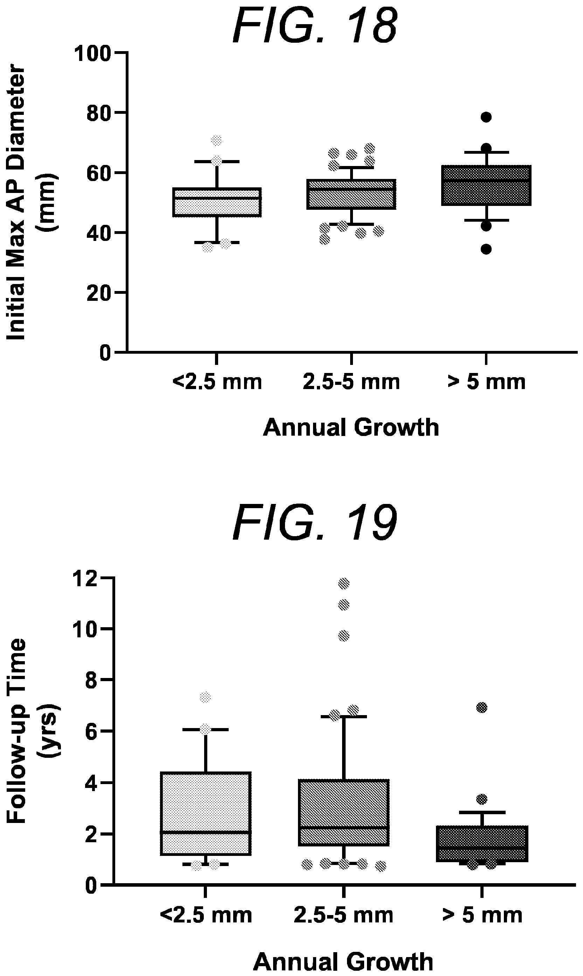

Figure 18 shows box plots of the initial maximum AP diameter of the samples of the training cohort. As can be seen from the figure, there is not a significant difference between initial maximum AP diameters across the three categories.

Figure 19 shows box plots of the follow-up time in years of the samples of the training cohort. As can be seen from the figure, there is not a significant difference between follow-up times across the three categories.

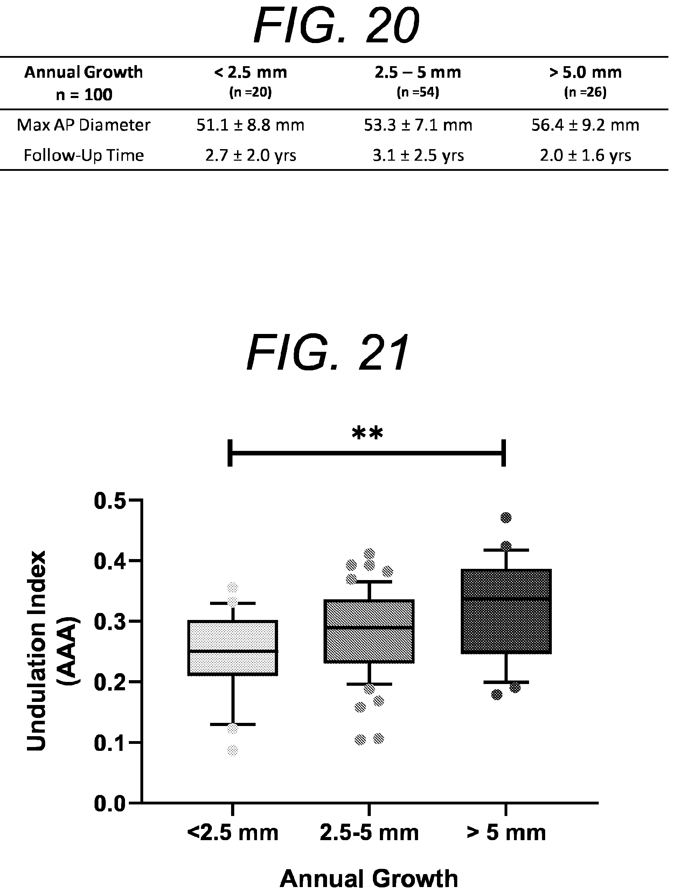

Figure 20 shows a table with the mean and standard deviation maximum AP diameters for each subgroup of the training cohort, and with the mean and standard deviation follow up time for each subgroup of the training cohort.

Figure 21 shows boxplots of the undulation index for the aneurysms in each subgroup of the training cohort. A one way ANOVA was performed that determined that the difference between the "less than 2.5 mm per year" subgroup and the "greater than 5 mm per year" subgroup was statistically significant with a p value of $p < 0.01$ (as indicated by the horizontal bar with two asterisks).

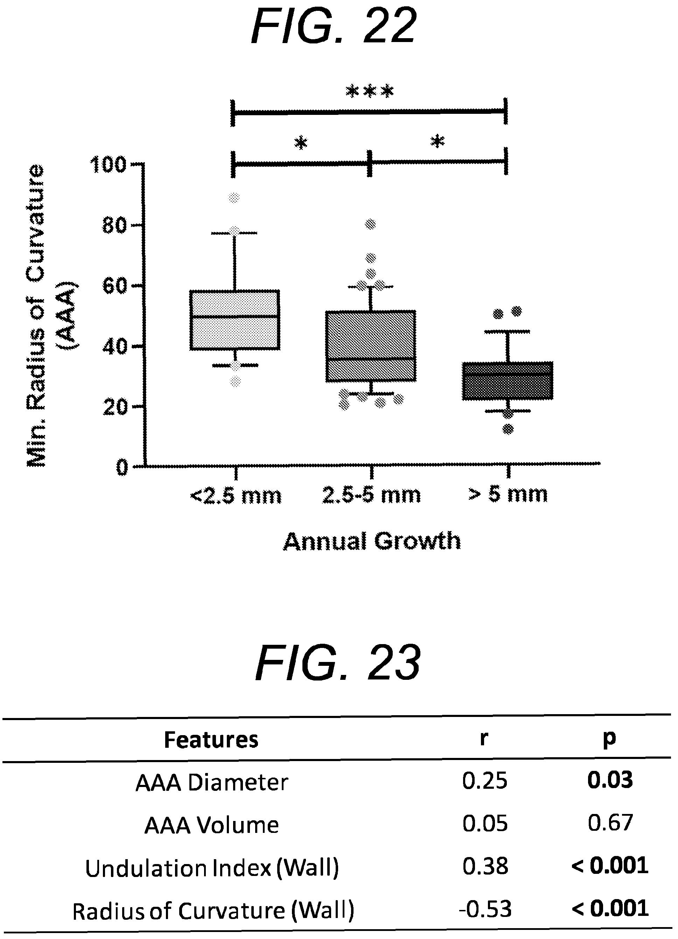

Figure 22 shows box plots of the minimum radius of curvature for the aneurysms in each subgroup of the training cohort. A one way ANOVA was performed which indicated that the difference between the "less than 2.5 mm per year" subgroup and the "2.5-5 mm per year" subgroup was statistically significant with a p value of $p < 0.05$ (as indicated by the horizontal bar with a single asterisk). A one way ANOVA indicated that the difference between the "2.5-5 mm per year" subgroup and the "greater than 5 mm per year" subgroup was statistically significant with a p value of $p < 0.05$ (as indicated by the horizontal bar with a single asterisk). A one way ANOVA indicated that the difference between the "less than 2.5 mm per year" subgroup and the "greater than 5 mm per year" subgroup was statistically significant with a p value of p < 0.001 (as indicated by the horizontal bar with three asterisks).

Figure 23 shows a table summarising the correlation of certain measurable properties of a volumetric model with the growth rate of aneurysms of the training set. As has already been shown above, the correlation between each of the initial AP diameter, undulation index, and minimum radius of curvature and the growth rate was shown to be statistically significant. For the 100 patients of the training cohort, the relationship between the initial aneurysmal volume and the annual growth of the aneurysm was also considered but found not to be statistically significant.

A multinomial logistic regression was performed on the training cohort with 10-fold cross validation. Multinomial logistic regression is a known method for predicting categorical placement in or the probability of category membership on a dependent variable y based on multiple independent variables $x_i$ (for example, initial maximum AP diameter, undulation index, minimum radius of curvature). As the correlation between the aneurysm volume and the annual growth of the aneurysm was found to be weak, the volume was not considered in the multinomial logistic regression.

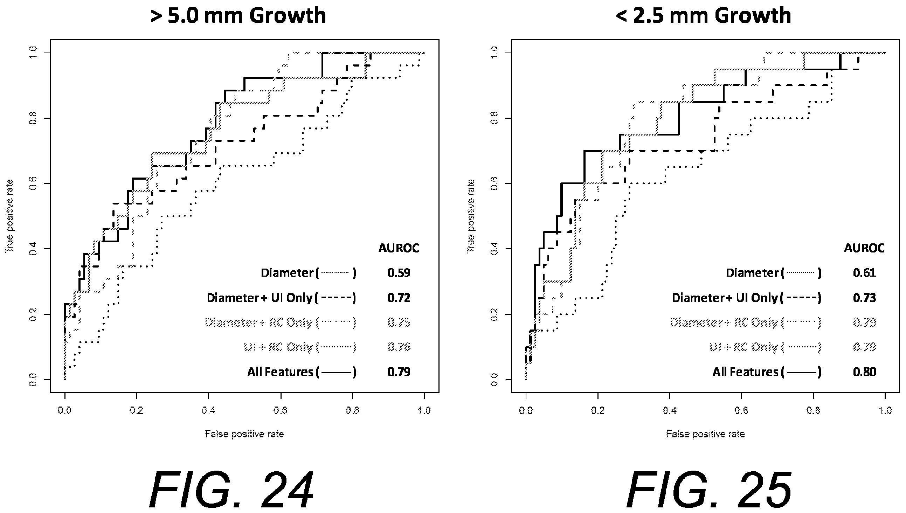

Figure 24 shows receiver operator characteristic (ROC) curves for assigning samples of the testing cohort to the "greater than 5.0 mm per year" subgroup. Each ROC curve is calculated for a different measure of combination of measures. The areas under each ROC curve (AUROC) are indicated in the figure. The diameter of the aneurysm (dotted line) had the smallest AUROC and therefore was not, on its own, particularly useful for determining whether an aneurysm of the training cohort would be categorised as having a growth rate of "greater than 5.0 mm per year". Combinations of geometric measures produced greater AUROC scores.

Figure 25 shows ROC curves for assigning samples of the testing cohort to the "less than 2.5 mm per year" subgroup. Once again, combinations of geometric measures produced greater AUROC scores.

It has therefore been indicated that geometric measures of a volumetric model of an aneurysm can be used to predict a categorical growth rate of an aneurysm.

The inventors have investigated whether geometric features from aortic aneurysms can be used to predict continuous growth (that is, to be able to predict a numeric value for the annual growth rate of an aneurysm). As can be seen from the table of Figure 23, the AAA volume did not appear to be statistically significant in predicting the categorical growth rate of the aneurysms, while the AAA diameter, the undulation index, and the radius of curvature were all shown to be statistically significant. Accordingly, for investigation whether geometric features from aortic aneurysms can be used to predict continuous growth, the AAA volume was not considered while combinations of the AAA volume, undulation index, and minimum radius of curvature, were considered.

The inventors used multiple linear regression models, with 10-fold cross validation. In multiple linear regression models, observations are assumed to be the result of random deviations from an underlying relationship between a dependent variable, y (for example, aneurysm growth rate) and several independent variables $x_i$ (for example, initial maximum AP diameter, undulation index, minimum radius of curvature), the underlying relationship characterised by corresponding coefficients $\beta_i$.

Cross-validation is a technique to evaluate predictive models by partitioning the original sample into a training set to train the model, and a test set to evaluate it. In k-fold cross-validation, the original sample is randomly partitioned into k equal size subsamples. Of the k subsamples, a single subsample is retained as the validation data for testing the model, and the remaining k-1 subsamples are used as training data. The cross-validation process is then repeated k times, with each of the k subsamples used once as the validation data. The k results from the folds can then be averaged (or otherwise combined) to produce a single estimation.

Of the coefficients of the 10-fold models, the inventors selected the median $\beta$ coefficients for the optimised linear regression models before evaluating these optimised models on the testing cohort.

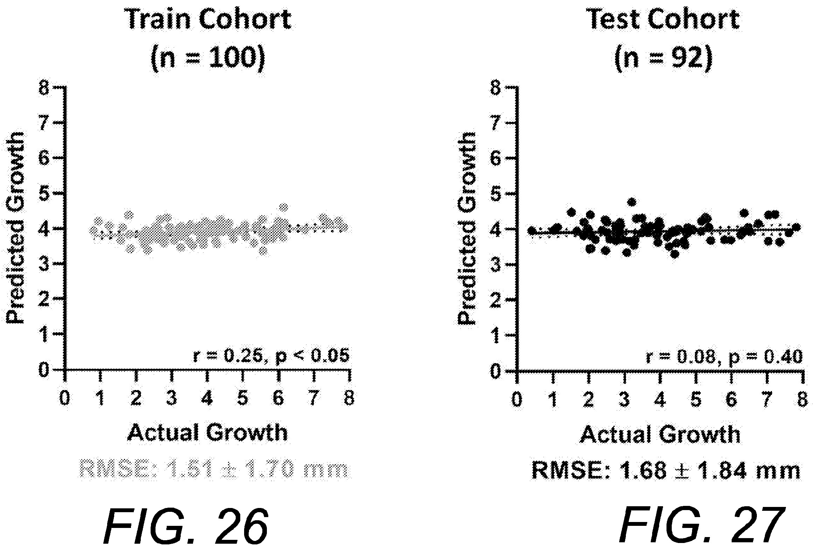

A first linear regression model was used to check whether the initial maximum AP diameter alone could be used to predict continuous growth rate. Figure 26 shows a scatter plot of the predicted growth rate (in mm per year) of the 100 aneurysms of the training cohort as predicted by the first linear regression model against the actual growth rate of the aneurysms. The root mean squared error for the growth rate was found to be $1.51 \pm 1.70$ mm per year. The Spearman coefficient was found to be r = 0.25, with a p-value of $p < 0.05$. The solid line shows this positive correlation. The dashed lines indicate the 95% confidence interval.

Figure 27 shows a scatter plot of the predicted growth rate of the 92 aneurysms of the testing cohort as predicted by the first linear regression model against the actual growth rate of the aneurysms. The root mean squared error for the growth rate was found to be $1.68 \pm 1.84$ mm per year. The Spearman coefficient was found to be 0.08, with a p-value of p = 0.4. The solid line shows this positive correlation. The dashed lines indicate the 95% confidence interval.

Figures 26 and 27 indicate that the initial aneurysm diameter alone is a weak predictor of a numeric growth rate of the aneurysm.

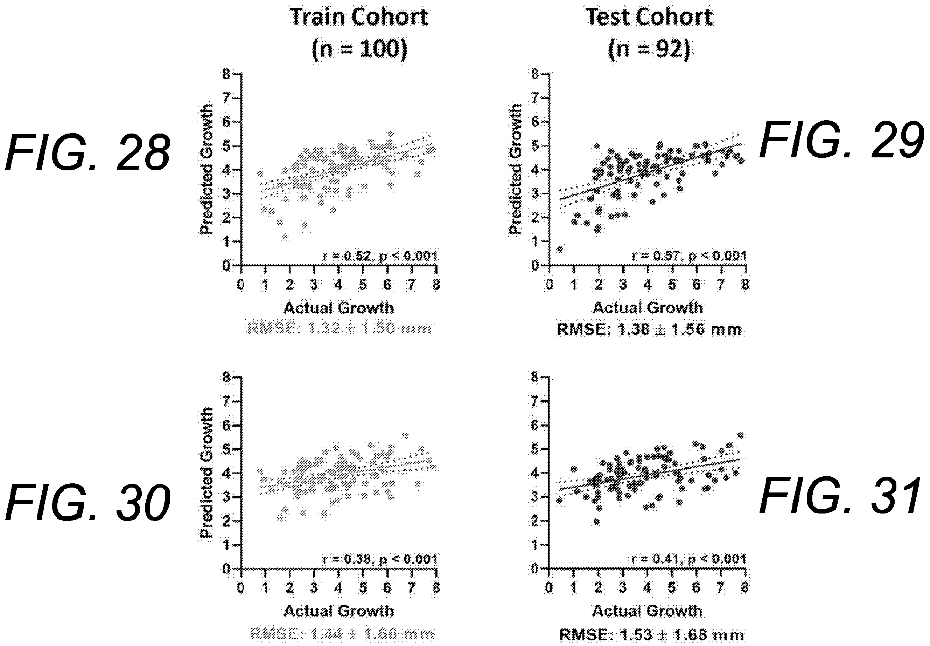

A second linear regression model was used to check whether the minimum radius of curvature alone could be used to predict continuous growth rate. Figure 28 shows a scatter plot of the predicted growth rate (in mm per year) of the 100 aneurysms of the training cohort as predicted by the second linear regression model against the actual growth rate of the aneurysms. The root mean squared error for the growth rate was found to be $1.32 \pm 1.50$ mm per year. The Spearman coefficient was found to be r = 0.52, with a p-value of $p < 0.001$. The solid line shows this positive correlation. The dashed lines indicate the 95% confidence interval.

Figure 29 shows a scatter plot of the predicted growth rate (in mm per year) of the 92 aneurysms of the testing cohort as predicted by the second linear regression model against the actual growth rate of the aneurysms. The root mean squared error for the growth rate was found to be $1.38 \pm 1.56$ mm per year. The Spearman coefficient was found to be r = 0.57, with a p-value of $p < 0.001$. The solid line shows this positive correlation. The dashed lines indicate the 95% confidence interval.

Figures 28 and 29 indicate that the minimum radius of curvature alone is a better predictor of the growth rate of the aneurysm than the initial maximum AP diameter alone.

A third linear regression model was used to check whether the undulation index alone could be used to predict continuous growth rate. Figure 30 shows a scatter plot of the predicted growth rate (in mm per year) of the 100 aneurysms of the training cohort as predicted by the third linear regression model against the actual growth rate of the aneurysms. The root mean squared error for the growth rate was found to be $1.44 \pm 1.66$ mm per year. The Spearman coefficient was found to be r = 0.38, with a p-value of $p < 0.001$. The solid line shows this positive correlation. The dashed lines indicate the 95% confidence interval.

Figure 31 shows a scatter plot of the predicted growth rate (in mm per year) of the 92 aneurysms of the testing cohort as predicted by the third linear regression model against the actual growth rate of the aneurysms. The root mean squared error for the growth rate was found to be $1.53 \pm 1.68$ mm per year. The Spearman coefficient was found to be r = 0.41, with a p-value of $p < 0.001$. The solid line shows this positive correlation. The dashed lines indicate the 95% confidence interval.

Figures 30 and 31 indicate that the undulation index alone is a better predictor of the growth rate of the aneurysm than the initial maximum AP diameter alone.

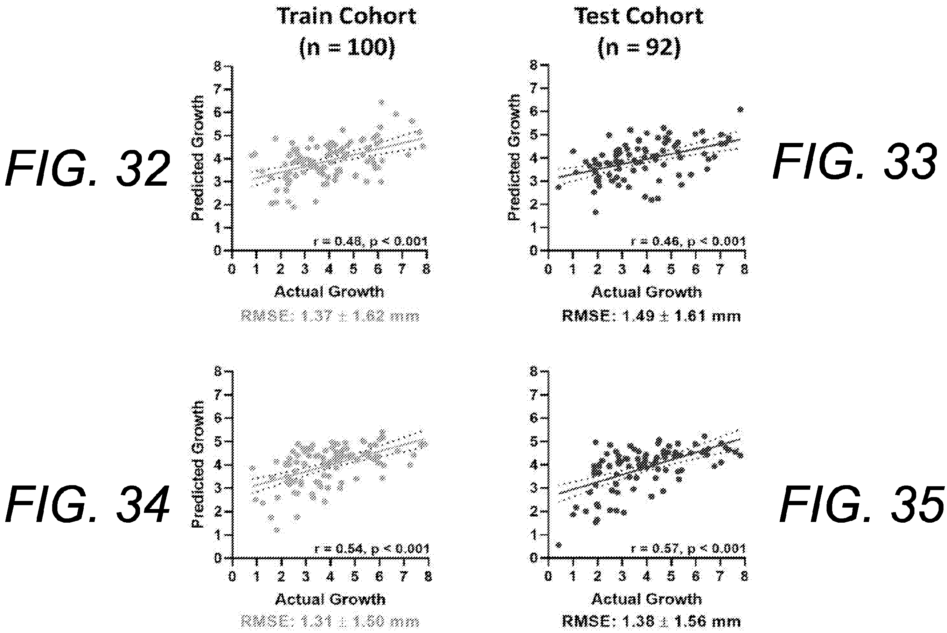

A fourth linear regression model was used to check whether the undulation index and the initial aneurysm diameter together could be used to predict continuous growth rate. Figure 32 shows a scatter plot of the predicted growth rate (in mm per year) of the 100 aneurysms of the training cohort as predicted by the fourth linear regression model against the actual growth rate of the aneurysms. The root mean squared error for the growth rate was found to be $1.37 \pm 1.62$ mm per year. The Spearman coefficient was found to be r = 0.48, with a p-value of $p < 0.001$. The solid line shows this positive correlation. The dashed lines indicate the 95% confidence interval.

Figure 33 shows a scatter plot of the predicted growth rate (in mm per year) of the 92 aneurysms of the testing cohort as predicted by the fourth linear regression model against the actual growth rate of the aneurysms. The root mean squared error for the growth rate was found to be 1.49 $\pm$ 1.61 mm per year. The Spearman coefficient was found to be r = 0.46, with a p-value of p < 0.001. The solid line shows this positive correlation. The dashed lines indicate the 95% confidence interval.

A fifth linear regression model was used to check whether the minimum radius of curvature and the initial aneurysm diameter together could be used to predict continuous growth rate. Figure 34 shows a scatter plot of the predicted growth rate (in mm per year) of the 100 aneurysms of the training cohort as predicted by the fifth linear regression model against the actual growth rate of the aneurysms. The root mean squared error for the growth rate was found to be 1.31 $\pm$ 1.50 mm per year. The Spearman coefficient was found to be r = 0.54, with a p-value of p < 0.001. The solid line shows this positive correlation. The dashed lines indicate the 95% confidence interval.

Figure 35 shows a scatter plot of the predicted growth rate (in mm per year) of the 92 aneurysms of the testing cohort as predicted by the fifth linear regression model against the actual growth rate of the aneurysms. The root mean squared error for the growth rate was found to be 1.38 $\pm$ 1.56 mm per year. The Spearman coefficient was found to be r = 0.57, with a p-value of p < 0.001. The solid line shows this positive correlation. The dashed lines indicate the 95% confidence interval.

A sixth linear regression model was used to check whether the minimum radius of curvature and the undulation index together could be used to predict continuous growth rate.

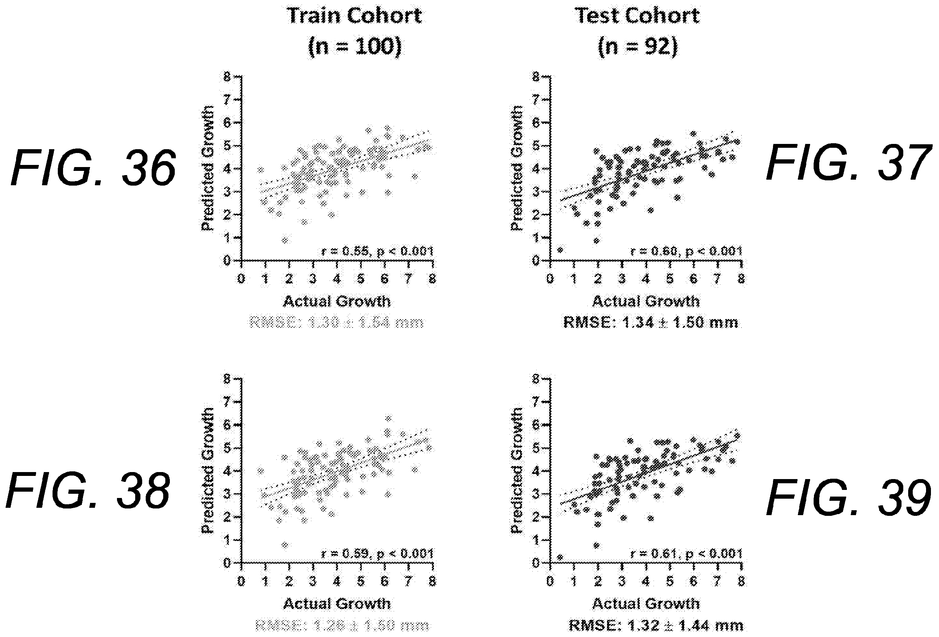

Figure 36 shows a scatter plot of the predicted growth rate (in mm per year) of the 100 aneurysms of the training cohort as predicted by the sixth linear regression model against the actual growth rate of the aneurysms. The root mean squared error for the growth rate was found to be 1.30 $\pm$ 1.54 mm per year. The Spearman coefficient was found to be r = 0.55, with a p-value of p < 0.001. The solid line shows this positive correlation. The dashed lines indicate the 95% confidence interval.

Figure 37 shows a scatter plot of the predicted growth rate (in mm per year) of the 92 aneurysms of the testing cohort as predicted by the sixth linear regression model against the actual growth rate of the aneurysms. The root mean squared error for the growth rate was found to be 1.34 $\pm$ 1.50 mm per year. The Spearman coefficient was found to be r = 0.6, with a p-value of p < 0.001. The solid line shows this positive correlation. The dashed lines indicate the 95% confidence interval.

A seventh linear regression model was used to check whether the initial aneurysm diameter, minimum radius of curvature and the undulation index together could be used to predict continuous growth rate. Figure 38 shows a scatter plot of the predicted growth rate (in mm per year) of the 100 aneurysms of the training cohort as predicted by the seventh linear regression model against the actual growth rate of the aneurysms. The root mean squared error for the growth rate was found to be 1.26 $\pm$ 1.50 mm per year. The Spearman coefficient was found to be $r$ = 0.59, with a p-value of p < 0.001. The solid line shows this positive correlation. The dashed lines indicate the 95% confidence interval.

Figure 39 shows a scatter plot of the predicted growth rate (in mm per year) of the 92 aneurysms of the testing cohort as predicted by the seventh linear regression model against the actual growth rate of the aneurysms. The root mean squared error for the growth rate was found to be 1.32 $\pm$ 1.44 mm per year. The Spearman coefficient was found to be r = 0.61, with a p-value of p < 0.001. The solid line shows this positive correlation. The dashed lines indicate the 95% confidence interval.

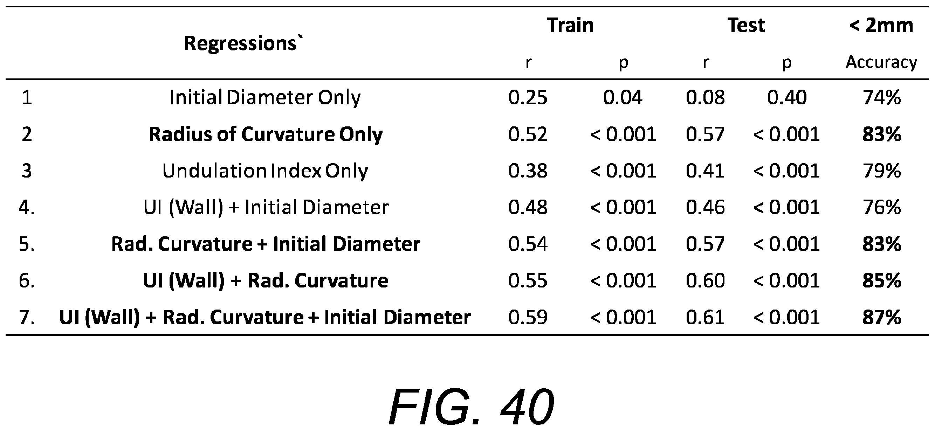

Figure 40 shows a table summarising the properties of the seven regression models used. For each regression model, the accuracy of the regression model in predicting an annual growth that was within 2mm of the actual growth is indicated. The second, fifth, sixth and seventh regression models were all found to have an accuracy to within 2mm of over 80%.

Accordingly, it has been demonstrated that by analysing one or more geometric measures of a volumetric model of at least a portion of an artery having an aneurysm, the numeric/continuous growth rate of the aneurysm can be predicted.

In what follows, it will now be demonstrated that PCA derived geometric features can also be used to estimate the growth rate of an aneurysm.

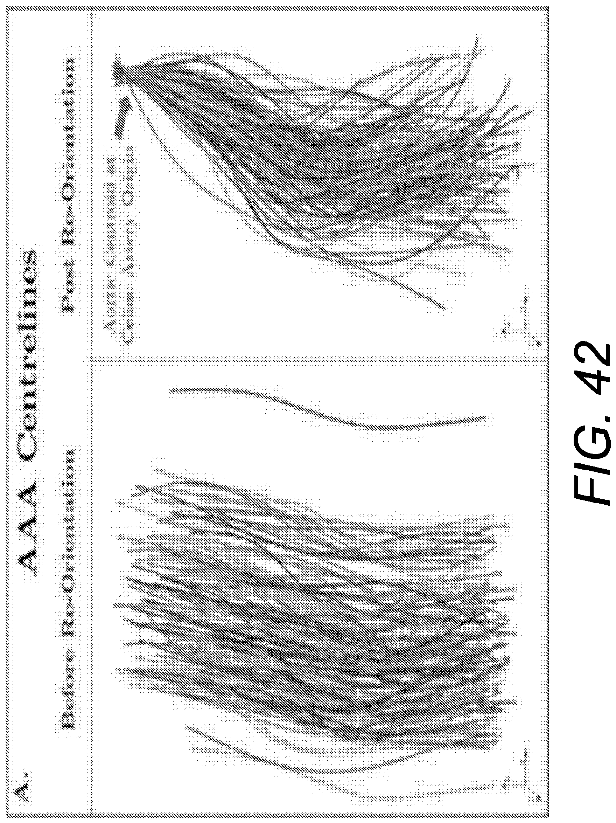

The inventors performed three sets of PCA in order to capture the diverse AAA shapes in forms that were easy to analyse. PCA was performed to assess the correspondences between different components in the shape-based, surface-based, and branch point-based input matrices, as explained in relation to figures 42-66 below. Here, the primary goal was to investigate and potentially identify additional features that not only characterize the AAA but also predict aneurysmal progression. PCA was performed separately for each of the input matrices and coefficients were calculated by using the residuals (Input Data - Mean of Input Data) and singular value decomposition. The obtained principal components were analysed for their variance percentage and component coefficients, to determine their significance. For each of the three PCAs described below, the principal components that collectively captured 95% of the variance percentage were used for growth prediction analysis and an iterated 10-Fold optimization with shuffling (n = 100 iterations) approach was applied using data from all 192 patients for model training/optimization and validation.

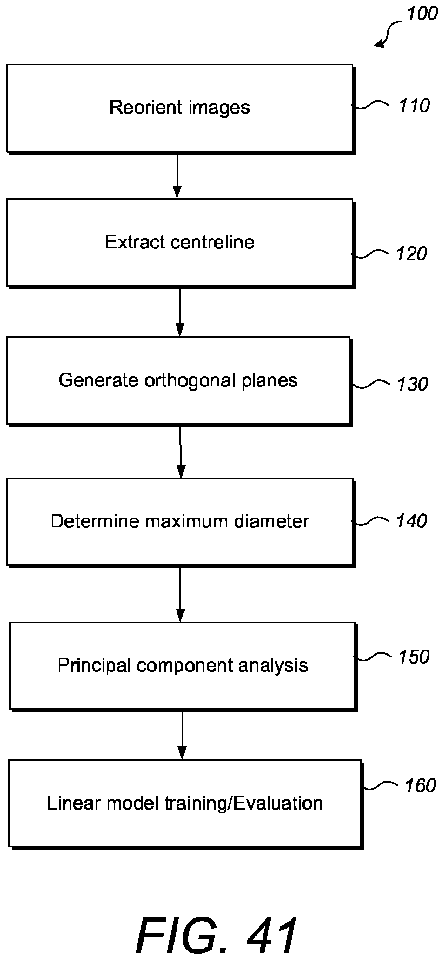

Figure 41 shows a flowchart of a first method 100 for predicting a growth rate of an aortic aneurysm using PCA derived geometric features.

At step 110, the method comprises reorienting the images obtained from each of the 192 patients to the same coordinate axis based on a fixed point. This is because inherently, aortic aneurysms display high levels of variability not only in terms of geometry but also in terms of location and extent within the infra-renal region.

The goal of the PCA is to capture this variability in meaningful principal components. However, this is complicated by the presence of additional variability secondary to the movement of patient locations between imaging studies. Minimization of this variability is achieved by reorienting all images to the same coordinate axis based on a fixed point. In this instance, the fixed point was selected to be the 2D aortic centroid at the level of the origin of the celiac artery. This was selected as a reference point as it was present in all patients, and consisted of a non-pathological point proximal to the pathological aneurysm. Figure 42 displays the AAA centrelines before and after the re-orientation.

Returning now to figure 41, at step 120, the method comprises extracting a centreline through, for example, a volumetric model of an AAA. Extracting a centreline may comprise determining coordinates of a centreline through the AAA volumetric model. Optimized Matlab implementation of the homotopic thinning algorithm (Skeletonization) was used to extract the centrelines and the centrelines were smoothed using a Savitzky-Golay filter.

At step 130, the method comprises constructing or generating planes orthogonal to the extracted centreline.

At step 140, the method comprises determining a maximum diameter of each orthogonal plane generated in step 130.

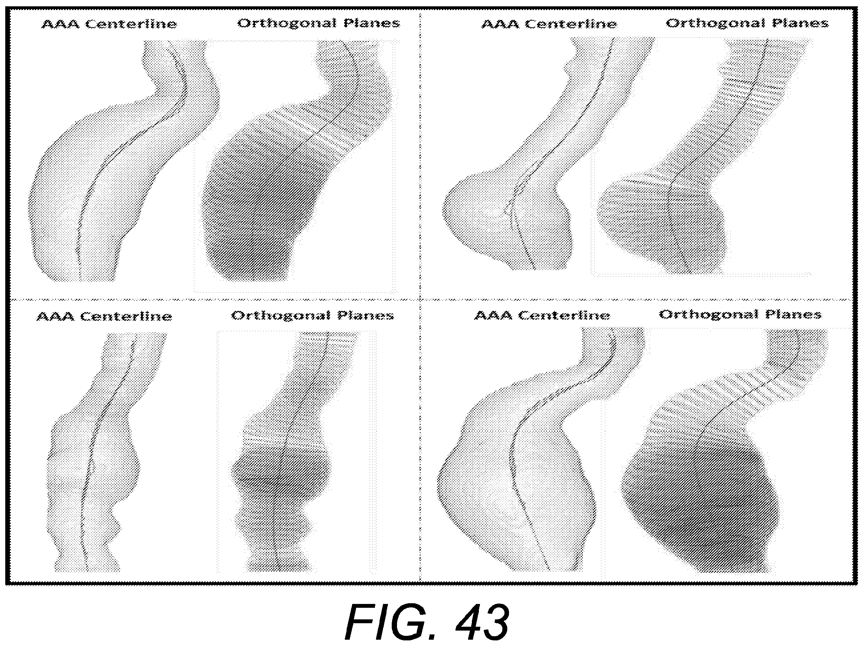

Figure 43 shows an example of centreline extraction and orthogonal plane generation for four different AAA volumetric models. For each of the four AAA volumetric models in figure 43, the left side shows the extracted centreline, and the right side shows planes orthogonal to the extracted centreline.

Steps 110-140 as described above in relation to figure 41, are used to characterise the shape of each AAA into a singular vector which consists of the centreline coordinates corresponding to the centreline through the AAA volumetric model and the maximum diameter of each orthogonal plane. Fifty equidistant points along the centreline were interpolated and orthogonal planes were sampled.

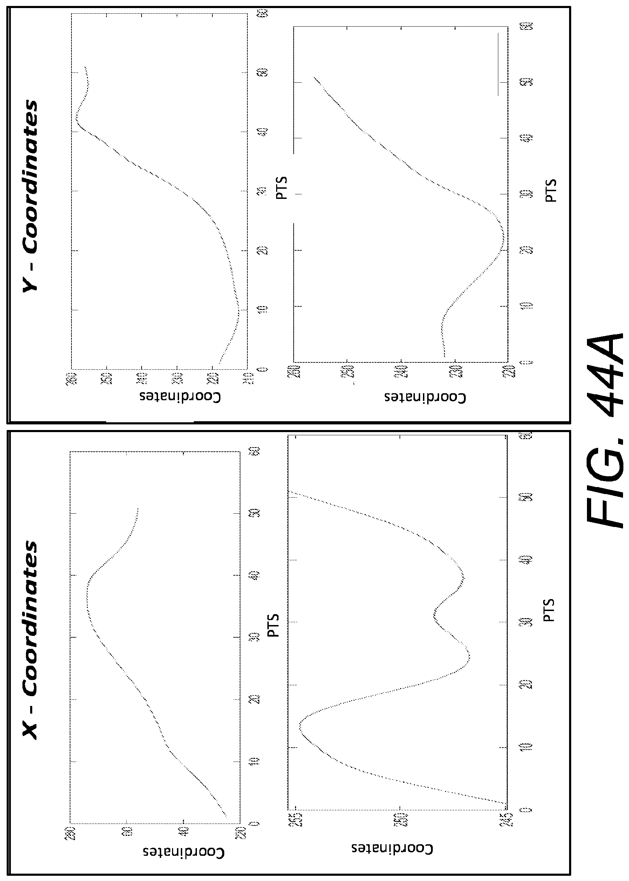

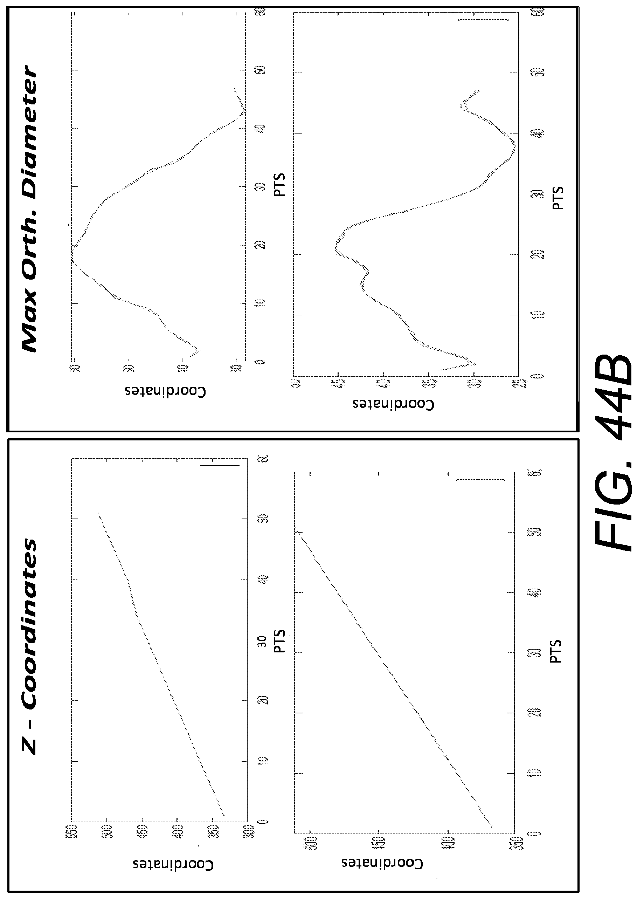

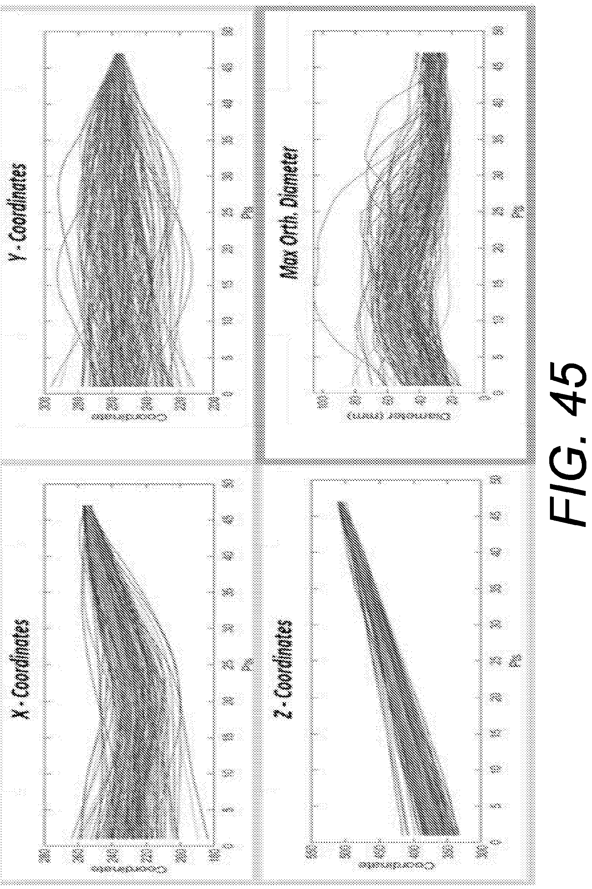

At step 150, the method comprises performing PCA using the x, y, and z coordinates and the maximum diameter measurements which form the input matrix for subsequent PCA. Figures 44A-B show examples of graphs relating to the x, y and z coordinates against points (pts) and the maximum orthogonal diameter measurements (mm) against points (pts) where figure 44A shows the x and y coordinates against points and figure 44B shows the z coordinates and the maximum orthogonal diameter measurements (mm) against points (pts). Figure 45 shows these graphs for all 192 patients. Each of the 192 patient vectors was composed of 188 points (46 X-coordinates + 46 Y-coordinates + 46 Z-coordinates + 46 maximum orthogonal diameters). The input matrix (192 patients x 188 points) was then normalized per component prior to analysis.

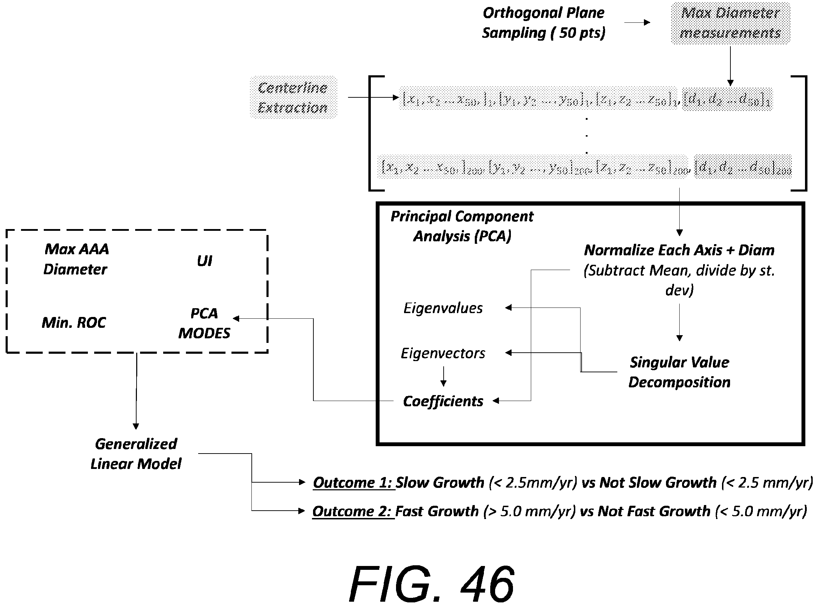

Figure 46 shows the matrix as described above to be taken as an input for PCA. As shown in figure 46 in the box labelled Principal component analysis (PCA), all x, y, and z coordinates and the maximum diameter measurements are normalized by subtracting the mean and dividing by the standard deviation. However, the first and last two points/planes along the aortic centreline were excluded from all patients as these orthogonal planes were predominantly located outside the AAA volume and would have incorrect maximum diameters. Singular value decomposition is then performed on each of the normalised x, y, and z coordinates and the maximum diameter measurements to obtain eigenvalues from which PCA coefficients are obtained. The PCA coefficients are then used to determine PCA modes, which are then taken as input to a generalised linear model, as explained below in relation to step 160. PCA modes are also known as modes of variance as would be understood by the skilled person. These modes of variance or PCA modes represent a combination of the input variables that capture most of the variability of the data and are commonly used for feature reduction.

Steps 110-150 above highlight the extraction or determination of shape-based modes, where the shape-based modes are modes extracted or determined by extracting a centreline through a AAA volumetric model and determining the maximum diameters of planes orthogonal to the extracted centreline.

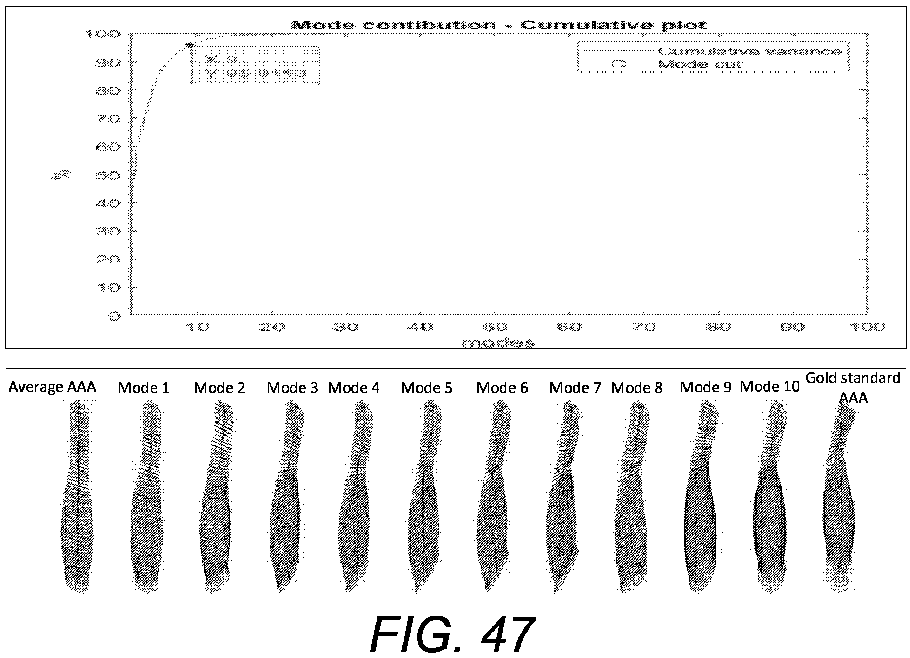

Figure 47 shows the cumulative variance captured by the PCA derived shape-based modes which suggests that the first 9 modes are sufficient to capture 95% of the variance. Figure 47 also shows the reconstruction of AAA using shape-based modes 1-10, where each reconstruction is shown labelled with the corresponding mode that was used to reconstruct it. Figure 47 also shows a reconstruction of the average AAA and the "gold standard" AAA.

At step 160 of figure 41, the method comprises using the derived PCA shape-based modes as inputs to train a generalised linear model for classifying the growth rate into one or more categories. For example, the generalised linear model, which may be a multinomial logistic regression (such as that described above in relation to Figures 16-25), can be used for the binary classification task of predicting either: 1.) Slow Growth or Not-Slow Growth or 2.) Fast Growth or Not-Fast Growth, where "slow growth" is defined as an annual growth rate of less than 2.5 mm per year and "not-slow growth" is defined as an annual growth rate of more than 2.5 mm per year. The "fast growth" is defined as an annual growth rate of more than 5 mm per year and the "not-fast growth" is defined as an annual growth rate of less than 5 mm per year. This is illustrated in figure 46, where the PCA modes as shown inside the dashed box are taken as input to a generalised linear model which outputs either outcome 1 or outcome 2, where outcome 1 relates to classifying the growth rates as slow growth or not-slow growth and output 2 relates to classifying the growth rate as fast growth or not-fast growth.

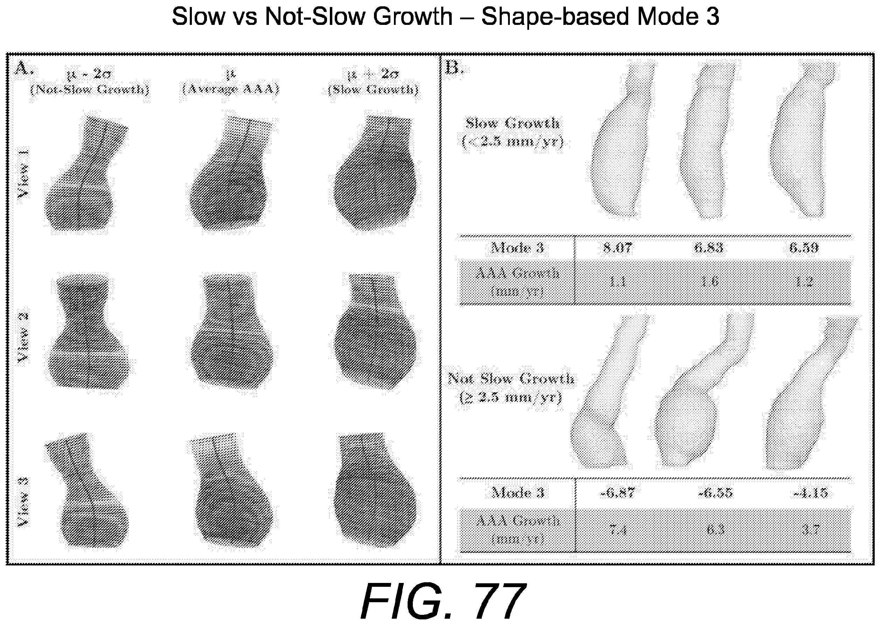

The inventors devised three different models, models IV-VI, to predict the growth rate of, for example, an aortic aneurysm, where each of the three models were trained using different features. Model IV was trained using only modes 1-9 of the derived PCA shape-based modes. Linear discriminant analysis (LDA) was used to determine mode significance and only significant modes were used for subsequent analysis. In terms of shape-based modes, modes 3, 7 and 1, 4 were statistically significant for predicting slow and fast growth, respectively. The significance of modes 3, 7 and 4 is discussed below in relation to figures 77-79 below. Model V was trained using a combination of these significant modes of the derived PCA shape-based modes, herein referred to as selected shape-based modes and geometric features (e.g. radius of curvature and undulation index). Model VI was trained using a combination of the selected shape-based modes, geometric features (radius of curvature and undulation index) and maximum AAA diameter. This is illustrated in figure 46, where one or more of the above features as shown in the dashed box are taken as an input to train a generalised linear model, where each model then outputs outcome 1: slow growth or not-slow growth or outcome 2: fast growth or not-fast growth. All models were trained using iterative 10-fold cross-validation (n = 100 iterations).

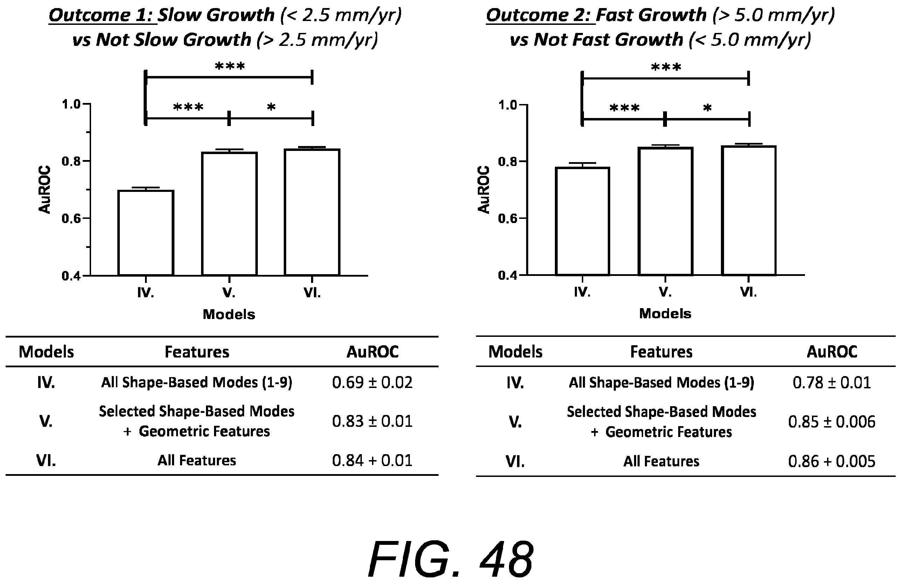

Figure 48 shows a bar graph relating to the AUROC scores obtained from each of models IV-VI used to predict outcome 1: slow growth or not-slow growth and outcome 2: fast growth or not-fast growth. The tables under each graph summarise the features used for each model. Each table also shows the AUROC scores obtained for each of models IV-VI for the two outcomes, where the AUROCs for predicting slow and fast growth using model IV were 0.69 and 0.78, respectively. Combining maximum AAA diameter and geometric features with the selected shape-based modes significantly improved model predictive capacity (Model VI vs Model IV, p < 0.001).

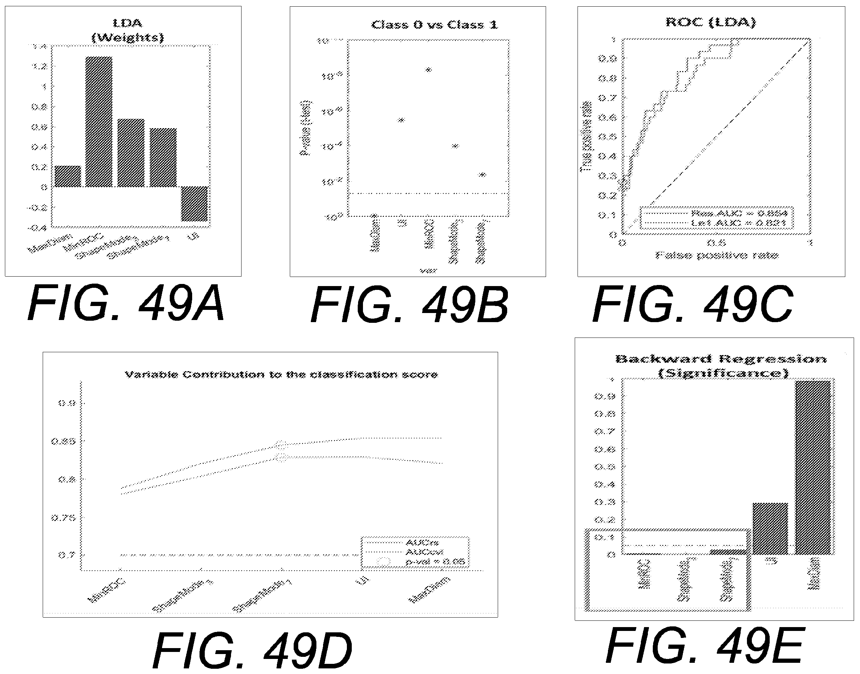

Figures 49A-E illustrate the performance of the different variables relating to PCA derived shape-based modes 3 and 7 and geometric measures (UI, radius of curvature and maximum diameter) used to train the generalised linear models IV-VI for predicting outcome 1 (slow vs not-slow growth). Figure 49A shows a graph relating to LDA (weights) for each variable within the generalized linear model. Figure 49B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 49C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 49D illustrates the influence of each variable to the calculated AUC. Figure 49E shows the significance of each variable using backward regression, which is a method used to calculate the significance of the input variables by iteratively removing the variable and evaluating model performance.

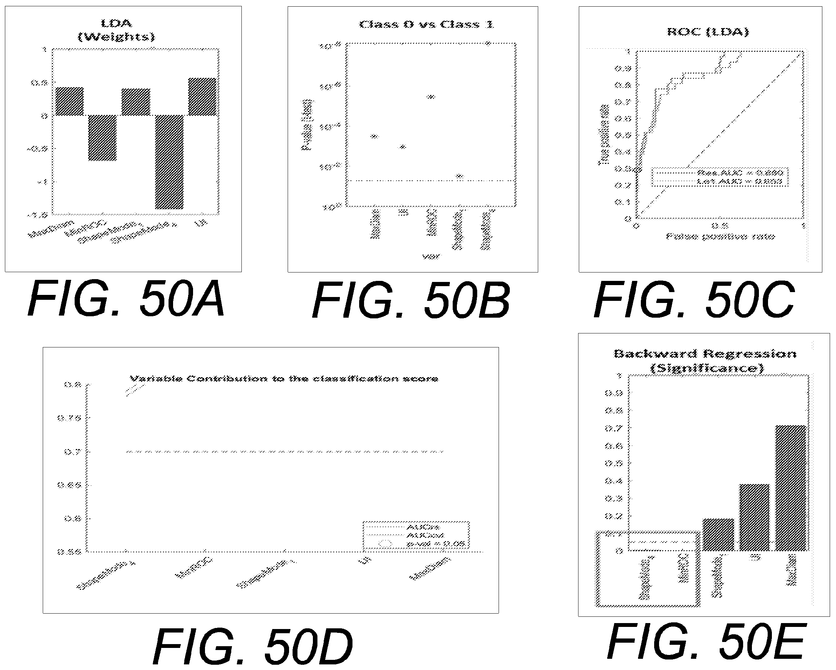

Figures 50A-E illustrate the performance of the different variables relating to PCA derived shape-based modes 1 and 4 and geometric measures (UI, radius of curvature and maximum diameter) used to train the generalised linear models IV-VI for predicting outcome 2 (fast vs not-fast growth). Figure 50A shows a graph relating to LDA (weights) for each variable within the generalized linear model. Figure 50B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 50C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 50D illustrates the influence of each variable to the calculated AUC. Figure 50E shows the significance of each variable using backward regression, which is a method used to calculate the significance of the input variables by iteratively removing the variable and evaluating model performance.

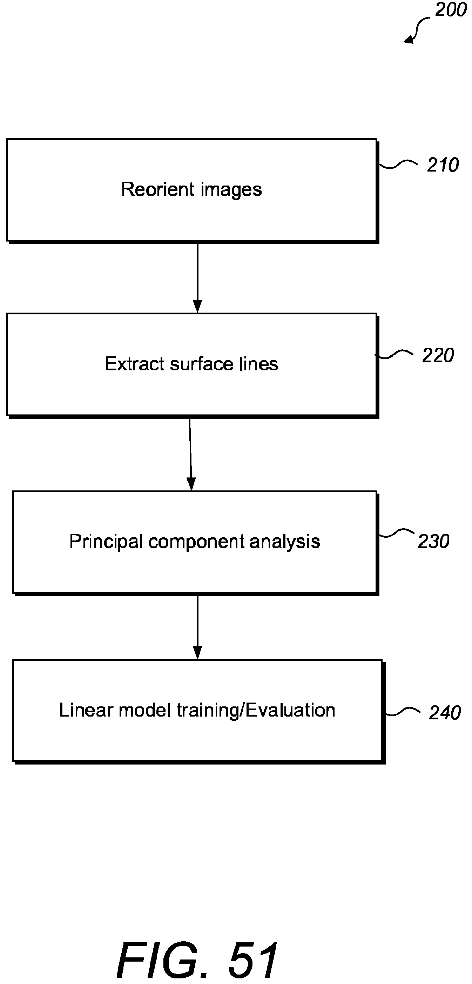

In addition to the aortic shape, the inventors hypothesized that surface curvature and structure of the aorta/AAA may play a role in predicting aneurysmal growth. Figure 51 shows a flowchart of a second method 200 for predicting a growth rate of an aortic aneurysm using PCA derived geometric features which focus on surface curvature and structure of the aorta/AAA.

At step 210, the method comprises reorienting the images obtained from each of the 192 patients to the same coordinate axis based on a fixed point. This is because inherently, aortic aneurysms display high levels of variability not only in terms of geometry but also in terms of location and extent within the infra-renal region. The goal of the PCA is to capture this variability in meaningful principal components. However, this is complicated by the presence of additional variability secondary to the movement of patient locations between imaging studies. Minimization of this variability is achieved by reorienting all images to the same coordinate axis based on a fixed point. In this instance, the fixed point was selected to be the 2D aortic centroid at the level of the origin of the celiac artery. This was selected as the reference point as it was present in all patients, and consisted of a non-pathological point proximal to the pathological aneurysm.

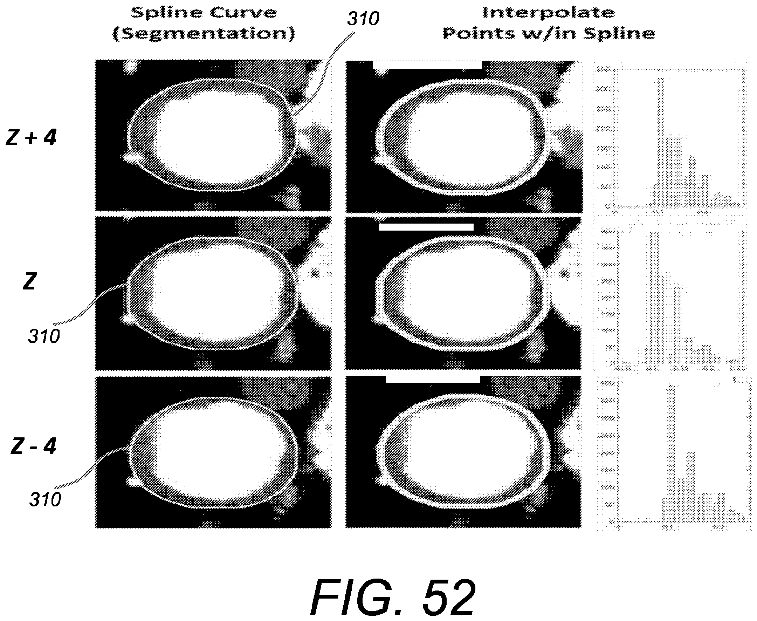

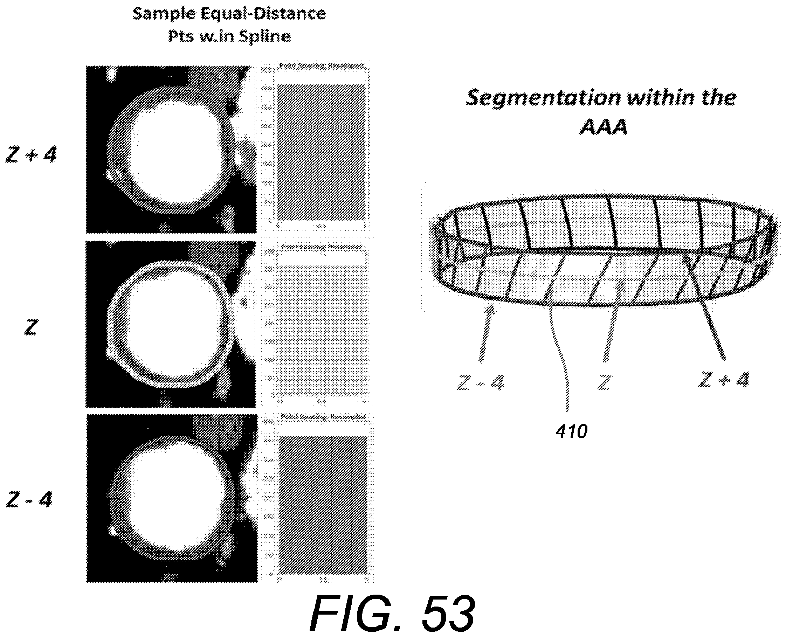

At step 220, the method comprises extracting surface lines by constructing or generating curves along the aortic surface. Extraction of the surface lines is discussed below in relation to figures 52-53, where surface lines 410 are lines on the aortic surface which connect points that are closest together between neighbouring axial slices of, for example, a CT image (see figure 53). Extraction of a surface line may comprise determining a set of coordinates corresponding to the surface line.

As shown in figure 52, a spline curve 310 is first generated to encompass the AAA segmentation from each axial slice along the AAA shape as shown in the three adjacent CT axial slices at axial levels Z-4, Z and Z+4 in figure 52. Figure 52 also shows interpolation of points within the generated spline curves and the spacing between the interpolated points is also highlighted in the bar graphs shown in figure 52 for each axial slice.

The spline curves 310 were then sampled into 360 equidistant points for each axial slice (Z-4, Z and Z+4) as illustrated in figure 53. Figure 53 also shows a bar graph relating to the point spacing for each axial slice. Subsequently, the surface lines 410 were derived by connecting points that are closest together between the axial slices, as shown in figure 53. Surface lines 410 were smoothed using a Savitzky-Golay filter in MATLAB.

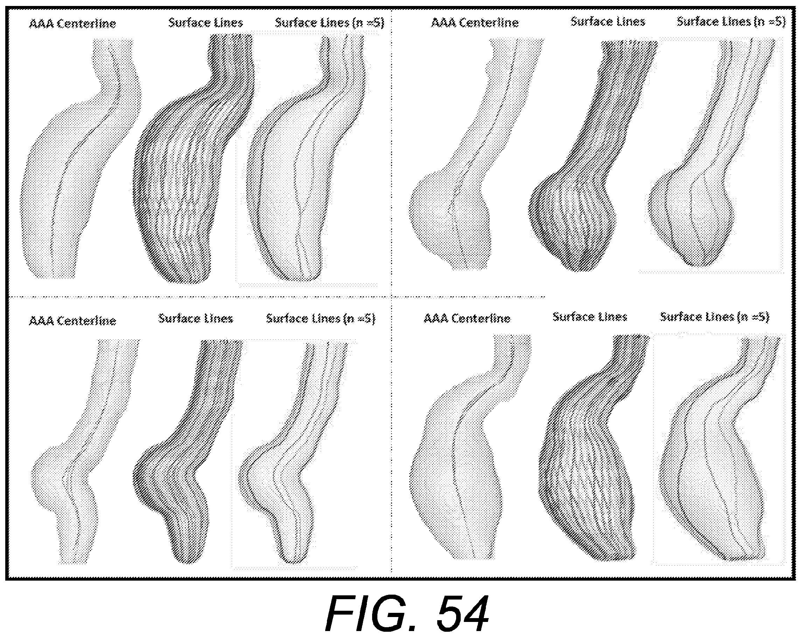

Figure 54 displays four aortas with AAAs characterized in this format. Five surface lines (at 72° increments) were used for subsequent analysis as this was deemed sufficient to capture the degree of surface curvature, irregularity and asymmetry. Fifty equidistant points were interpolated from the five surface lines to obtain a total of 750 coordinates (5 surface lines x [50 X-coordinates, 50 Y-coordinates, 50 Z-coordinates] = 750 coordinates). The x, y and z coordinates of surface lines at identical starting points (at 72° increments) between patients were normalized by subtracting the mean and dividing by the standard deviation. This matrix (dimensions 750 x 192) served as the input to the PCA.

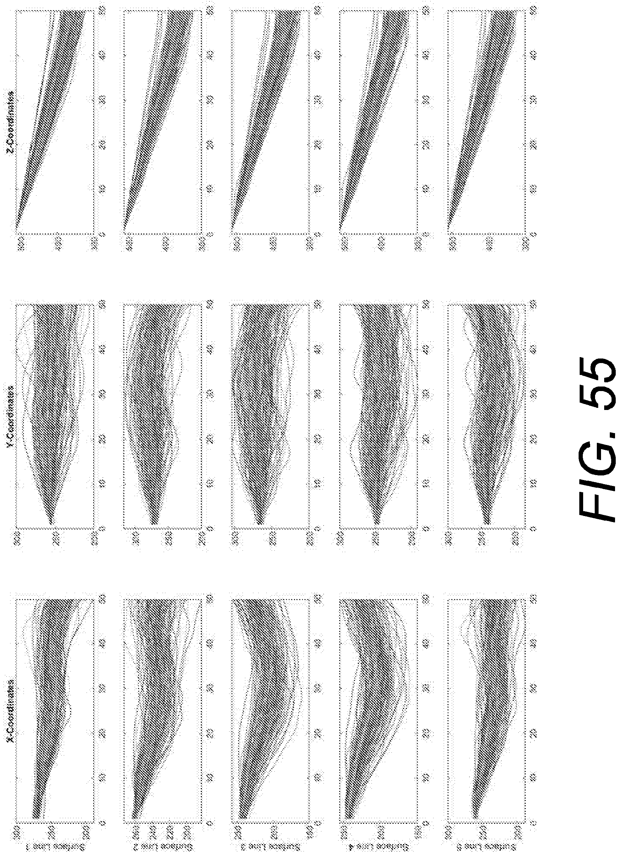

Figure 55 shows the x, y and z coordinates of the five surface lines for all patients.

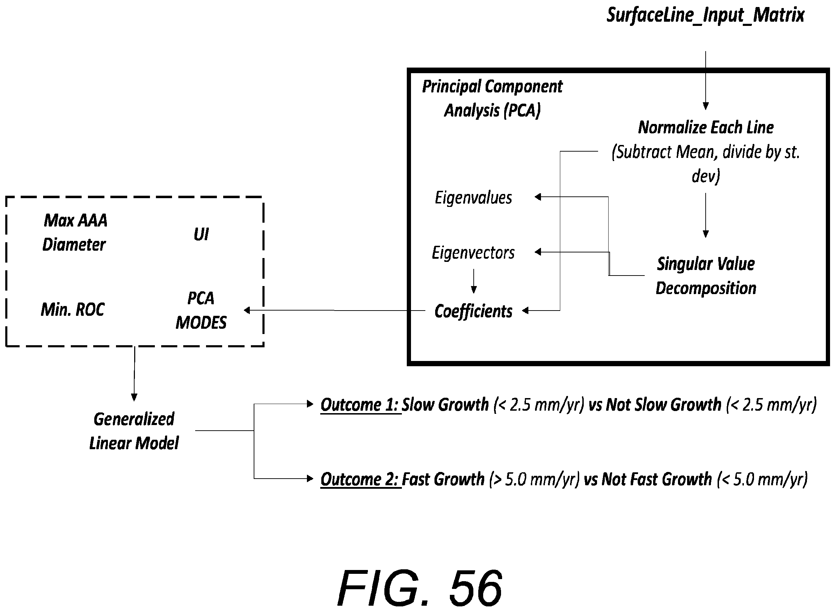

At step 230 of figure 51, the method comprises performing PCA using the x, y, and z coordinates corresponding to the surface lines. Figure 56 shows the matrix as described above to be taken as an input for PCA. As shown in figure 56 in the box labelled Principal component analysis (PCA), all x, y, and z coordinates are normalized by subtracting the mean and dividing by the standard deviation. Singular value decomposition is then performed on each of the normalised x, y, and z coordinates to obtain eigenvalues from which PCA coefficients are obtained. The PCA coefficients are then used to determine PCA modes.

Steps 210-230 of figure 51 highlight the extraction or determination of surface-based modes, where the surface-based modes are modes extracted or determined by generating curves which characterise the aneurysmal outer wall. These surface-based modes are then taken as input to a generalised linear model, as explained below in relation to step 250.

At step 240 of figure 51, the method comprises using the derived PCA surface-based modes as inputs to train a generalised linear model for classifying the growth rate into one or more categories. For example, the generalised linear model, which may be a multinomial logistic regression (such as that described above in relation to Figures 16-25), can be used for the binary classification task of predicting either: 1.) Slow Growth or Not-Slow Growth or 2.) Fast Growth or Not-Fast Growth, where "slow growth" is defined as an annual growth rate of less than 2.5 mm per year and "not-slow growth" is defined as an annual growth rate of more than 2.5 mm per year. The "fast growth" is defined as an annual growth rate of more than 5 mm per year and the "not-fast growth" is defined as an annual growth rate of less than 5 mm per year. This is illustrated in figure 56 where the PCA modes as shown inside the dashed box are taken as input to a generalised linear model which outputs either outcome 1 or outcome 2, where outcome 1 relates to classifying the growth rates as slow growth or not-slow growth and output 2 relates to classifying the growth rate as fast growth or not-fast growth.

The inventors devised three different models, models VII-IX, to predict the growth rate of, for example, an aortic aneurysm, where each of the three models were trained using different features. Model VII was trained using only modes 1-10 of the derived PCA surface-based modes. Linear discriminant analysis (LDA) was used to determine mode significance and only significant modes were used for subsequent analysis. In terms of surface-based modes, modes 6 and 3 were statistically significant for predicting slow and fast growth, respectively. Model VIII was trained using a combination of these significant modes of the derived PCA surface-based modes, herein referred to as selected surface-based modes and geometric features (e.g. radius of curvature and undulation index). Model IX was trained using a combination of the selected surface-based modes, geometric features (e.g. radius of curvature and undulation index) and maximum AAA diameter. This is also illustrated in figure 56, where one or more of the above features as shown in the dashed box are taken as an input to train a generalised linear model, where each model then outputs outcome 1: slow growth or not-slow growth or outcome 2: fast growth or not-fast growth. All models were trained using iterative 10-fold cross-validation (n = 100 iterations).

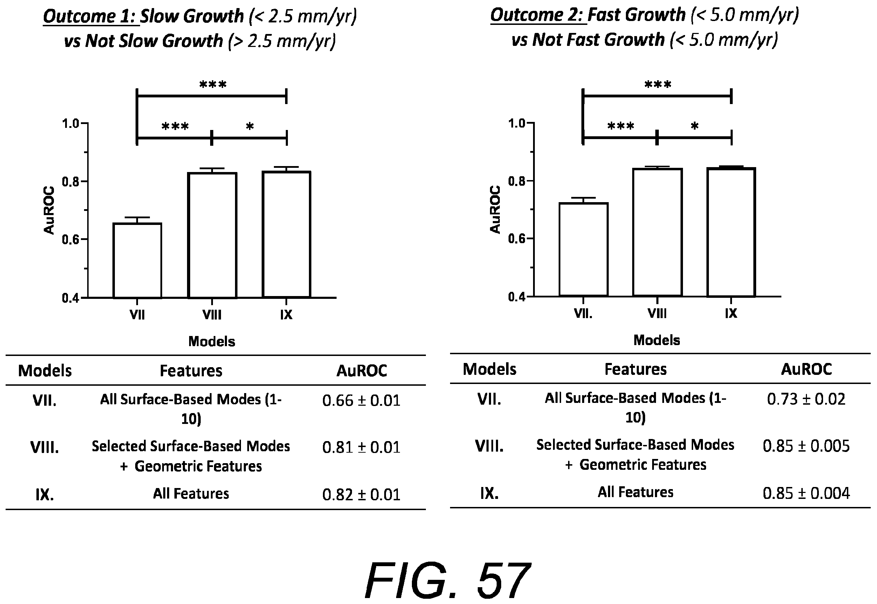

Figure 57 shows a bar graph relating to the AUROC scores obtained from each of models VII-IX used to predict outcome 1: slow growth or not-slow growth and outcome 2: fast growth or not-fast growth. The tables under each graph summarise the features used for each model. Each table also shows the AUROC scores obtained for each of models VII-IX for the two outcomes, where the AUROCs for predicting slow and fast growth using model VII were 0.66 and 0.73, respectively. Combining maximum diameter and geometric features such as undulation index (UI) and radius of curvature (RC) with the selected surface-based modes improved model predictive capacity (Model IX vs Model VII, p < 0.001).

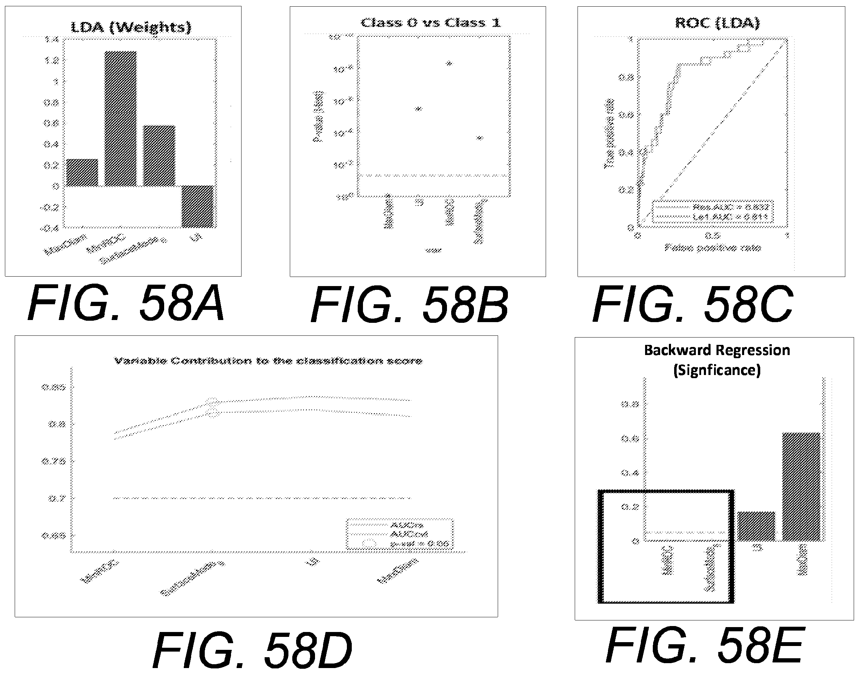

Figures 58A-E illustrate the performance of the different variables relating to PCA derived surface-based mode 6 and geometric measures (UI, radius of curvature and maximum diameter) used to train the generalised linear models VII-IX for predicting outcome 1 (slow vs not-slow growth). Figure 58A shows a graph relating to LDA (weights) for each variable within the generalized linear model. Figure 58B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 58C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 58D illustrates the influence of each variable to the calculated AUC. Figure 58E shows the significance of each variable using backward regression, which is a method used to calculate the significance of the input variables by iteratively removing the variable and evaluating model performance.

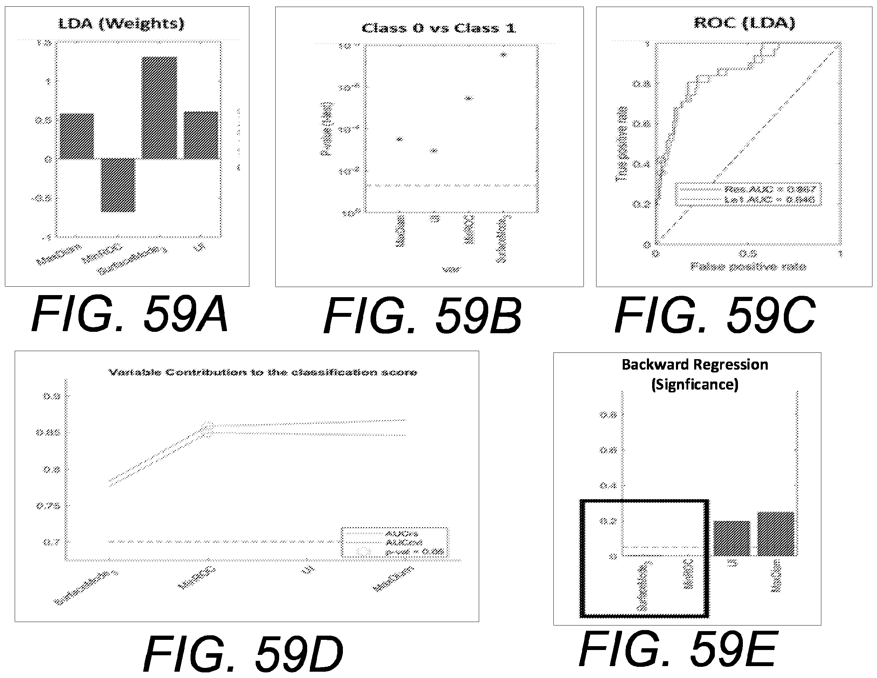

Figures 59A-E illustrate the performance of the different variables relating to PCA derived surface-based mode 3 and geometric measures (UI, radius of curvature and maximum diameter) used to train the generalised linear models VII-IX for predicting outcome 2 (fast vs not-fast growth). Figure 59A shows a graph relating to LDA (weights) for each variable within the generalized linear model. Figure 59B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 59C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 59D illustrates the influence of each variable to the calculated AUC. Figure 59E shows the significance of each variable using backward regression, which is a method used to calculate the significance of the input variables by iteratively removing the variable and evaluating model performance.

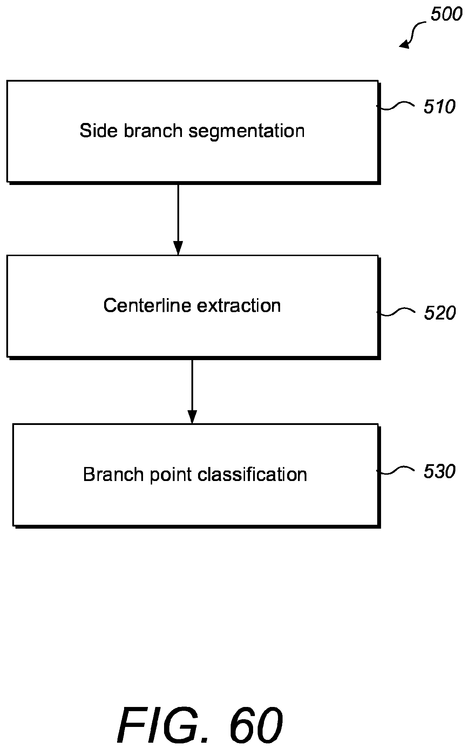

In addition to the aortic shape and surface, the inventors hypothesized that the location of the aortic side branches as they arise from the aorta may play a role in predicting aneurysmal growth secondary to alterations in hemodynamic forces. As such, figure 60 shows a flowchart of a method 500 for extracting the locations of the aortic side branches.

At step 510, the method comprises, performing side branch segmentation on a AAA volumetric model to obtain a volumetric model of AAA with side branches.

At step 520, the method comprises performing centreline extraction on a volumetric model of the AAA with aortic side branches to obtain an aortic centreline.

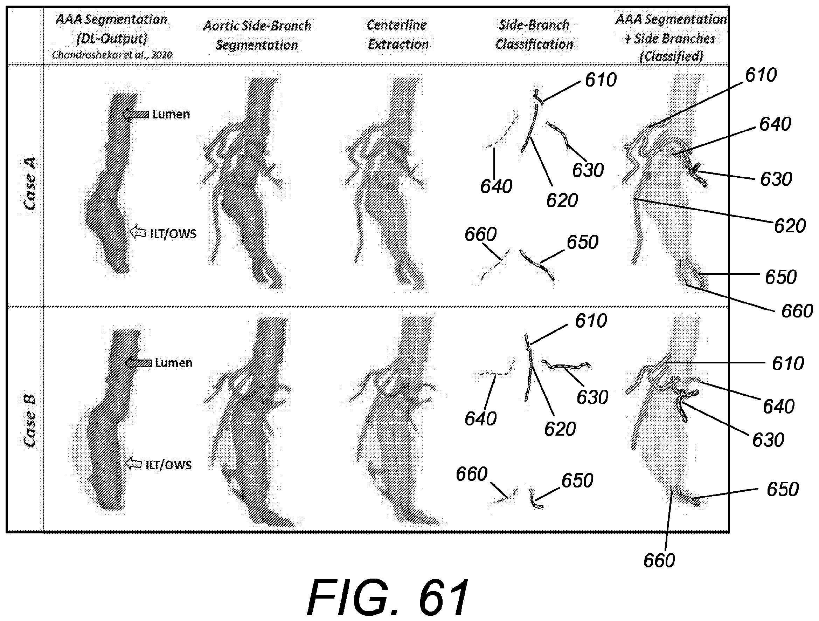

At step 530, the method comprises performing branch point classification to classify the branches into six categories based on the branch artery, where the six categories as labelled in figure 61 are as follows: Celiac Artery 610, Superior Mesenteric Artery (SMA) 620, Left Renal artery 630, Right Renal artery 640, Left iliac artery 650, and right iliac artery 660. The classification algorithm was based on the locations of the branches along the aorta and spatial relationships between the branches.

Figure 61 is an illustration of the above method, which shows from left to right the AAA segmentation, aortic side-branch segmentation, centerline extraction, side-branch classification and the AAA segmentation plus the side branches 610-660 classified for two AAA volumetric models (cases A and B).

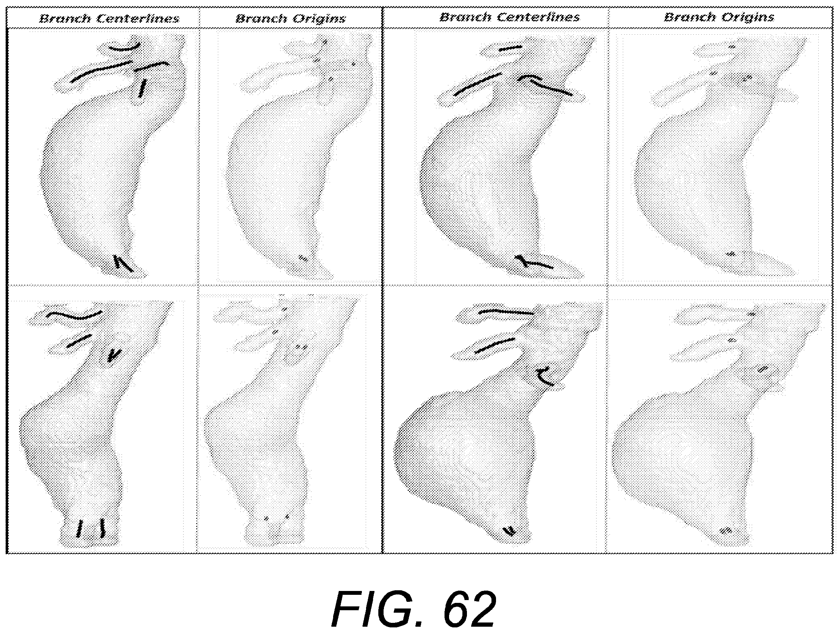

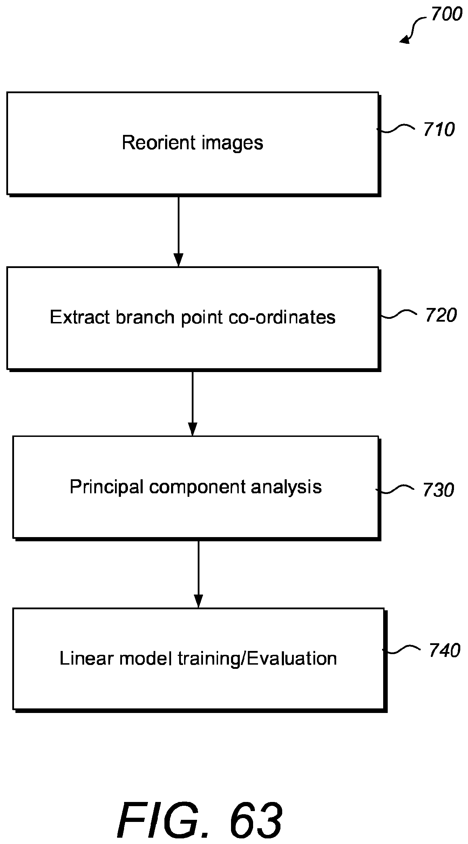

Figure 62 illustrates four expanded segmentations with classified branch centrelines and the branch origins, where the branch origins were defined as the first 5 coordinates (x, y, z) of each branch. Origin points were used instead of the entire branch centreline to focus the analysis at the intersection points and standardize the segment used from each branch. For example, as can be seen in figure 62, the length of the iliac centrelines were visually different between the patients. This variability is due to the semi-automatic nature of branch segmentation. The x, y and z coordinates for each set of branch origins between patients were normalized by subtracting the mean and dividing by the standard deviation. This matrix ([6 branches x 5 origin points/branch] x 192 patients) served as the input into the PCA, as explained in relation to figure 63.

Figure 63 shows a flowchart of a third method 700 for predicting a growth rate of an aortic aneurysm using PCA derived geometric features relating to aortic side branches.

At step 710, the method comprises reorienting the images obtained from each of the 192 patients to the same coordinate axis based on a fixed point.

At step 720, the method comprises extracting a set of branch-point co-ordinates corresponding to regions along the volumetric model where an aortic side branch originates, as described above in relation to figure 60.

At step 730, the method comprises performing PCA on the extracted set of branch-point coordinates.

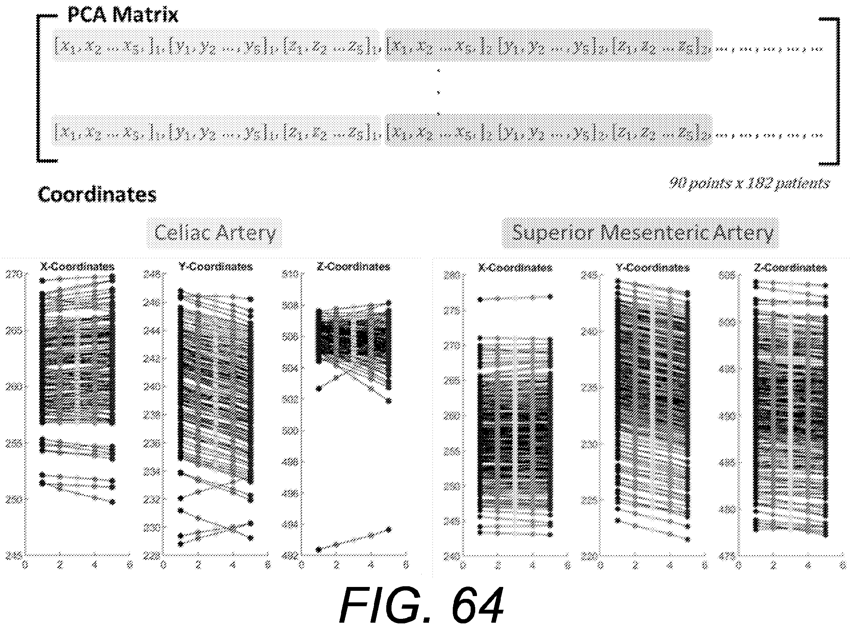

Figure 64 shows an example of a matrix on which PCA is performed, where the matrix comprises x, y and z co-ordinates corresponding to the branch origins. Figure 64 also shows examples of the x, y and z coordinates for the celiac artery and the SMA.

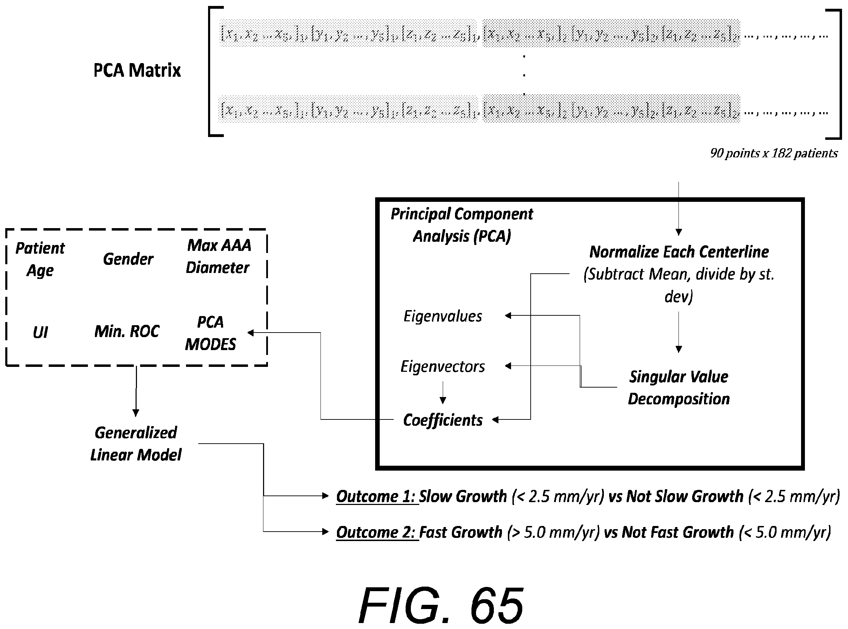

Figure 65 shows the matrix as described above to be taken as an input for PCA. As shown in figure 65 in the box labelled Principal component analysis (PCA), all x, y, and z coordinates are normalized by subtracting the mean and dividing by the standard deviation. Singular value decomposition is then performed on each of the normalised x, y, and z coordinates to obtain eigenvalues from which PCA coefficients are obtained. The PCA coefficients are then used to determine PCA modes.

Steps 710-730 of figure 63 highlight the extraction or determination of branch-point (BP) modes, where the BP modes are modes extracted or determined from the location/origin of the aortic side branches exiting the aorta or along the aorta. These BP modes are then taken as input to a generalised linear model, as explained below in relation to step 740.

At step 740, the method comprises using the derived PCA BP modes as inputs to train a generalised linear model for classifying the growth rate into one or more categories. For example, the generalised linear model, which may be a multinomial logistic regression (such as that described above in relation to Figures 16-25), can be used for the binary classification task of predicting either: 1.) Slow Growth or Not-Slow Growth or 2.) Fast Growth or Not-Fast Growth, where "slow growth" is defined as an annual growth rate of less than 2.5 mm per year and "not-slow growth" is defined as an annual growth rate of more than 2.5 mm per year. The "fast growth" is defined as an annual growth rate of more than 5 mm per year and the "not-fast growth" is defined as an annual growth rate of less than 5 mm per year. This is illustrated in figure 65, where the PCA modes as shown inside the dashed box are taken as input to a generalised linear model which outputs either outcome 1 or outcome 2, where outcome 1 relates to classifying the growth rates as slow growth or not-slow growth and output 2 relates to classifying the growth rate as fast growth or not-fast growth.

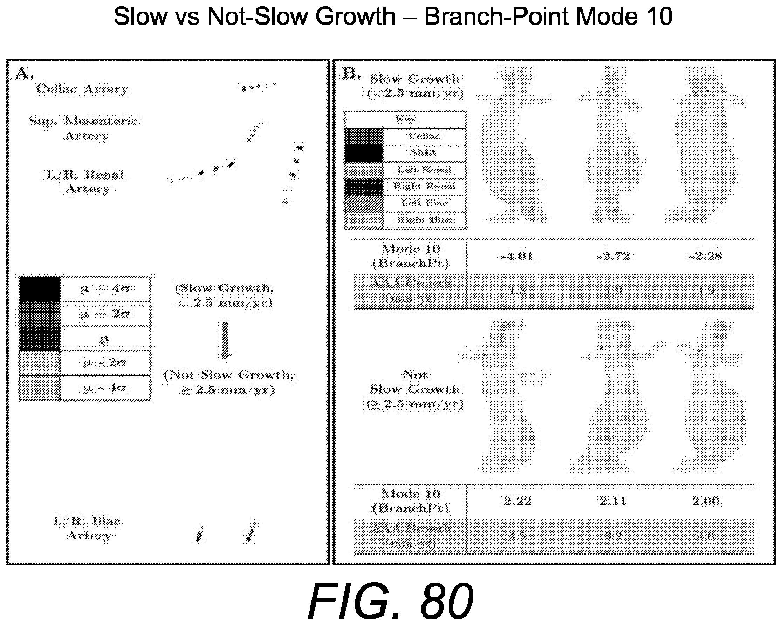

The inventors devised three different models, models X-XII, to predict the growth rate of, for example, an aortic aneurysm, where each of the three models were trained using different features. Model X was trained using only modes 1-10 of the derived PCA branch-point modes. Linear discriminant analysis (LDA) was used to determine mode significance and only significant modes were used for subsequent analysis. In terms of BP modes, modes 10 and 3 were statistically significant for predicting slow and fast growth, respectively. The significance of modes 3 and 10 is discussed below in relation to figures 80-81. Model XI was trained using a combination of these significant modes of the derived PCA BP modes, herein referred to as selected BP modes and geometric features (e.g. radius of curvature and undulation index). Model XII was trained using a combination of the selected BP modes, geometric features (e.g. radius of curvature and undulation index) and maximum AAA diameter. This is illustrated in 65, where one or more of the above features as shown in the dotted box are taken as an input to train a generalised linear model, where each model then outputs outcome 1: slow growth or not-slow growth or outcome 2: fast growth or not-fast growth. All models were trained using iterative 10-fold cross-validation (n = 100 iterations).

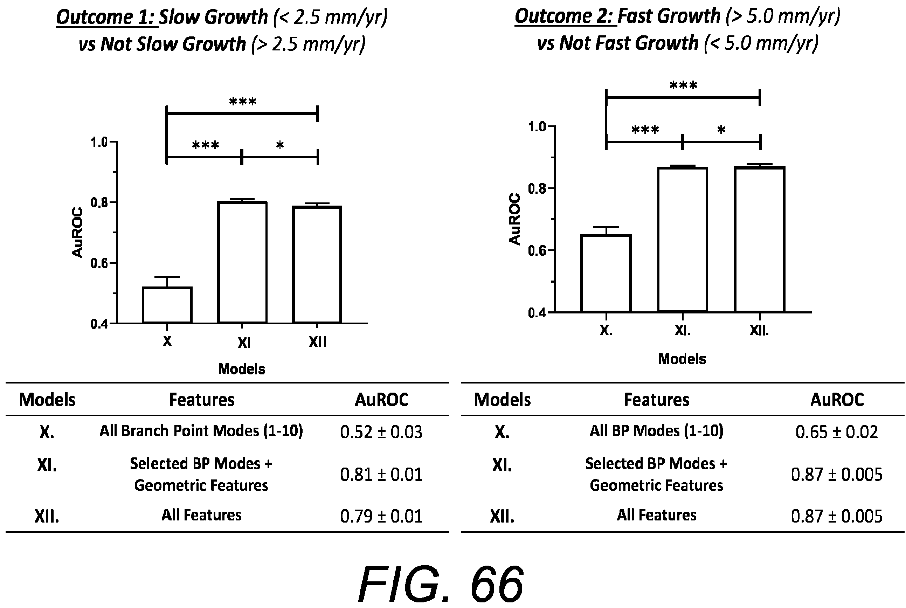

Figure 66 shows a bar graph relating to the AUROC scores obtained from each of models X-XII used to predict outcome 1: slow growth or not-slow growth and outcome 2: fast growth or not-fast growth. The tables under each graph summarise the features used for each model. Each table also shows the AUROC scores obtained for each of models X-XII for the two outcomes, where the AUROCs for predicting slow and fast growth using model X were 0.52 and 0.65, respectively. Combining maximum AAA diameter and geometric features with the selected BP modes improved model predictive capacity (Model XII vs Model X, p < 0.001).

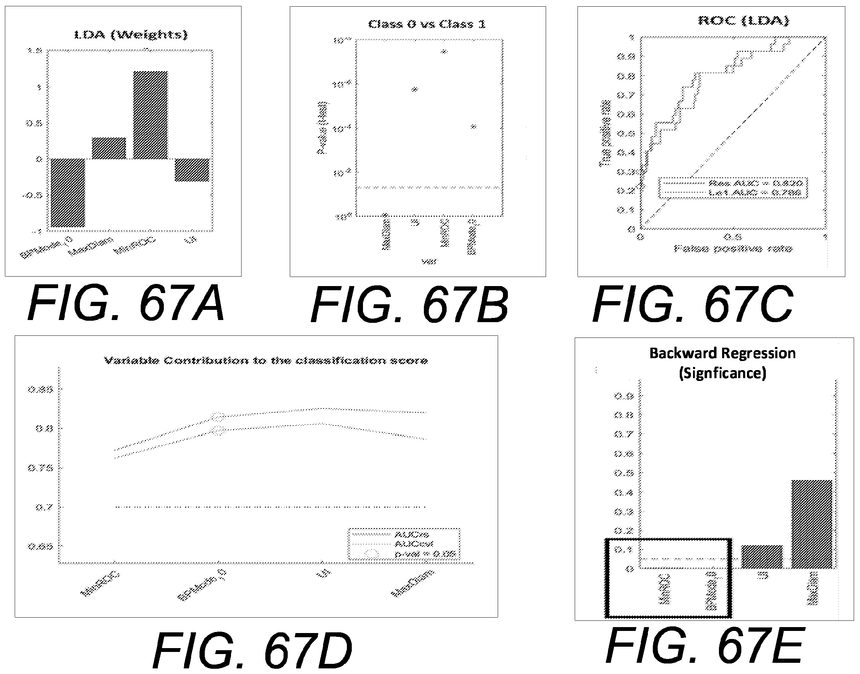

Figures 67A-E illustrate the performance of the different variables relating to PCA derived BP mode 1 and geometric measures (UI, RC and maximum diameter) used to train the generalised linear models X-XII for predicting outcome 1 (slow vs not-slow growth). Figure 67A shows a graph relating to LDA (weights) for each variable within the generalized linear model. Figure 67B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 67C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 67D illustrates the influence of each variable to the calculated AUC. Figure 67E shows the significance of each variable using backward regression, which is a method used to calculate the significance of the input variables by iteratively removing the variable and evaluating model performance.

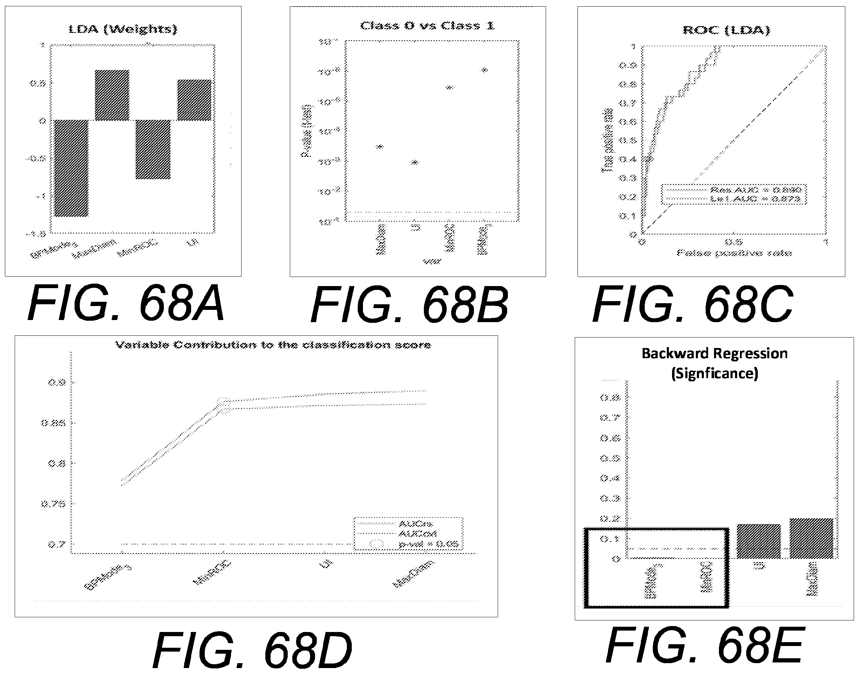

Figures 68A-E illustrate the performance of the different variables relating to PCA derived BP mode 1 and geometric measures (UI, RC and maximum diameter) used to train the generalised linear models X-XII for predicting outcome 2 (fast vs not-fast growth). Figure 67A shows a graph relating to LDA (weights) for each variable within the generalized linear model. Figure 67B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 67C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 67D illustrates the influence of each variable to the calculated AUC. Figure 67E shows the significance of each variable using backward regression, which is a method used to calculate the significance of the input variables by iteratively removing the variable and evaluating model performance.

In addition to the above, the inventors investigated the impact of combining the relevant shape-based, surface-based and BP modes on the predictive capacity for both classification tasks relating to fast and slow growth. Models XIII-XV were trained by combining the relevant shape-based, surface-based and BP modes for both classification tasks, as explained below.

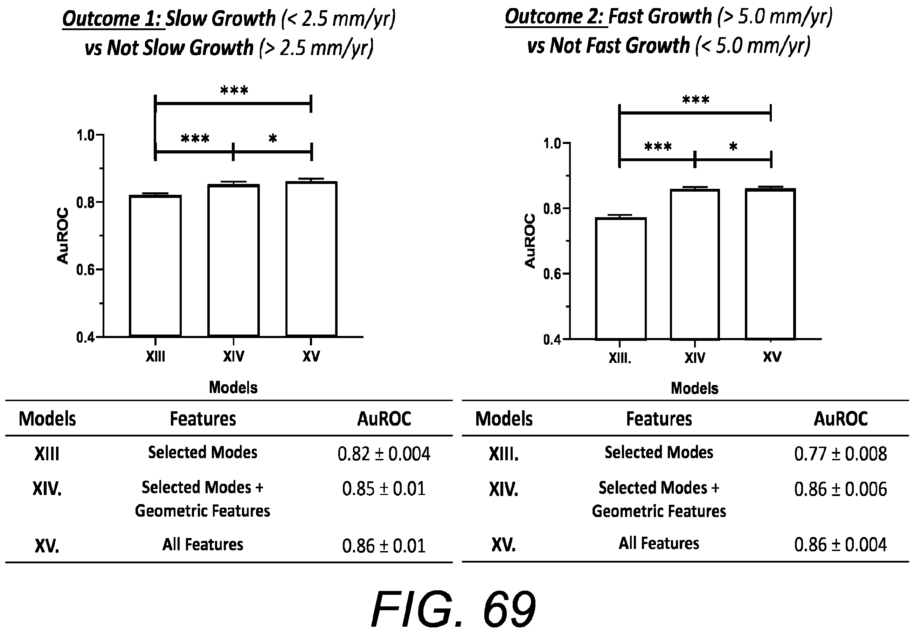

Figure 69 shows a bar graph relating to the AUROC scores obtained from each of models XIII-XV. The tables under each graph summarise the features used for each model, where model XIII was trained using selected shape-based, surface-based and BP modes only. These features are referred to as "Selected modes" in the table shown in figure 69. Model XIV was trained using these selected shape-based, surface-based, BP modes and geometric features (UI and RC). Model XV was trained using only those features which remained significant after integration with the geometric features, as explained below. Each table in figure 69 also shows the AUROC scores obtained for each of models X-XII.

Selected Modes or PCA-derived geometric features were different for both classification tasks. For slow growth, model XIII was trained using selected modes which included shape-based modes 3 and 7, surface-based mode 6 and branch point mode 10. These four selected modes were then combined with geometric features which formed the features used to train model XIV. Of the four selected modes, shape-based modes 3 and 7 and branch point mode 10 remained significant after integrating with geometric features. Furthermore, it was also noted that after integration with geometric features, max diameter and UI were no longer statistically significant and were removed from subsequent models. As such, model XV was trained using only the features which remained significant after training model XIV, where these features have been referred to as "All features" in the table shown in figure 69.

For fast growth, model XIII was trained using selected modes which included shape-based mode 4, surface-based mode 3, and branch point mode 3. Of these three selected modes, shape-based mode 4 and branch point mode 3 remained significant after integrating with geometric features. Furthermore, it was also noted that after integration with geometric features, max diameter and UI were no longer statistically significant and were removed from subsequent models. As such, model XV was trained using only the features which remained significant after training model XIV, where these features have been referred to as "All features" in the table shown in figure 69. All of the above models were trained using iterative 10-fold cross-validation and model performance was assessed by comparing the AUROC between models for the 100 iterations.

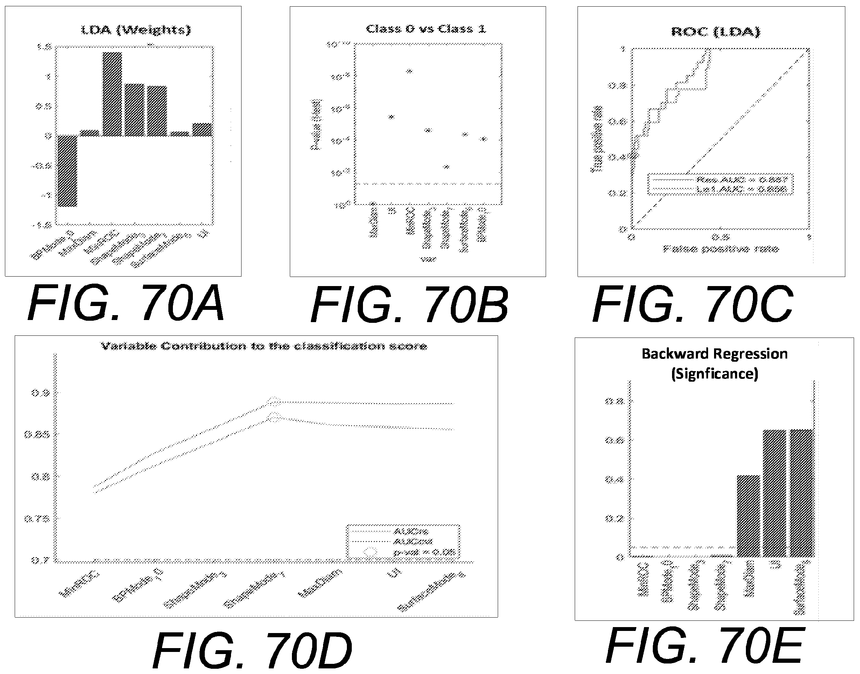

Figures 70A-E illustrate the performance of the different variables relating to PCA derived shape-based modes 3 and 7, surface-based mode 6, BP mode 10 and geometric measures (UI, RC and maximum diameter) used to train the generalised linear models X-XIV for predicting outcome 1 (slow vs not-slow growth). Figure 70A shows a graph relating to LDA (weights) for each variable within the generalized linear model. Figure 70B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 70C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 70D illustrates the influence of each variable to the calculated AUC. Figure 70E shows the significance of each variable using backward regression, which is a method used to calculate the significance of the input variables by iteratively removing the variable and evaluating model performance.

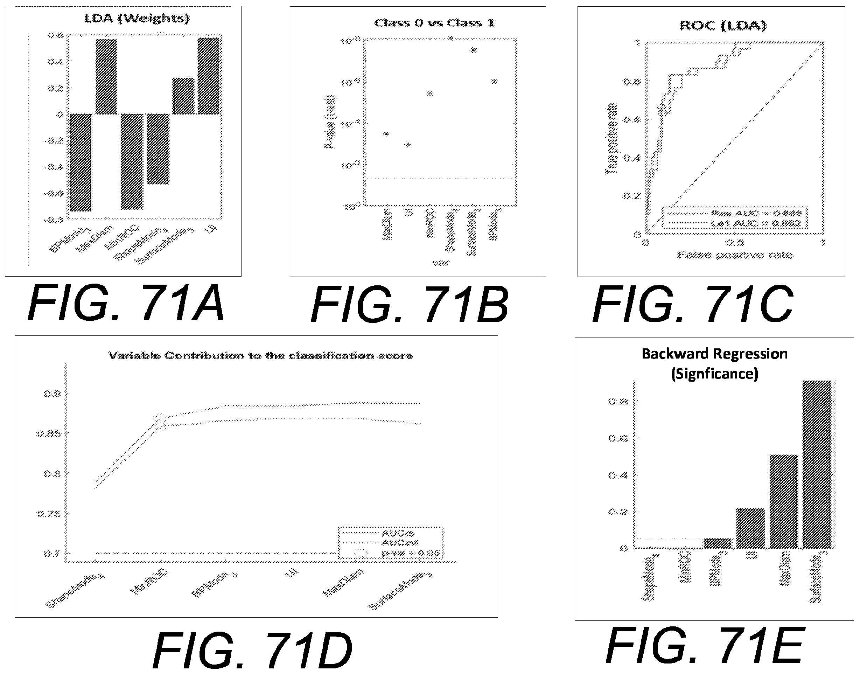

Figures 71A-E illustrate the performance of the different variables relating to PCA derived shape-based mode 4, surface-based mode 3, BP mode 3 and geometric measures (UI, RC and maximum diameter) used to train the generalised linear models X-XIV for predicting outcome 2 (fast vs not-fast growth). Figure 71A shows a graph relating to LDA (weights) for each variable within the generalized linear model. Figure 71B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 71C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 71D illustrates the influence of each variable to the calculated AUC. Figure 71E shows the significance of each variable using backward regression, which is a method used to calculate the significance of the input variables by iteratively removing the variable and evaluating model performance.

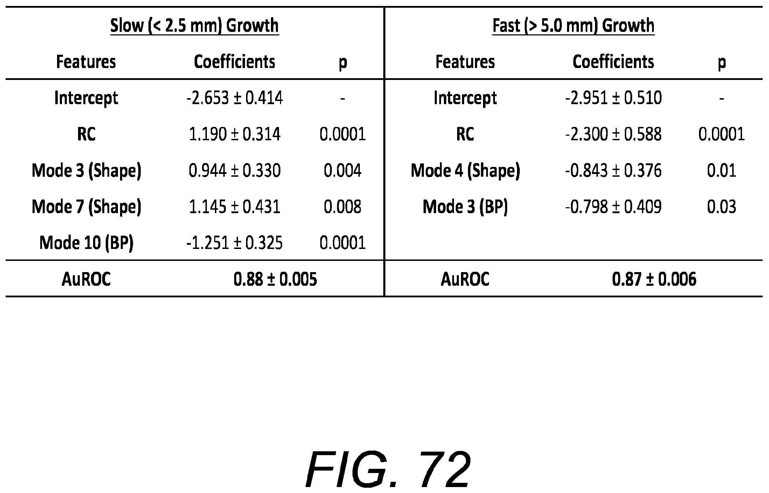

Figure 72 shows a table relating to model XV, referred to as the optimised model herein. Model performance is highlighted using AUROC. The coefficients and p-values for each of the variables within the optimized logistic regression models as well as their performance are highlighted in Error! Reference source not found. table shown in figure 72.

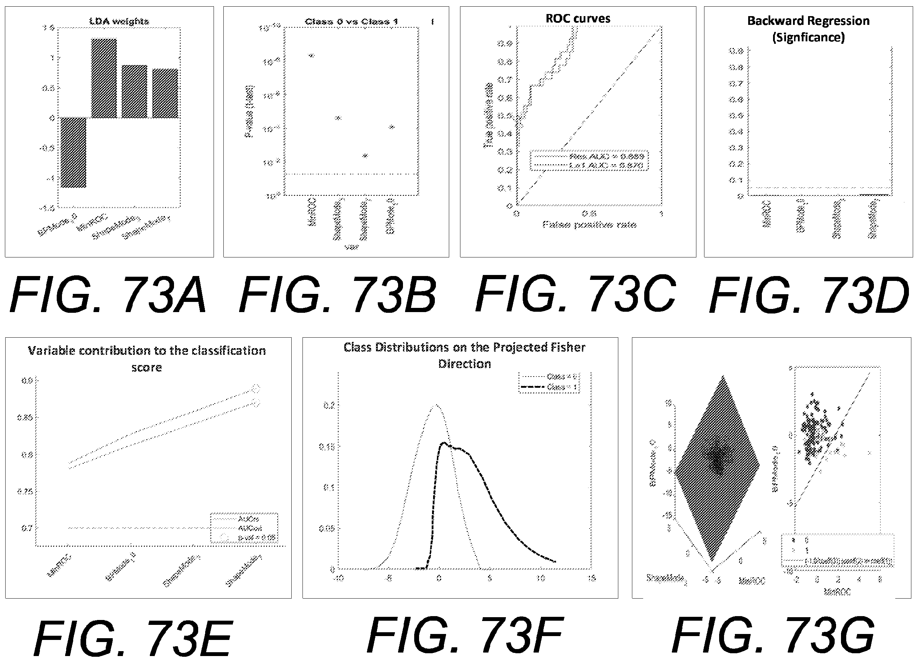

Figures 73A-G illustrate the performance of the different variables relating to the features which were used to train model XV for predicting outcome 1 (slow vs not-slow growth). As explained above, the features used to train model XV were the features which remained significant after training model XIV. These features included geometric features (RC) and the PCA derived shape-based modes 3 and 7 and BP 10 for predicting outcome 1. Figure 73A shows a graph relating to LDA (weights) for each variable within the generalized linear model. Figure 73B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 73C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 73D shows the significance of each variable using backward regression, which is a method used to calculate the significance of the input variables by iteratively removing the variable and evaluating model performance. Figure 73E illustrates the influence of each variable to the calculated AUC. Figure 73F shows the class distributions on the projected fisher direction indicating sufficient separation between the two classes. Figure 73G shows LDA on the most significant variables, where the left graph shows the LDA on 3 significant variables and the right graph shows LDA on 2 significant variables.

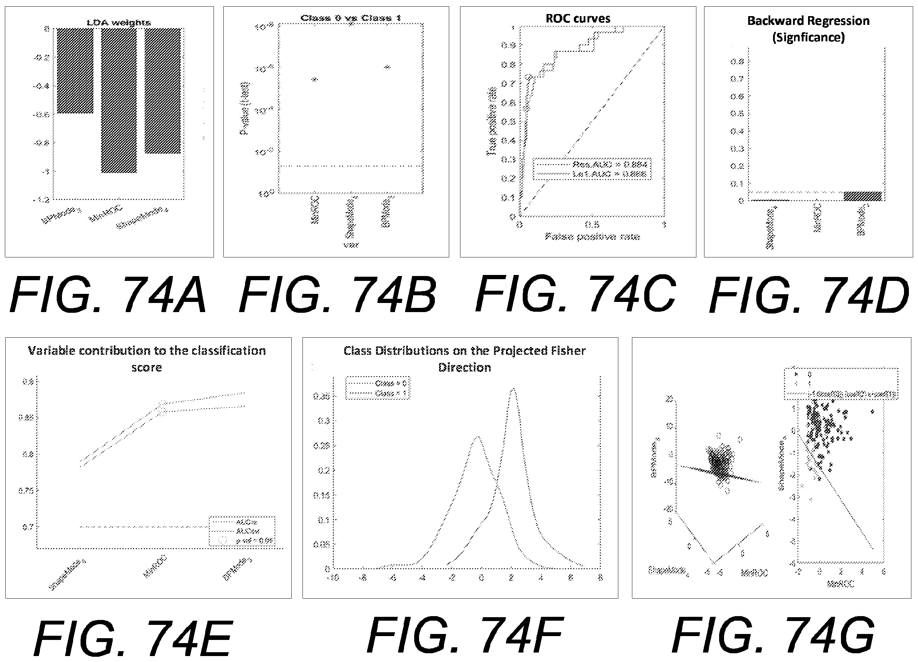

Figures 74A-G illustrate the performance of the different variables relating to the features which were used to train model XV for predicting outcome 2 (fast vs not-fast growth). As explained above, the features used to train model XV were the features which remained significant after training model XIV. These features included geometric features (RC) and the PCA derived shape-based modes 3 and 7 and BP 10 for predicting outcome 1. Figure 74A shows a graph relating to LDA (weights) for each variable within the generalized linear model. Figure 74B shows the significance of each of the variables within the generalized linear model for the classification task (Class 0 vs 1). The dotted line indicates a p-value of 0.05. Asterisks above the dotted line indicate significant variables. The dotted line indicates a p-value of 0.05 and the asterisks above the dotted line indicate significant variables. Figure 74C shows receiver operator characteristic curves with calculated area under the curves (AUC) including Leave-One-Out Cross Validation (Le.1 AUC) and Residual (Res. AUC). These curves highlight the performance of a classification model at all thresholds. Figure 74D shows the significance of each variable using backward regression, which is a method used to calculate the significance of the input variables by iteratively removing the variable and evaluating model performance. Figure 74E illustrates the influence of each variable to the calculated AUC. Figure 74F shows the class distributions on the projected fisher direction indicating sufficient separation between the two classes. Figure 74G shows LDA on the most significant variables, where the left graph shows the LDA on 3 significant variables and the right graph shows LDA on 2 significant variables.

Accordingly, it has been demonstrated that by analysing one or more geometric measures such as maximum AAA diameter, radius of curvature (RC), undulation index (UI) and PCA-derived geometric features of a volumetric model of at least a portion of an artery having an aneurysm, a categorical growth rate of an aneurysm can be predicted.

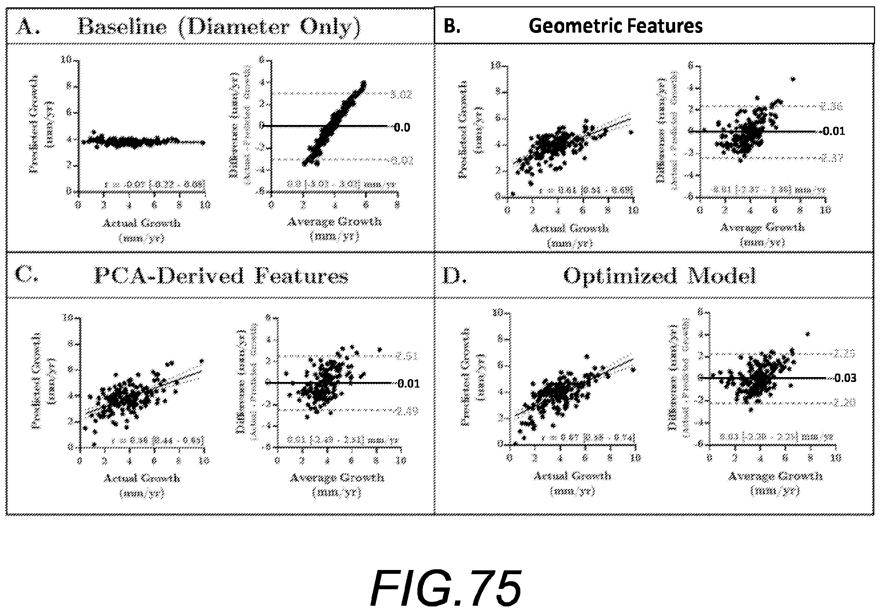

The inventors also assessed the effectiveness of different models at predicting a continuous growth rate. The results of these models are shown in figure 75.

Figures 75 shows scatter plots of the predicted growth rate against the actual growth rate of the aneurysms and scatter plots of the difference against the average growth rate for four linear regression models (A-D) trained to predict a continuous AAA growth rate, where the four linear regression models (A-D) were trained using 10-fold cross-validation to predict annual AAA growth. The results of each validation fold are reported against actual AAA growth values. The correlation coefficient and its 95% confidence interval are highlighted on each graph (Dotted lines). Bland-Altman analysis was performed for each model to assess the differences between model output and ground truth measurements. Bias (solid horizontal line) and corresponding 95% limits of agreement (dashed horizontal lines) are highlighted for each plot. Scatter plots relating to the model A were obtained from a baseline model which was trained using maximum AP diameter only, as this is a commonly used criteria to monitor AAA progression within the clinic. Scatter plots relating to models B-C were obtained from models trained using geometric features (Max Diameter, UI and RC) and PCA-derived geometric features, respectively. As explained above, shape-based modes 3, 4, and 7 and BP modes 3, and 10 were identified to play a significant role in the binary classification of aneurysmal growth when combined with geometric features. Thus, scatter plots relating to model D corresponds to an optimised model trained using PCA derived features and geometric features.

Comparison of the scatter plots obtained from models A-B show that with regards to geometric features, the linear model trained using Max AP diameter, UI and RC (model B) was able to predict annual AAA growth to a greater accuracy than the model trained using only max AP Diameter (model A). This was similar to that of the logistic regression model of AAA growth. Growth predictions from this model were significantly correlated (r = 0.61, p <0.001) against actual growth measurements. Subsequently, Bland-Altman plots between the geometric features model output and ground truth measurements indicated more narrow limits of agreements when compared against that of the baseline model (Diameter only). Similarly, the geometric features model (model B) is able to predict annual AAA growth to within 2 mm error in 90% of cases (vs. 69% using the baseline model).

The scatter plots obtained from model C show that the model trained using PCA-derived geometric features was able to predict annual AAA growth to a greater accuracy than that trained using only max AP diameter (model A) and a similar accuracy to that trained using the geometric features model (model B). Similar limits of agreement were observed between the outputs from the hypothesis-driven and PCA-derived models. Predicted AAA growth was strongly correlated against actual growth measurements (r = 0.56, p <0.001) with a mean absolute error between measurements of 1.10 $\pm$ 0.78 mm/yr. This model (using PCA-derived features) was able to predict annual AAA growth to within 2 mm error in 88% of cases (vs. 69% using the baseline model).

The scatter plots obtained from model D show that integrating the selected geometric features and PCA-derived geometric features further improved model performance. The correlation coefficient between predicted and actual AAA growth increased to 0.67 and the limits of agreement reduced when compared against to the baseline model (Diameter only). This optimized model, also predicts annual AAA growth to within 2 mm error in 92.5% of cases (vs. 69% using the baseline model) and within 1 mm error in 73.5% of cases (vs. 52% using the baseline model).

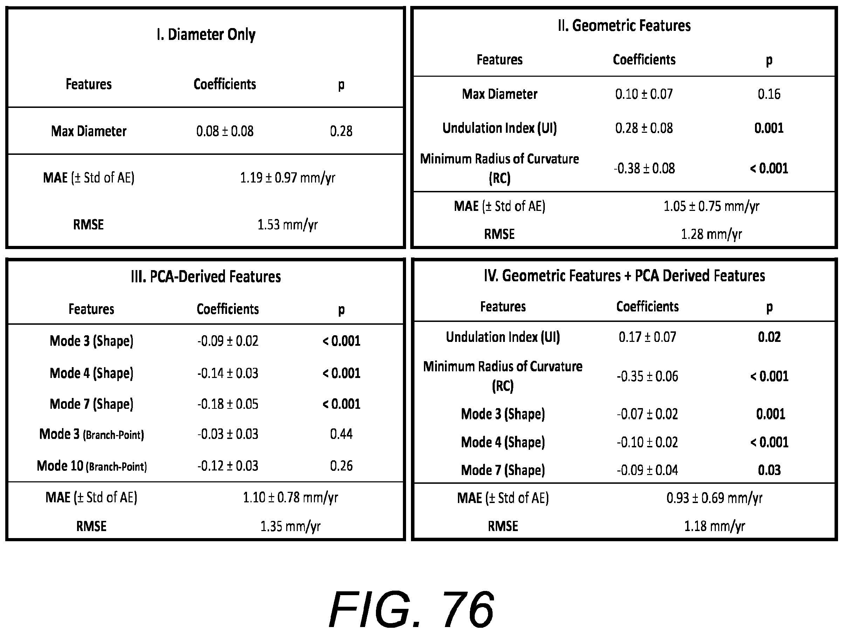

Figure 76 shows tables I-IV with the feature coefficients corresponding to the above mentioned four linear regression models trained to predict AAA growth, where the significance of each feature is highlighted. Model performance is indicated using mean of the absolute error (MAE) and root-mean-square-error (RMSE) between model prediction and ground truth AAA growth measurements.

Accordingly, it has been demonstrated that by analysing one or more geometric measures such as max AAA diameter, radius of curvature (RC), undulation index (UI) and PCA-derived geometric features of a volumetric model of at least a portion of an artery having an aneurysm, the numeric/continuous growth rate of an aneurysm can be predicted.

Figures 77-81 below highlight the significance of the different PCA derived shape-based and surface-based modes for the prediction of slow and fast aneurysmal growth.

Figures 77A-B show the relevance of mode 3 (shape-based PCA) on the average AAA shape and its impact on aneurysmal growth (slow vs not-slow growth). Figure 77A shows the average AAA shape ($\mu$) constructed from the PCA input matrix which consisted of a centreline (46 X, Y, Z coordinates) with planes orthogonal to the centreline (circular planes with defined maximum diameters, 46 max diameters). The influence of the selected mode (mode = 3) was applied to the average shape to obtain the modified aneurysms. In figure 77A,

the $+2\sigma$ and $-2\sigma$ represent the modified aneurysms, 2 standard deviations from the $\mu$, where $\sigma$ represents the deviation from average AAA shape due to the selected mode or feature. The greater the $\sigma$, the larger the deviation from the mean and the impact of the selected mode. This was visualized to appreciate the influence of the selected mode. Three views are shown at 90° increments (view 1 -3). Figure 77B shows the visualisation of six aneurysms with similar max AAA diameter at the starting timepoint. Figure 77B also shows a table with the mode value and the AAA growth rate (mm/yr) for each of the six aneurysms for slow vs not-slow growth. It is visually apparent from figure 77B that aneurysms, which start adjacent to the location of the renal arteries and are more fusiform in nature, are more likely to present with slow growth patterns. Figure 77B also shows that mode 3 is inversely correlated with aneurysmal growth.

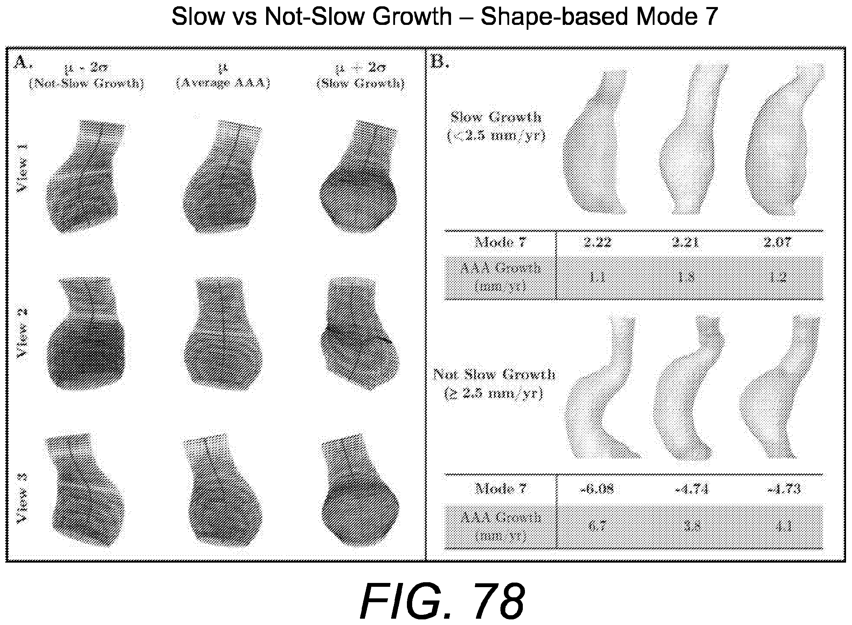

Figure 78A-B show the relevance of mode 7 (shape-based PCA) on the average AAA shape and its impact on aneurysmal growth (slow vs not-slow growth). Figure 78A shows the average AAA shape ($\mu$) constructed from the PCA input matrix which consisted of a centreline (46 X, Y, Z coordinates) with planes orthogonal to the centreline (circular planes with defined maximum diameters, 46 max diameters). The influence of the selected mode (mode = 7) was applied to the average shape to obtain the modified aneurysms. In figure 78A, the $+2\sigma$ and $-2\sigma$ represent the modified aneurysms, 2 standard deviations from the $\mu$, where $\sigma$ represents the deviation from average AAA shape due to the selected mode or feature. The greater the $\sigma$, the larger the deviation from the mean and the impact of the selected mode. This was visualized to appreciate the influence of the selected mode. Three views are shown at 90° increments (view 1 -3). Figure 78A suggests that aneurysms with not only a greater degree but also a particular direction of localized tortuosity within aneurysmal neck region are less likely to represent a slow growth phenotype. This is further supported in Figure 78B, which highlights six AAAs with similar diameters at baseline with different growth phenotypes. Mode values, and the AAA growth (mm/yr) for each of these aneurysms are shown in the table shown in Figure 78B for slow vs not-slow growth.

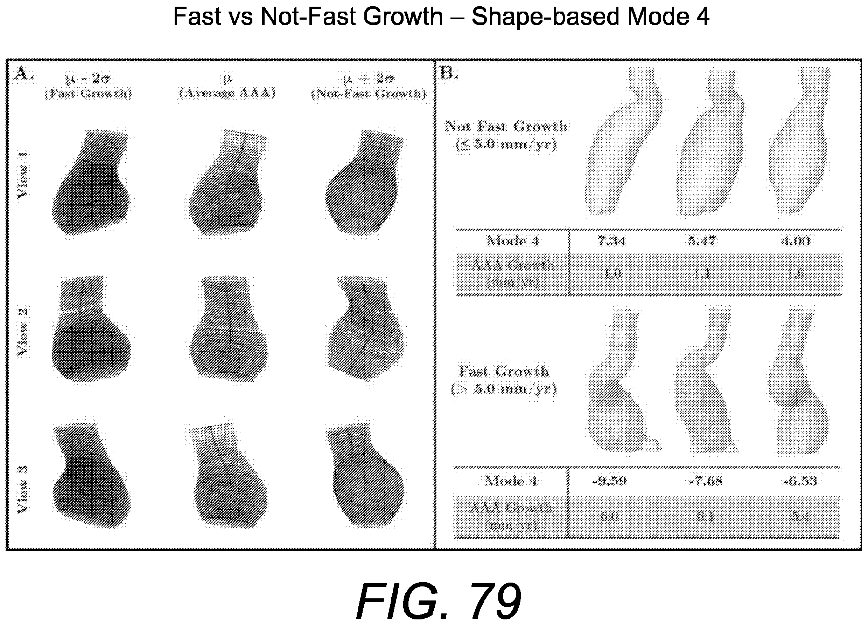

Figures 79A-B show the relevance of mode 4 (shape-based PCA) on the average AAA shape and its impact on aneurysmal growth (fast vs not-fast growth). Figure 79A shows the average AAA shape ($\mu$) constructed from the PCA input matrix which consisted of a centreline (46 X, Y, Z coordinates) with planes orthogonal to the centreline (circular planes with defined maximum diameters, 46 max diameters). The influence of the selected mode (mode = 4) was applied to the average shape to obtain the modified aneurysms. In Figure 79A, the $+2\sigma$ and $-2\sigma$ represent the modified aneurysms, 2 standard deviations from the $\mu$, where $\sigma$ represents the deviation from average AAA shape due to the selected mode or feature. The greater the $\sigma$, the larger the deviation from the mean and the impact of the selected mode.

This was visualized to appreciate the influence of the selected mode. Three views are shown at 90° increments (view 1 -3). Figure 79B shows the visualisation of six aneurysms with similar max AAA diameter at the starting timepoint. Figure 79B also shows a table with the mode value and the AAA growth rate (mm/yr) for each of the six aneurysms for fast vs not-fast growth. Figure 79B shows that mode 4 is inversely correlated with aneurysmal growth and that aneurysms with not only a greater degree but also a particular direction of localized tortuosity are more susceptible to fast growth. For example, increased centreline tortuosity with a right-sided preference is linked with a rapid-growth phenotype than that with a left-sided preference. Additionally, the location of the tortuous region also seems to be linked with AAA progression.

In addition to shape-based modes, certain branch-point modes (mode 3 and 10) played a significant role in the classification of AAA growth phenotype.

Figures 80A-B show the influence of BP mode 10 on the average branch locations and its impact on aneurysmal growth (Slow vs Not-Slow Growth). Figure 80A shows the average branch origins ($\mu$) which were derived from the PCA input matrix and consisted of the first 5 coordinates (X, Y, Z) of each branch centreline. The influence of the selected mode (mode = 10) was applied to the average origins to obtain the modified origins, which were calculated at 2 standard deviations from the average and four standard deviations from the average (+4$\sigma$, +2$\sigma$, -2$\sigma$ and -4$\sigma$). Figure 80A shows the modified origins at +4$\sigma$, +2$\sigma$, -2$\sigma$ and -4$\sigma$ to visually appreciate the influence of BP mode 10 for each of the six aortic branches.

Figure 80B shows visualisations of six aneurysms with similar max AAA diameter at the starting timepoint for slow growth vs not slow growth prediction. The locations of the branch origins for the arteries as categorised above are also shown on each aneurysm. Figure 80B also shows a table with the mode value and the AAA growth in mm/yr for each aneurysm. Figure 80B shows that BP mode 10 is positively correlated with aneurysmal growth and increased vertical placement of the left renal artery origin and medial placement of the right renal artery origin (next to the AAA shape) is associated with the slow growth phenotype.

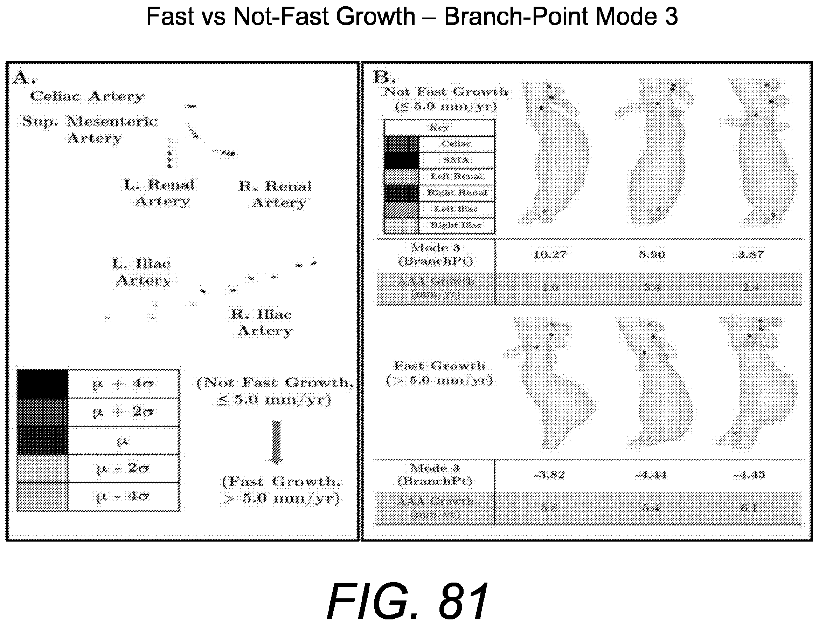

Figure 81A-B show the influence of BP mode 3 on the average branch locations and its impact on aneurysmal growth (Fast vs Not-Fast Growth).

Figure 81A shows the average branch origins ($\mu$) which were derived from the PCA input matrix and consisted of the first 5 coordinates (X, Y, Z) of each branch centreline. The influence of the selected mode (mode = 3) was applied to the average origins to obtain the modified origins, which were calculated at 2 standard deviations from the average ($\mu$) and four standard deviations from the average (+4$\sigma$, +2$\sigma$, -2$\sigma$ and -4$\sigma$). Figure XA (page 63) shows the modified origins at +4$\sigma$, +2$\sigma$, -2$\sigma$ and -4$\sigma$ to visually appreciate the influence of BP mode 3 for each of the six aortic branches.

Figure 81B shows visualisations of six aneurysms with similar max AAA diameter at the starting timepoint for fast growth vs not-fast growth prediction. The locations of the branch origins for the arteries as categorised above are also shown on each aneurysm. Figure 81B also shows a table with the maximum diameter, mode value and the AAA growth in mm/yr for each aneurysm. Figure 81B shows that mode 3 is negatively correlated with aneurysmal growth and that infero-lateral placement of the iliac artery origin (away from the AAA shape) is associated with the fast growth phenotype.

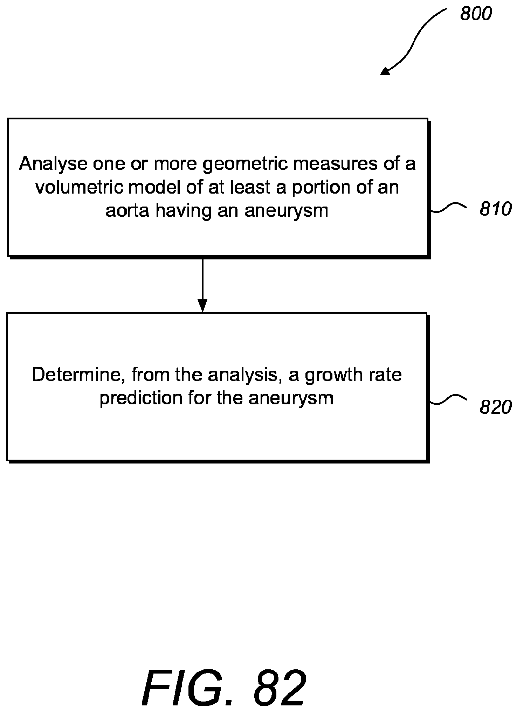

Figure 82 shows a flowchart of a method 800 for predicting a growth rate of an aortic aneurysm. The method 800 may implement any of the methods or combination thereof as described above.

At 810, the method comprises analysing one or more geometric measures of a volumetric model of at least a portion of an aorta having an aneurysm.

In some embodiments, the volumetric model may be received from an external source, for example a third party server.

In other embodiments, the volumetric model may be generated by one of any number of means. For example, the volumetric model may be generated from received CT scan data, such as a CT angiogram. A CT scan may be performed on a subject to capture information on an artery within the subject, the artery having an aneurysm. The CT scan may result in data for a plurality of CT images / slices that can be used to construct the volumetric model. The arterial region, including the aneurysm, may be identified in each image using an image segmentation algorithm or other means, such that the volume of at least a portion of the artery can be determined and may be reconstructed. Image segmentation is the process of assigning a label to pixels/voxels in an image such that pixels/voxels with the same label share certain characteristics or computed properties such as colour or intensity, and can be used to identify features of an artery.

In some embodiments, generating the volumetric model may comprise receiving a plurality of CT image slices, the CT image slices showing the portion of the artery having an aneurysm, segmenting the artery including the aneurysm in each CT image slice, and constructing the volumetric model from the segmented images.

In some embodiments, generating the volumetric model may comprise receiving CT image data, inputting the received CT image data into a trained machine learning model for identifying features of the artery and aneurysm within CT image data, and producing, from the output of the trained machine learning model, a volumetric model of the portion of the artery having the aneurysm. For example, international patent application number PCT/GB2020/052013, entitled "Computerised Tomography Image Processing", filed on 21 August 2020, and international patent application number PCT/GB2020/052014, entitled "Computerised Tomography Image Processing", filed on 21 August 2020, the contents of which are hereby incorporated by reference, both describe methods of identifying structural features of at least a portion of an artery having an aneurysm from CT scan data. A volumetric model may be constructed from the identified features output from such methods.

If a trained machine learning model is used, the machine learning model may be any suitable trained machine learning model. For example, the machine learning model may comprise a neural network, and in particular a convolutional neural network. The machine learning model may be trained by minimising a cost function involving segmentation mask information ("ground truth") and the output of the final layer of the network. The cost function may comprise any suitable cost function such as a quadratic cost function, a cross-entropy cross function, a log-likelihood cost function. The minimisation may be performed for example by gradient descent, stochastic gradient descent or variations thereof, using backpropagation to adjust weights and biases within the neural network accordingly. Training may involve the use of further techniques known to the skilled person, such as regularization. Mini-batch sizes and numbers of epochs may be selected and fine-tuned during training. The neural network may comprise several layers of neurons (which may be, for example, perceptrons, sigmoid neurons, tanh neurons, or rectified linear units/rectified linear neurons), and may include one or more convolution layers, and may include one or more maxpool layers, and may include a soft-max layer.

The machine learning model may comprise, for example, a generative adversarial network (GAN). A GAN may be used to generate a volumetric model from input CT image data.

While the generation of a volumetric model from CT image data has been described above, other data sources may be used, for example a positive emission tomography (PET) scan data, and in particular FDG-PET scan data, or single photon emission computed tomography (SPECT) scan data.

The geometric measure is a measure of the volumetric model, being a measure of the centreline curvature of the volumetric model ( in this case the radius of curvature of the centreline ) or a shape-based measure of the aneurysm (in this case the undulation index). The geometric measure may be a PCA derived geometric measure such as shape-based modes, where the shape-based modes are derived from a AAA volumetric model using its centreline and maximum diameters along planes orthogonal to its centreline. The geometric measure may be a PCA derived geometric measure such as surface-based modes, where the surface-based modes are derived from curves characterizing the aneurysmal outer wall. The geometric measure may be a PCA derived geometric measure such as branch-point modes, where the branch-point modes are derived from the location/origin of aortic side branches exiting the aorta.

Referring again to Figure 82, at 820, the method comprises determining, from the analysis, a growth rate prediction for the aortic aneurysm.

Determining a growth rate prediction for the aneurysm may comprise determining a growth rate category for the aneurysm. For example, the aneurysm growth rate may fall into one of two or more categories such as "slow", "medium" & "fast", or "less than 2.5 mm per year", "2.5-5 mm per year" & "greater than 5 mm per year", according to some function of the one or more analysed geometric measures. The skilled person would appreciate that the number and definition of the categories may differ from the specific examples described herein.

Determining a growth rate category for the aneurysm may comprise inserting the values of the analysed one or more geometric measures of the volumetric model into a function. For example, a multinomial logistic regression (such as that described above in relation to Figures 16-25) may be used to categorise the growth rate of the aneurysm. As the skilled person would appreciate, other methods may be used for determining a predicted growth rate category for an aneurysm. For example, one may compare the analysed one or more geometric measures with one or more known values, wherein the one or more known values correspond to a geometric measure of a volumetric model of a portion of an artery having a reference aneurysm for which the growth rate of reference aneurysm is substantially known. In other examples, a machine learning algorithm may be used to predict a growth rate category for the aneurysm.

Determining a growth rate prediction for the aneurysm may comprise determining a numeric/continuous growth rate prediction, for example a prediction in millimetres per year. The skilled person would appreciate that the units of the growth rate prediction may differ.

Determining a growth rate prediction value may comprise inserting the values of the analysed one or more geometric measures of the volumetric model into a function. For example, a linear regression model (such as those described above in relation to Figures 26-39) may be used to predict a value for the growth rate of the aneurysm. As the skilled person would appreciate, other methods may be used for determining a predicted growth rate value for an aneurysm. For example, one may compare the analysed one or more geometric measures with one or more known values, wherein the one or more known values correspond to a geometric measure of a volumetric model of a portion of an artery having a reference aneurysm for which the growth rate of reference aneurysm is substantially known. In other examples, a machine learning algorithm may be used to predict a growth rate value for the aneurysm.

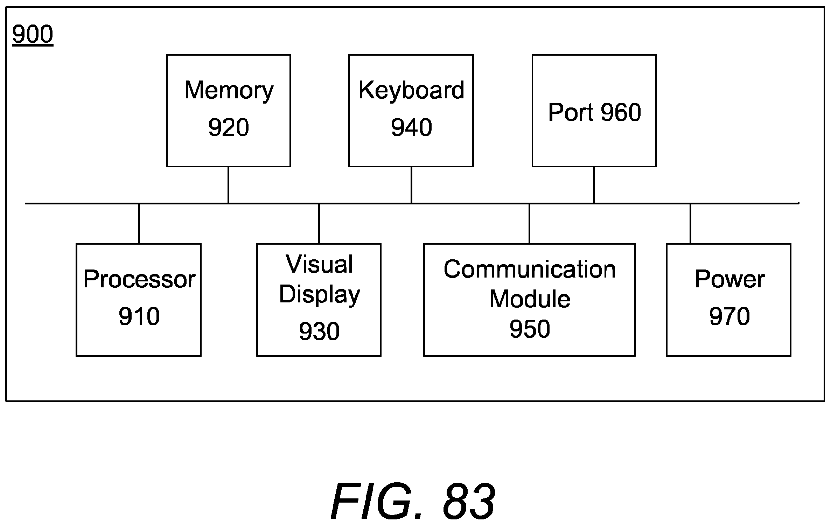

Figure 83 shows a block diagram of a computing apparatus 900 suitable for performing a method as described herein. Computing apparatus 900 may comprise a computing device, a server, a mobile or portable computer and so on. Computing apparatus 900 may be distributed across multiple connected devices. Other architectures to that shown in Figure 83 may be used as will be appreciated by the skilled person.

Referring to Figure 83, computing apparatus 900 includes one or more processors 910, one or more memories 920, a number of optional user interfaces such as visual display 930 and virtual or physical keyboard 940, a communications module 950, and optionally a port 960 and optionally a power source 970. Each of components 910, 920, 930, 940, 950, 960, and 970 are interconnected using various busses. Processor 210 can process instructions for execution within the computing apparatus 200, including instructions stored in memory 920, received via communications module 950, or via port 960.

Memory 920 is for storing data within computing apparatus 900. The one or more memories 920 may include a volatile memory unit or units. The one or more memories 920 may also be another form of computer-readable medium, such as a magnetic or optical disk. One or more memories 920 may provide mass storage for the computing apparatus 900. Instructions for performing a method as described herein may be stored within the one or more memories 920.

The apparatus 900 includes a number of user interfaces including visualising means such as a visual display 930 and a virtual or dedicated user input device such as keyboard 940.

The communications module 950 is suitable for sending and receiving communications between processor 910 and remote systems.

The port 960 is suitable for receiving, for example, a non-transitory computer readable medium containing one or more instructions to be processed by the processor 910.

The processor 910 is configured to receive data, access the memory 920, and to act upon instructions received either from said memory 920 or a computer-readable storage medium connected to port 960, from communications module 950 or from user input device 240.

As an example, the computing apparatus 900 may receive, via the communications module 950, data of a volumetric model of at least a portion of an aorta having an aortic aneurysm. In another example, the computing apparatus may receive data such as CT scan data. The processor 910 may be configured to follow instructions stored in the one or more memories 920 to construct or generate the volumetric model from the received scan data.

The processor 910 may be configured to follow instructions stored in one or more memories 920 to analyse the received data to calculate or compute one or more geometric measures of the volumetric model. The processor 910 may be further configured to follow instructions stored in the one or more memories 920 to determine, from the values of those geometric measures, a predicted growth rate of the aneurysm.

The determined predicted growth rate may be output to the display 930, or via the communications module 950 to a third party. The processor 910 may be configured to take further actions such as to propose an action based on the determined growth rate prediction.

In another example, the processor 910 of the computing apparatus may be configured to follow instructions stored in one or more memories 920 that when executed cause the computing apparatus to carry out any of the methods described herein.

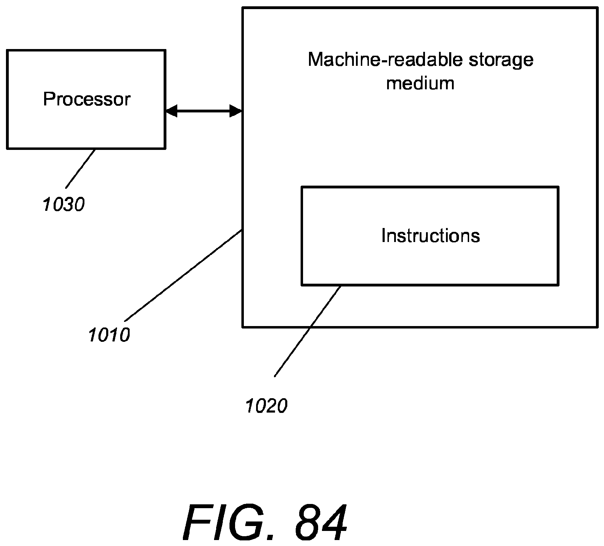

Figure 84 illustrates a computer readable medium 1010 according to some examples.

The computer readable medium 1010 stores units, with each unit including instructions 1020 that, when executed, cause a processor 1030 (such as the processor 910 of computing apparatus 900) or other processing/computing device or apparatus to perform particular operations.

The computer readable medium 1010 may include instructions 1020 that, when executed, cause a processing device 1030 to analyse one or more geometric measures of a volumetric model of at least a portion of an aorta having an aortic aneurysm. The computer readable medium 1010 may include instructions 1020 that, when executed, cause a processing device 1030 to determine, from the analysis, a growth rate prediction for the aneurysm.

In another example, the computer readable medium 1010 may include instructions, that when executed, cause the processing device 1030 to carry out any of the methods described herein.

It will be appreciated that embodiments of the present invention can be realised in the form of hardware, software or a combination of hardware and software. Any such software may be stored in the form of volatile or non-volatile storage such as, for example, a storage device like a ROM, whether erasable or rewritable or not, or in the form of memory such as, for example, RAM, memory chips, device or integrated circuits or on an optically or magnetically readable medium such as, for example, a CD, DVD, magnetic disk or magnetic tape. It will be appreciated that the storage devices and storage media are embodiments of machine-readable storage that are suitable for storing a program or programs that, when executed, implement embodiments of the present invention.

Accordingly, embodiments provide a program comprising code for implementing a system or method as described herein and a machine-readable storage storing such a program. Still further, embodiments of the present invention may be conveyed electronically via any medium such as a communication signal carried over a wired or wireless connection and embodiments suitably encompass the same.

Many variations of the methods described herein will be apparent to the skilled person.

For example, subject may be understood to mean a human or animal or other suitable organism having blood vessels, or a sample therefrom.

In the discussion above, CT image data has been described as a good source of data from which to generate a volumetric model of an aorta. The CT image data may be contrast CT image data (x-ray image data obtained from a CT scan performed on a subject with a contrast agent present within the subject during scanning) or non-contrast image data (x-ray image data obtained from a CT scan performed on a subject in the absence of a contrast agent).

The invention is not restricted to the details of any foregoing embodiments. The claims should not be construed to cover merely the foregoing embodiments, but also any embodiments which fall within the scope of the claims.

**Claims**

1. A computer-implemented method (100) for predicting a growth rate of an aortic aneurysm, the method comprising:

   generating a volumetric model based on non-contrast computed tomography, CT, image data, analysing one or more geometric measures of the volumetric model of at least a portion of an aorta having an aortic aneurysm, wherein the one or more geometric measures comprises at least a radius of a curvature of a path through the volumetric model and an undulation index;
   determining, from the analysis, a numerical growth rate prediction for the aortic aneurysm.

2. A computer-implemented method according to claim 1, wherein the measure of curvature of a path through the volumetric model comprises a measure of the centreline curvature of the volumetric model.

3. A computer-implemented method according to any preceding claim, wherein the geometric measure of the one or more geometric measures is the radius of curvature of a path through the volumetric model and further comprises a measure of a branchpoint angle at a branchpoint of the volumetric model.

4. A computer-implemented method according to any preceding claim , wherein a geometric measure of the one or more geometric measures of the volumetric model comprises a measure derived using principal component analysis, PCA, and the measure derived using PCA comprises a shape based mode extracted from the volumetric model, and extracting the shape-based modes comprises:

   extracting a centreline from the volumetric model;
   generating planes orthogonal to the centreline;
   determining a maximum diameter of each orthogonal plane; and
   performing PCA on a set of coordinates corresponding to the extracted centreline and the maximum diameter of each orthogonal plane.

5. A computer-implemented method according to any preceding claim , wherein a geometric measure of the one or more geometric measures of the volumetric model comprises a measure derived using principal component analysis, PCA, and the measure derived using PCA comprises a surface-based mode extracted from the volumetric model.

6. A computer-implemented method according to claim 5, wherein extracting the surface-based mode comprises:

   extracting a surface line from the volumetric model by generating a curve along the aortic surface; and
   performing PCA on a set of coordinates corresponding to the extracted surface line.

7. A computer-implemented method according to any preceding claim, wherein a geometric measure of the one or more geometric measures of the volumetric model comprises a measure derived using principal component analysis, PCA, and the measure derived using PCA comprises a branch-point, BP, mode extracted from the volumetric model.

8. A computer-implemented method according to claim 7, wherein extracting the BP mode comprises:

   extracting a set of branch-point co-ordinates corresponding to regions along the volumetric model where an aortic side branch originates; and
   performing PCA on the extracted set of branch-point coordinates.

9. A computer-implemented method according to any preceding claim, wherein generating the volumetric model comprises:

   receiving the non-contrast computed tomography, CT, image data, the non-contrast CT image data showing the portion of the aorta having an aortic aneurysm; and
   inputting the received non-contrast CT image data into a trained machine learning model for identifying features of the aorta and aneurysm within the non-contrast CT image data; and
   producing, from the output of the trained machine learning model, a volumetric model of the portion of the aorta having the aneurysm.

**10.** A computer-implemented method according to any preceding claim, wherein the aneurysm comprises an abdominal aortic aneurysm.

**Patentansprüche**

**1.** Computerimplementiertes Verfahren (100) zum Vorhersagen einer Wachstumsrate eines Aortenaneurysmas, wobei das Verfahren Folgendes umfasst:

Erzeugen eines volumetrischen Modells auf der Grundlage von kontrastmittelfrei erzeugten Computertomographie-, CT-, Bilddaten, Analysieren eines oder mehrerer geometrischer Maße des volumetrischen Modells mindestens eines Abschnitts einer Aorta mit einem Aortenaneurysma, wobei das eine oder die mehreren geometrischen Maße mindestens einen Radius einer Krümmung eines Pfades durch das volumetrische Modell und einen Wellenindex umfassen;

Bestimmen, aus der Analyse, einer numerischen Wachstumsratenvorhersage für das Aortenaneurysma.

**2.** Computerimplementiertes Verfahren gemäß Anspruch 1, wobei das Maß der Krümmung eines Pfades durch das volumetrische Modell ein Maß der Mittellinienkrümmung des volumetrischen Modells umfasst.

**3.** Computerimplementiertes Verfahren gemäß einem vorhergehenden Anspruch, wobei das geometrische Maß des einen oder der mehreren geometrischen Maße der Krümmungsradius eines Pfads durch das volumetrische Modell ist und ferner ein Maß eines Verzweigungspunktwinkels an einem Verzweigungspunkt des volumetrischen Modells umfasst.

**4.** Computerimplementiertes Verfahren gemäß einem vorhergehenden Anspruch, wobei ein geometrisches Maß des einen oder der mehreren geometrischen Maße des volumetrischen Modells ein unter Verwendung von Hauptkomponentenanalyse, PCA, abgeleitetes Maß umfasst, und wobei das unter Verwendung von PCA abgeleitete Maß einen aus dem volumetrischen Modell extrahierten formbasierten Modus umfasst, und wobei das Extrahieren der formbasierten Modi Folgendes umfasst:

Extrahieren einer Mittellinie aus dem volumetrischen Modell;
Erzeugen von Ebenen orthogonal zu der Mittellinie;
Bestimmen eines maximalen Durchmessers jeder orthogonalen Ebene; und

Durchführen von PCA an einem Satz von Koordinaten, die der extrahierten Mittellinie und dem maximalen Durchmesser jeder orthogonalen Ebene entsprechen.

**5.** Computerimplementiertes Verfahren gemäß einem vorhergehenden Anspruch, wobei ein geometrisches Maß des einen oder der mehreren geometrischen Maße des volumetrischen Modells ein unter Verwendung von Hauptkomponentenanalyse, PCA, abgeleitetes Maß umfasst, und wobei das unter Verwendung von PCA abgeleitete Maß einen aus dem volumetrischen Modell extrahierten oberflächenbasierten Modus umfasst.

**6.** Computerimplementiertes Verfahren gemäß Anspruch 5,
wobei das Extrahieren des oberflächenbasierten Modus Folgendes umfasst:

Extrahieren einer Oberflächenlinie aus dem volumetrischen Modell durch Erzeugen einer Kurve entlang der Aortenoberfläche; und
Durchführen von PCA an einem Satz von Koordinaten, die der extrahierten Oberflächenlinie entsprechen.

**7.** Computerimplementiertes Verfahren gemäß einem vorhergehenden Anspruch, wobei ein geometrisches Maß des einen oder der mehreren geometrischen Maße des volumetrischen Modells ein unter Verwendung von Hauptkomponentenanalyse, PCA, abgeleitetes Maß umfasst, und wobei das unter Verwendung von PCA abgeleitete Maß einen aus dem volumetrischen Modell extrahierten Verzweigungspunkt-, BP-, Modus umfasst.

**8.** Computerimplementiertes Verfahren gemäß Anspruch 7,
wobei das Extrahieren des BP-Modus Folgendes umfasst:

Extrahieren eines Satzes von Verzweigungspunktkoordinaten, die Regionen entlang des volumetrischen Modells entsprechen, wo ein Aortenseitenast entsteht; und
Durchführen von PCA an dem extrahierten Satz von Verzweigungspunktkoordinaten.

**9.** Computerimplementiertes Verfahren gemäß einem vorhergehenden Anspruch, wobei das Erzeugen des volumetrischen Modells Folgendes umfasst:

Empfangen der kontrastmittelfrei erzeugten Computertomographie-, CT-, Bilddaten, wobei die kontrastmittelfrei erzeugten CT-Bilddaten den Teil der Aorta zeigen, der ein Aortenaneurysma aufweist; und

Eingeben der empfangenen kontrastmittelfrei erzeugten CT-Bilddaten in ein trainiertes maschinelles Lernmodell zum Identifizieren von Merkmalen der Aorta und des Aneurysmas innerhalb der kontrastmittelfrei erzeugten CT-Bilddaten; und

Erzeugen eines volumetrischen Modells des Teils der Aorta mit dem Aneurysma aus der Ausgabe des trainierten Modells für maschinelles Lernen.

10. Computerimplementiertes Verfahren gemäß einem vorhergehenden Anspruch, wobei das Aneurysma ein Bauchaortenaneurysma umfasst.

**Revendications**

1. Procédé mis en œuvre par ordinateur (100) permettant de prédire le taux de croissance d'un anévrisme aortique, le procédé comprenant :

la génération d'un modèle volumétrique sur la base de données d'images de tomographie assistée par ordinateur non contrastée, CT,

l'analyse d'une ou plusieurs mesures géométriques du modèle volumétrique d'au moins une partie d'une aorte comportant un anévrisme aortique, lesdites une ou plusieurs mesures géométriques comprenant au moins un rayon d'une courbure d'un trajet à travers le modèle volumétrique et un indice d'ondulation ;

la détermination, à partir de l'analyse, d'une prédiction numérique du taux de croissance de l'anévrisme aortique.

2. Procédé mis en œuvre par ordinateur selon la revendication 1, ladite mesure de la courbure d'un trajet à travers le modèle volumétrique comprenant la mesure de la courbure de la ligne centrale du modèle volumétrique.

3. Procédé mis en œuvre par ordinateur selon l'une quelconque des revendications précédentes, ladite mesure géométrique parmi lesdites une ou plusieurs mesures géométriques étant le rayon de courbure d'un trajet à travers le modèle volumétrique et comprenant en outre la mesure d'un angle de point de branchement au niveau d'un point de branchement du modèle volumétrique.

4. Procédé mis en œuvre par ordinateur selon l'une quelconque des revendications précédentes, une mesure géométrique parmi lesdites une ou plusieurs mesures géométriques du modèle volumétrique comprenant une mesure dérivée en utilisant une analyse de composants principaux, PCA, et ladite mesure dérivée en utilisant une PCA comprenant un mode basé sur la forme extrait du modèle volumétrique, et ladite extraction des modes basés sur la forme comprenant :

l'extraction d'une ligne centrale du modèle volumétrique ;

la génération de plans orthogonaux à la ligne centrale ;

la détermination du diamètre maximal de chaque plan orthogonal ; et

la réalisation d'une PCA sur un ensemble de coordonnées correspondant à la ligne centrale extraite et au diamètre maximal de chaque plan orthogonal.

5. Procédé mis en œuvre par ordinateur selon l'une quelconque des revendications précédentes, une mesure géométrique parmi lesdites une ou plusieurs mesures géométriques du modèle volumétrique comprenant une mesure dérivée en utilisant une analyse de composants principaux, PCA, et ladite mesure dérivée en utilisant une PCA comprenant un mode basé sur la surface extrait du modèle volumétrique.

6. Procédé mis en œuvre par ordinateur selon la revendication 5, ladite extraction du mode basé sur la surface comprenant :

l'extraction d'une ligne de surface du modèle volumétrique en générant une courbe le long de la surface aortique ; et

la réalisation d'une PCA sur un ensemble de coordonnées correspondant à la ligne de surface extraite.

7. Procédé mis en œuvre par ordinateur selon l'une quelconque des revendications précédentes, une mesure géométrique parmi lesdites une ou plusieurs mesures géométriques du modèle volumétrique comprenant une mesure dérivée en utilisant une analyse de composants principaux, PCA, et ladite mesure dérivée en utilisant une PCA comprenant un mode de point de branchement, BP, extrait du modèle volumétrique.

8. Procédé mis en œuvre par ordinateur selon la revendication 7, ladite extraction du mode de BP comprenant :

l'extraction d'un ensemble de coordonnées de points de branchement correspondant aux zones le long du modèle volumétrique d'où provient un branchement latérale aortique ; et

la réalisation d'une PCA sur l'ensemble extrait de coordonnées de points de branchement.

9. Procédé mis en œuvre par ordinateur selon l'une quelconque des revendications précédentes, ladite génération du modèle volumétrique comprenant :

la réception des données d'images de tomographie assistée par ordinateur, CT, non contrastée, les données d'images de CT non contrastée montrant la partie de l'aorte comportant un anévrisme aortique ; et

l'entrée des données d'images de CT non contrastées reçues dans un modèle d'apprentissage automatique formé pour identifier les caractéristiques de l'aorte et de l'anévrisme au sein des données d'images de CT non contrastées ; et

la production, à partir de la sortie du modèle d'apprentissage automatique entraîné, d'un modèle volumétrique de la partie de l'aorte comportant l'anévrisme.

10. Procédé mis en œuvre par ordinateur selon l'une quelconque des revendications précédentes, ledit anévrisme comprenant un anévrisme aortique abdominal.

FIG. 1

18.16          14.54          9.62

*FIG. 2*

*FIG. 3*

*FIG. 4*

*FIG. 5*

## FIG. 6

## FIG. 7

## FIG. 8

## FIG. 9

r = -0.21, p < 0.05

# FIG. 10

# FIG. 11

FIG. 12

## FIG. 13

## FIG. 14

FIG. 15

*FIG. 16*

*FIG. 17*

EP 4 225 146 B1

FIG. 18

FIG. 19

# FIG. 20

| Annual Growth n = 100 | < 2.5 mm (n =20) | 2.5 − 5 mm (n =54) | > 5.0 mm (n =26) |
|---|---|---|---|
| Max AP Diameter | 51.1 ± 8.8 mm | 53.3 ± 7.1 mm | 56.4 ± 9.2 mm |
| Follow-Up Time | 2.7 ± 2.0 yrs | 3.1 ± 2.5 yrs | 2.0 ± 1.6 yrs |

# FIG. 21

# FIG. 22

# FIG. 23

| Features | r | p |
|---|---|---|
| AAA Diameter | 0.25 | **0.03** |
| AAA Volume | 0.05 | 0.67 |
| Undulation Index (Wall) | 0.38 | **< 0.001** |
| Radius of Curvature (Wall) | -0.53 | **< 0.001** |

**< 2.5 mm Growth**

| | AUROC |
|---|---|
| Diameter ( ·········· ) | 0.61 |
| Diameter + UI Only ( ----- ) | 0.73 |
| Diameter + RC Only ( ·········· ) | 0.79 |
| UI + RC Only ( ·········· ) | 0.79 |
| All Features ( ——— ) | 0.80 |

True positive rate

False positive rate

*FIG. 25*

**> 5.0 mm Growth**

| | AUROC |
|---|---|
| Diameter ( ·········· ) | 0.59 |
| Diameter + UI Only ( ----- ) | 0.72 |
| Diameter + RC Only ( ·········· ) | 0.75 |
| UI + RC Only ( ·········· ) | 0.76 |
| All Features ( ——— ) | 0.79 |

True positive rate

False positive rate

*FIG. 24*

## Train Cohort
### (n = 100)

r = 0.25, p < 0.05

RMSE: 1.51 ± 1.70 mm

*FIG. 26*

## Test Cohort
### (n = 92)

r = 0.08, p = 0.40

RMSE: 1.68 ± 1.84 mm

*FIG. 27*

EP 4 225 146 B1

Train Cohort
(n = 100)

*FIG. 28*

Predicted Growth / Actual Growth

r = 0.52, p < 0.001

RMSE: 1.32 ± 1.50 mm

Test Cohort
(n = 92)

*FIG. 29*

Predicted Growth / Actual Growth

r = 0.57, p < 0.001

RMSE: 1.38 ± 1.56 mm

*FIG. 30*

Predicted Growth / Actual Growth

r = 0.38, p < 0.001

RMSE: 1.44 ± 1.66 mm

*FIG. 31*

Predicted Growth / Actual Growth

r = 0.41, p < 0.001

RMSE: 1.53 ± 1.68 mm

**Train Cohort**
**(n = 100)**

Predicted Growth

r = 0.48, p < 0.001

Actual Growth

RMSE: 1.37 ± 1.62 mm

*FIG. 32*

**Test Cohort**
**(n = 92)**

Predicted Growth

r = 0.46, p < 0.001

Actual Growth

RMSE: 1.49 ± 1.61 mm

*FIG. 33*

Predicted Growth

r = 0.54, p < 0.001

Actual Growth

RMSE: 1.31 ± 1.50 mm

*FIG. 34*

Predicted Growth

r = 0.57, p < 0.001

Actual Growth

RMSE: 1.38 ± 1.56 mm

*FIG. 35*

EP 4 225 146 B1

FIG. 36

FIG. 37

FIG. 38

FIG. 39

| | Regressions` | Train | | Test | | < 2mm |
|---|---|---|---|---|---|---|
| | | r | p | r | p | Accuracy |
| 1 | Initial Diameter Only | 0.25 | 0.04 | 0.08 | 0.40 | 74% |
| 2 | **Radius of Curvature Only** | 0.52 | < 0.001 | 0.57 | < 0.001 | **83%** |
| 3 | Undulation Index Only | 0.38 | < 0.001 | 0.41 | < 0.001 | 79% |
| 4. | UI (Wall) + Initial Diameter | 0.48 | < 0.001 | 0.46 | < 0.001 | 76% |
| 5. | **Rad. Curvature + Initial Diameter** | 0.54 | < 0.001 | 0.57 | < 0.001 | **83%** |
| 6. | **UI (Wall) + Rad. Curvature** | 0.55 | < 0.001 | 0.60 | < 0.001 | **85%** |
| 7. | **UI (Wall) + Rad. Curvature + Initial Diameter** | 0.59 | < 0.001 | 0.61 | < 0.001 | **87%** |

*FIG. 40*

EP 4 225 146 B1

*100*

| Reorient images | ~ *110* |

↓

| Extract centreline | ~ *120* |

↓

| Generate orthogonal planes | ~ *130* |

↓

| Determine maximum diameter | ~ *140* |

↓

| Principal component analysis | ~ *150* |

↓

| Linear model training/Evaluation | ~ *160* |

# FIG. 41

FIG. 42

*FIG. 43*

FIG. 44A

FIG. 44B

*FIG. 45*

**Orthogonal Plane Sampling ( 50 pts)** → **Max Diameter measurements**

**Centerline Extraction** →

$$\left[ [x_1, x_2 \ldots x_{50}]_1, [y_1, y_2 \ldots y_{50}]_1, [z_1, z_2 \ldots z_{50}]_1, [d_1, d_2 \ldots d_{50}]_1 \right.$$
$$\vdots$$
$$\left. [x_1, x_2 \ldots x_{50}]_{200}, [y_1, y_2 \ldots y_{50}]_{200}, [z_1, z_2 \ldots z_{50}]_{200}, [d_1, d_2 \ldots d_{50}]_{200} \right]$$

**Principal Component Analysis (PCA)**

**Normalize Each Axis + Diam** (Subtract Mean, divide by st. dev)

Eigenvalues ←

Eigenvectors ←

**Singular Value Decomposition**

Coefficients ←

**Max AAA Diameter**  **UI**

**Min. ROC**  **PCA MODES**

**Generalized Linear Model**

<u>**Outcome 1:**</u> *Slow Growth (< 2.5mm/yr) vs Not Slow Growth (< 2.5 mm/yr)*

<u>**Outcome 2:**</u> *Fast Growth (> 5.0 mm/yr) vs Not Fast Growth (< 5.0 mm/yr)*

# FIG. 46

*FIG. 47*

**Outcome 1:** *Slow Growth (< 2.5 mm/yr)*
*vs Not Slow Growth (> 2.5 mm/yr)*

| Models | Features | AuROC |
|--------|----------|-------|
| IV. | All Shape-Based Modes (1-9) | 0.69 ± 0.02 |
| V. | Selected Shape-Based Modes + Geometric Features | 0.83 ± 0.01 |
| VI. | All Features | 0.84 + 0.01 |

**Outcome 2:** *Fast Growth (> 5.0 mm/yr)*
*vs Not Fast Growth (< 5.0 mm/yr)*

| Models | Features | AuROC |
|--------|----------|-------|
| IV. | All Shape-Based Modes (1-9) | 0.78 ± 0.01 |
| V. | Selected Shape-Based Modes + Geometric Features | 0.85 ± 0.006 |
| VI. | All Features | 0.86 + 0.005 |

*FIG. 48*

EP 4 225 146 B1

**FIG. 49A**

**FIG. 49B**

**FIG. 49C**

**FIG. 49D**

**FIG. 49E**

**FIG. 50A**

**FIG. 50B**

**FIG. 50C**

**FIG. 50D**

**FIG. 50E**

*200*

```
┌─────────────────────────────┐
│                             │  ⌐ 210
│      Reorient images        │
│                             │
└─────────────────────────────┘
              │
              ▼
┌─────────────────────────────┐
│                             │  ⌐ 220
│     Extract surface lines   │
│                             │
└─────────────────────────────┘
              │
              ▼
┌─────────────────────────────┐
│                             │  ⌐ 230
│  Principal component analysis│
│                             │
└─────────────────────────────┘
              │
              ▼
┌─────────────────────────────┐
│                             │  ⌐ 240
│ Linear model training/Evaluation │
│                             │
└─────────────────────────────┘
```

# FIG. 51

FIG. 52

EP 4 225 146 B1

Sample Equal-Distance Pts w.in Spline

Z + 4

Z

Z - 4

Segmentation within the AAA

Z - 4    Z    Z + 4

410

FIG. 53

EP 4 225 146 B1

*FIG. 54*

EP 4 225 146 B1

**FIG. 55**

**Principal Component Analysis (PCA)**

**Normalize Each Line**
*(Subtract Mean, divide by st. dev)*

Eigenvalues

Eigenvectors

**Singular Value Decomposition**

Coefficients

Max AAA Diameter

UI

Min. ROC

PCA MODES

**Generalized Linear Model**

**Outcome 1:** Slow Growth (< 2.5 mm/yr) vs **Not Slow Growth** (< 2.5 mm/yr)

**Outcome 2:** Fast Growth (> 5.0 mm/yr) vs **Not Fast Growth** (< 5.0 mm/yr)

*FIG. 56*

**Outcome 1:** *Slow Growth (< 2.5 mm/yr)*
*vs Not Slow Growth (> 2.5 mm/yr)*

| Models | Features | AuROC |
|--------|----------|-------|
| VII. | All Surface-Based Modes (1-10) | 0.66 ± 0.01 |
| VIII. | Selected Surface-Based Modes + Geometric Features | 0.81 ± 0.01 |
| IX. | All Features | 0.82 ± 0.01 |

**Outcome 2:** *Fast Growth (< 5.0 mm/yr)*
*vs Not Fast Growth (< 5.0 mm/yr)*

| Models | Features | AuROC |
|--------|----------|-------|
| VII. | All Surface-Based Modes (1-10) | 0.73 ± 0.02 |
| VIII. | Selected Surface-Based Modes + Geometric Features | 0.85 ± 0.005 |
| IX. | All Features | 0.85 ± 0.004 |

## FIG. 57

EP 4 225 146 B1

ROC (LDA)

FIG. 58C

Backward Regression
(Signficance)

FIG. 58E

Class 0 vs Class 1

FIG. 58B

Variable Contribution to the classification score

FIG. 58D

LDA (Weights)

FIG. 58A

FIG. 59A

FIG. 59B

FIG. 59C

FIG. 59D

FIG. 59E

500

Side branch segmentation — 510

Centerline extraction — 520

Branch point classification — 530

# FIG. 60

FIG. 61

*FIG. 62*

700

Reorient images ~ 710

Extract branch point co-ordinates ~ 720

Principal component analysis ~ 730

Linear model training/Evaluation ~ 740

*FIG. 63*

FIG. 64

**PCA Matrix**

$$[[x_1, x_2 \ldots x_5]_1, [y_1, y_2 \ldots y_5]_1, [z_1, z_2 \ldots z_5]_1, [x_1, x_2 \ldots x_5]_2, [y_1, y_2 \ldots y_5]_2, [z_1, z_2 \ldots z_5]_2 \ldots, \ldots, \ldots, \ldots, \ldots$$

$$\vdots$$

$$[x_1, x_2 \ldots x_5]_1, [y_1, y_2 \ldots y_5]_1, [z_1, z_2 \ldots z_5]_1, [x_1, x_2 \ldots x_5]_2, [y_1, y_2 \ldots y_5]_2, [z_1, z_2 \ldots z_5]_2 \ldots, \ldots, \ldots, \ldots, \ldots]$$

*90 points x 182 patients*

**Principal Component Analysis (PCA)**

**Normalize Each Centerline**
*(Subtract Mean, divide by st. dev)*

*Eigenvalues*

*Eigenvectors*

**Singular Value Decomposition**

**Coefficients**

| Patient Age | Gender | Max AAA Diameter |
| UI | Min. ROC | PCA MODES |

**Generalized Linear Model**

**Outcome 1:** *Slow Growth (< 2.5 mm/yr)* **vs** *Not Slow Growth (< 2.5 mm/yr)*

**Outcome 2:** *Fast Growth (> 5.0 mm/yr)* **vs** *Not Fast Growth (< 5.0 mm/yr)*

*FIG. 65*

**Outcome 1:** *Slow Growth (< 2.5 mm/yr)*
*vs Not Slow Growth (> 2.5 mm/yr)*

**Outcome 2:** *Fast Growth (> 5.0 mm/yr)*
*vs Not Fast Growth (< 5.0 mm/yr)*

| Models | Features | AuROC |
|--------|----------|-------|
| X. | All Branch Point Modes (1-10) | 0.52 ± 0.03 |
| XI. | Selected BP Modes + Geometric Features | 0.81 ± 0.01 |
| XII. | All Features | 0.79 ± 0.01 |

| Models | Features | AuROC |
|--------|----------|-------|
| X. | All BP Modes (1-10) | 0.65 ± 0.02 |
| XI. | Selected BP Modes + Geometric Features | 0.87 ± 0.005 |
| XII. | All Features | 0.87 ± 0.005 |

*FIG. 66*

EP 4 225 146 B1

FIG. 67A

FIG. 67B

FIG. 67C

FIG. 67D

FIG. 67E

**FIG. 68A**

**FIG. 68B**

**FIG. 68C**

**FIG. 68D**

**FIG. 68E**

EP 4 225 146 B1

**Outcome 1:** *Slow Growth (< 2.5 mm/yr)*
*vs Not Slow Growth (> 2.5 mm/yr)*

**Outcome 2:** *Fast Growth (> 5.0 mm/yr)*
*vs Not Fast Growth (< 5.0 mm/yr)*

| Models | Features | AuROC |
|---|---|---|
| XIII | Selected Modes | 0.82 ± 0.004 |
| XIV. | Selected Modes + Geometric Features | 0.85 ± 0.01 |
| XV. | All Features | 0.86 ± 0.01 |

| Models | Features | AuROC |
|---|---|---|
| XIII. | Selected Modes | 0.77 ± 0.008 |
| XIV. | Selected Modes + Geometric Features | 0.86 ± 0.006 |
| XV. | All Features | 0.86 ± 0.004 |

## FIG. 69

EP 4 225 146 B1

FIG. 70A

FIG. 70B

FIG. 70C

FIG. 70D

FIG. 70E

**FIG. 71A**

**FIG. 71B**

**FIG. 71C**

**FIG. 71D**

**FIG. 71E**

| Slow (< 2.5 mm) Growth | | | Fast (> 5.0 mm) Growth | | |
|---|---|---|---|---|---|
| Features | Coefficients | p | Features | Coefficients | p |
| Intercept | -2.653 ± 0.414 | - | Intercept | -2.951 ± 0.510 | - |
| RC | 1.190 ± 0.314 | 0.0001 | RC | -2.300 ± 0.588 | 0.0001 |
| Mode 3 (Shape) | 0.944 ± 0.330 | 0.004 | Mode 4 (Shape) | -0.843 ± 0.376 | 0.01 |
| Mode 7 (Shape) | 1.145 ± 0.431 | 0.008 | Mode 3 (BP) | -0.798 ± 0.409 | 0.03 |
| Mode 10 (BP) | -1.251 ± 0.325 | 0.0001 | | | |
| AuROC | 0.88 ± 0.005 | | AuROC | 0.87 ± 0.006 | |

*FIG. 72*

FIG. 73A  FIG. 73B  FIG. 73C  FIG. 73D

FIG. 73E     FIG. 73F     FIG. 73G

EP 4 225 146 B1

FIG. 74A  FIG. 74B  FIG. 74C  FIG. 74D

FIG. 74E  FIG. 74F  FIG. 74G

FIG.75

**I. Diameter Only**

| Features | Coefficients | p |
|---|---|---|
| Max Diameter | 0.08 ± 0.08 | 0.28 |
| MAE (± Std of AE) | 1.19 ± 0.97 mm/yr | |
| RMSE | 1.53 mm/yr | |

**II. Geometric Features**

| Features | Coefficients | p |
|---|---|---|
| Max Diameter | 0.10 ± 0.07 | 0.16 |
| Undulation Index (UI) | 0.28 ± 0.08 | **0.001** |
| Minimum Radius of Curvature (RC) | -0.38 ± 0.08 | **< 0.001** |
| MAE (± Std of AE) | 1.05 ± 0.75 mm/yr | |
| RMSE | 1.28 mm/yr | |

**III. PCA-Derived Features**

| Features | Coefficients | p |
|---|---|---|
| Mode 3 (Shape) | -0.09 ± 0.02 | **< 0.001** |
| Mode 4 (Shape) | -0.14 ± 0.03 | **< 0.001** |
| Mode 7 (Shape) | -0.18 ± 0.05 | **< 0.001** |
| Mode 3 (Branch-Point) | -0.03 ± 0.03 | 0.44 |
| Mode 10 (Branch-Point) | -0.12 ± 0.03 | 0.26 |
| MAE (± Std of AE) | 1.10 ± 0.78 mm/yr | |
| RMSE | 1.35 mm/yr | |

**IV. Geometric Features + PCA Derived Features**

| Features | Coefficients | p |
|---|---|---|
| Undulation Index (UI) | 0.17 ± 0.07 | **0.02** |
| Minimum Radius of Curvature (RC) | -0.35 ± 0.06 | **< 0.001** |
| Mode 3 (Shape) | -0.07 ± 0.02 | **0.001** |
| Mode 4 (Shape) | -0.10 ± 0.02 | **< 0.001** |
| Mode 7 (Shape) | -0.09 ± 0.04 | **0.03** |
| MAE (± Std of AE) | 0.93 ± 0.69 mm/yr | |
| RMSE | 1.18 mm/yr | |

*FIG. 76*

# Slow vs Not-Slow Growth – Shape-based Mode 3

FIG. 77

FIG. 78

Fast vs Not-Fast Growth – Shape-based Mode 4

FIG. 79

EP 4 225 146 B1

Slow vs Not-Slow Growth – Branch-Point Mode 10

FIG. 80

# Fast vs Not-Fast Growth – Branch-Point Mode 3

**A.**
Celiac Artery
Sup. Mesenteric Artery
L. Renal Artery
R. Renal Artery
L. Iliac Artery
R. Iliac Artery

| | |
|---|---|
| $\mu + 4\sigma$ | (Not Fast Growth, $\leq 5.0$ mm/yr) |
| $\mu + 2\sigma$ | |
| $\mu$ | |
| $\mu - 2\sigma$ | (Fast Growth, > 5.0 mm/yr) |
| $\mu - 4\sigma$ | |

**B.**
Not Fast Growth ($\leq 5.0$ mm/yr)

| Key | |
|---|---|
| Celiac | |
| SMA | |
| Left Renal | |
| Right Renal | |
| Left Iliac | |
| Right Iliac | |

| | | | |
|---|---|---|---|
| Mode 3 (BranchPt) | 10.27 | 3.90 | 3.87 |
| AAA Growth (mm/yr) | 1.0 | 5.4 | 2.4 |

Fast Growth (> 5.0 mm/yr)

| | | | |
|---|---|---|---|
| Mode 3 (BranchPt) | ~3.82 | ~4.44 | ~4.45 |
| AAA Growth (mm/yr) | 5.8 | 5.4 | 6.1 |

*FIG. 81*

EP 4 225 146 B1

800

```
┌─────────────────────────────────────────┐
│                                         │
│  Analyse one or more geometric measures of a  │     810
│  volumetric model of at least a portion of an │
│  aorta having an aneurysm               │
│                                         │
└─────────────────────────────────────────┘
                    │
                    ▼
┌─────────────────────────────────────────┐
│                                         │
│                                         │
│  Determine, from the analysis, a growth rate  │     820
│  prediction for the aneurysm            │
│                                         │
└─────────────────────────────────────────┘
```

# FIG. 82

**900**

| Memory 920 | Keyboard 940 | Port 960 |
|---|---|---|

| Processor 910 | Visual Display 930 | Communication Module 950 | Power 970 |
|---|---|---|---|

*FIG. 83*

EP 4 225 146 B1

Processor

1030

Machine-readable storage medium

Instructions

1010

1020

*FIG. 84*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016203288 A1
- GB 2020052013 W
- GB 2020052014 W